# EUROPEAN PATENT APPLICATION

(11) **EP 4 163 379 A1**
(43) Date of publication of application: **12.04.2023**
(21) Application number: 21909481.0
(22) Date of filing: 22.12.2021
(51) Int. Cl.: C12N 15/81, C12N 15/90, A61K 48/00, C07K 14/47, A61K 38/00, C12N 5/10

(54) **METHOD FOR IDENTIFYING AND/OR REGULATING SENESCENCE**

(30) Priority: 22.12.2020 CN 202011527970; 10.02.2021 CN 202110185630
(71) Applicant: Institute Of Zoology, Chinese Academy Of Sciences, Beijing 100101 (CN)
(72) Inventor: LIU, Guanghui, Beijing 100101 (CN); QU, Jing, Beijing 100101 (CN); ZHANG, Weiqi, Beijing 100101 (CN); LIU, Xiaoqian, Beijing 100101 (CN); LIU, Zunpeng, Beijing 100101 (CN); WU, Zeming, Beijing 100101 (CN); WANG, Si, Beijing 100053 (CN)
(74) Representative: Nederlandsch Octrooibureau
(86) International application number: PCT/CN2021/140550
(87) International publication number: WO 2022/135486

(57) **Abstract**

A method using an endogenous retrovirus (ERV) as a biomarker for detecting a degree of aging of an object or a cell, tissue or organ or isolated cell thereof. A method using an ERV as a target for treating, preventing or delaying aging of the subject or a cell, tissue or organ or isolated cell thereof.

## Description

### Field of the invention

The invention relates to the field of biomedicine. In particular, the invention provides a method for identifying and/or regulating senescence. More particularly, the invention relates to identifying the degree of senescence of a cell, tissue, organ or subject by detecting the activation level of an endogenous retrovirus, or provides a method for regulating the degree of senescence of a cell, tissue, organ or subject by inhibiting or promoting the activation level of an endogenous retrovirus.

### Background

Due to the development of medicine and improvement in public health, human life expectancy is steadily increasing, and various senescence manifestations associated with increasing age are thus more obvious. Senescence causes physiological degradation of multiple organs and the whole body as well as occurrence of chronic diseases, and to address the health problems accompanying aging, a lot of researches have been performed in the fields of gerontology and gereology, in order to slow aging rate and the occurrence and development of the corresponding aging associated chronic diseases. In addition, due to various stresses (including work stress, metabolic disorder, environmental pollution and the like) and genetic factors, some individuals present pathologic accelerated aging phenotype. Therefore, gereology encompasses a wide area, not only old age, but also the full life period, aimed at extending the time period that human beings are healthy with a younger state of body function. Hence, it is very important for gereology and the whole field of medicine to discover biological markers for identifying the degree of aging of an individual or a cell, tissue or organ thereof, and therapeutic targets for preventing or delaying the senescence of an individual or a cell, tissue or organ thereof.

Although studies about senescence have been advanced in many aspects and several assumptions have been proposed about the urge for senescence, the mechanism of senescence has not been fully elucidated, many potential molecular changes and mechanisms are barely known. Individual aging is generally considered to be at least partially associated with cell senescence in individuals. Cell senescence may be caused by various stimulating factors, including telomere shortening resulted from the replication of DNA ends, DNA damage, activity change in tumor suppressor genes and oncogenes, oxidative stress, inflammation, chemotherapeutic agent as well as the exposure to ultraviolet radiation and ionizing radiation, etc. Secretory proteome is one of the signs for cell senescence, which is a phenotype with significant increases in expression and secretion of different kinds of cytokines, also the most important environmental effect generated by senescent cells, called senescence-associated secretory phenotype (SASP). These secretory factors promote communications with adjacent cells and the immune system, and finally affect the fate of senescent cells. On the one hand, SASP may promote the development of tumor cells by secreting factors which accelerate angiogenesis, extracellular matrix remodeling or epithelial-mesenchymal transformation (EMT). On the other one hand, chronic inflammation induced by aging may lead to systematic immune disorder, causing tissue damage and degeneration associated with aging, and the occurrence of age-related diseases.

Current biological studies most focus on protein encoding parts in the genome, findings about non-protein coding parts are rare. In particular, the role of "dark matter" in genome-reverse transcription elements in senescence ares still unknown. Reverse transcription elements include long terminal repeat (LTR) and non-LTR retrotransposons. LTR retrotransposon includes endogenous retrovirus (ERV), which is the relic of ancient retroviral infection in human and integrated into human genome, and has been fixed in human genome during evolution. Human endogenous retrovirus (HERV) accounts for approximately 8% of human genome.

The inventors found that the activation of endogenous retrovirus locus was related to cell and individual aging, which was a novel type of SASP molecule, may be used as a biological marker for individual and cell senescence, as well as a target for the intervention of aging and related diseases.

### Summary

In the first aspect, the invention provides a method for identifying the degree of aging of the subject or a cell, tissue or organ thereof, or evaluating the physical age of the subject, or diagnosing aging-related diseases, e.g., progeroid diseases and frailty, or assessing the degree of senescence of isolated animal cell or cell population, comprising
a) detecting the activation level of endogenous retrovirus (ERV) in the subject's sample or the isolated animal cell; and
b) comparing the detection result with the reference value of the activation level of the ERV

In some embodiments, the ERV is selected from the group comprising families HERVK (including HERVK11), MER61, MER61C, Charlie4, MER101B, MamGyp, HERV16, HUERS-P2 and ERVW and homologs thereof. In some embodiments, the reference value is obtained by detecting reference subject or reference cell. In some embodiments, the reference value is obtained by detecting a population of reference subjects or a population reference cells.

In some embodiments, the activation level of the ERV includes the methylation level of the ERV locus, the level of an ERV transcript, the level of an ERV protein, the presence, localization of the ERV particles in the cell, and/or the release the ERV particles from the cell, and/or the transposition probability of the ERV locus. In some embodiments, the activation level of the ERV includes the methylation level of the ERV locus, the level of an ERV transcript, the level of an ERV protein, and the presence, localization of the ERV particles in the cell, and/or the release the ERV particles from the cell. In some embodiments, the activation level of the ERV includes the transposition probability of the ERV locus. In some embodiments, the activation level of the ERV is presented by the localization of ERV particles in the cell.

In some embodiments, the subject or animal is a mammal. In some embodiments, the subject or animal is a non-human mammal. For example, the subject or animal is a mouse, and optionally, the ERV is from MMTV family; or the subject or animal is a non-human primate, and optionally, the ERV is from ERVW family. Preferably, the subject or animal is a human, and optionally, the ERV is from HERVK family.

In some embodiments, the sample is a body fluid sample, including but not limited to urine, saliva and blood. In some embodiments, the blood includes but is not limited to whole blood, plasma or serum. In some embodiments, the sample comprises tissues from the subject or animal. In some embodiments, the tissues include but are not limited to skin, lung, liver tissues. In some embodiments, the sample comprises a cell from the subject or animal. In some embodiments, the cell includes but is not limited to fibroblast and mesenchymal stem cell.

In the second aspect, the invention provides a method for treating premature aging in a subject in need or a cell, tissue of organ thereof, or preventing or delaying the aging of a subject or a cell, tissue of organ thereof.

In some embodiments, the method comprises administering an agent inhibiting the activation of an ERV locus or an agent inhibiting the ERV to the subject. In some embodiments, the ERV is selected from the group comprising families HERVK (including HERVK11), MER61, MER61C, Charlie4, MER101B, MamGyp, HERV16, HUERS-P2 and ERVW and homologs thereof.

In some embodiments, the agent inhibiting the activation of the ERV locus increases the methylation level of the ERV locus, reduces the transcript level of the ERV locus, reduces the protein level of the ERV locus, reduces the presence of the ERV particles in the cell and/or the release of the ERV particles from the cell.

In some embodiments, the subject or animal is a mammal. In some embodiments, the subject or animal is a non-human mammal. For example, the subject or animal is a mouse, and optionally, the ERV is from MMTV family; or the subject or animal is a non-human primate, and optionally, the ERV is from ERVW family. Preferably, the subject or animal is a human, and optionally, the ERV is from HERVK family.

In some embodiments, the method includes administering retrovirus inhibitor to the subject, e.g., reverse transcriptase inhibitor, integrase inhibitor and/or protease inhibitor.

In the third aspect, the invention provides a method for regulating cell senescence, including contacting the cell with an agent regulating the activation of an ERV locus.

In some embodiments, the ERV is selected from the group comprising families HERVK (including HERVK11), MER61, MER61C, Charlie4, MER101B, MamGyp, HERV16, HUERS-P2 and ERVW and homologs thereof.

In some embodiments, the regulating agent is an agent inhibiting the activation of the ERV locus or the agent inhibiting the ERV. In some embodiments, the agent inhibiting the activation of the ERV locus increases the methylation level of the ERV locus, reduces the transcript level of the ERV locus, reduces the protein level of the ERV locus, reduces the presence of the ERV particles in the cell and/or the release of the ERV particles from the cell.

In some embodiments, the regulating agent is an agent promoting the activation of the ERV locus in the cell. In some embodiments, the agent promoting the activation of the ERV locus reduces the methylation level of the ERV locus, increases the transcript level of the ERV locus, increases the protein level of the ERV locus, increases the presence in and/or the release from the cell, of the ERV particles.

In some embodiments, the subject or animal is a mammal. In some embodiments, the subject or animal is a non-human mammal. For example, the subject or animal is a mouse, and optionally, the ERV is from MMTV family; or the subject or animal is a non-human primate, and optionally, the ERV is from ERVW family. Preferably, the subject or animal is a human, and optionally, the ERV is from HERVK family.

In the fourth aspect, the invention provides a method for culturing isolated animal cell or cell population or preventing or delaying cell senescence.

In some embodiments, the method comprises contacting the cell with an agent inhibiting the activation of an ERV locus in the cell or an agent inhibiting the ERV. In some embodiments, the ERV is selected from the group comprising families HERVK (including HERVK11), MER61, MER61C, Charlie4, MER101B, MamGyp, HERV16, HUERS-P2 and ERVW and homologs thereof.

In some embodiments, the agent inhibiting the activation of the ERV locus increases the methylation level of the ERV locus, reduces the transcript level of the ERV locus, reduces the protein level of the ERV locus, reduces the presence of the ERV particles in the cell and/or the release of the ERV particles from the cell.

In some embodiments, the subject or animal is a mammal. In some embodiments, the subject or animal is a non-human mammal. For example, the subject or animal is a mouse, and optionally, the ERV is from MMTV family; or the subject or animal is a non-human primate, and optionally, the ERV is from ERVW family. Preferably, the subject or animal is a human, and optionally, the ERV is from HERVK family.

In some embodiments, the method comprises contacting the cell and a retrovirus inhibitor. In some embodiments, the retrovirus inhibitor comprises a reverse transcriptase inhibitor, an integrase inhibitor and/or a protease inhibitor.

In the fifth aspect, the invention provides a method for promoting cell senescence.

In some embodiments, the method comprises contacting the cell with an agent promoting the activation of an ERV locus in the cell or the ERV. In some embodiments, the ERV is selected from the group comprising families HERVK (including HERVK11), MER61, MER61C, Charlie4, MER101B, MamGyp, HERV16, HUERS-P2 and ERVW and homologs thereof.

In some embodiments, the regulating agent is an agent promoting the activation of the ERV locus in the cell. In some embodiments, the agent promoting the activation of the ERV locus reduces the methylation level of the ERV locus, increases the transcript level of the ERV locus, increases the protein level of the ERV locus, increases the presence of the ERV particles in the cell and/or the release of the ERV particles from the cell.

In some embodiments, the cell is a mammal cell. In some embodiments, the cell is a non-human mammal cell. For example, the cell is a mouse cell, and optionally, the ERV is from MMTV family; or the cell is a non-human primate cell, and optionally, the ERV is from ERVW family. Preferably, the cell is a human cell, and optionally, the ERV is from HERVK family.

In some embodiments, the method comprises contacting the cell with a DNA methyltransferase inhibitor, e.g., decitabine, Lomeguatrib, SGI-1027 or 5-azacytidine. In some embodiments, the cell is a mammal cell, e.g., mouse cell, non-human primate cell and human cell.

In the sixth aspect, the invention provides a method for preventing or delaying tissue and organ degeneration, treating related diseases, or delaying the aging of body, for example, a therapeutic method for preventing or delaying skin aging, treating or preventing osteoarthritis and frailty.

In some embodiments, the method comprises administering a retrovirus inhibitor to the subject in need. In some embodiments, the retrovirus inhibitor comprises a reverse transcriptase inhibitor, an integrase inhibitor and/or a protease inhibitor.

In some embodiments, the method comprises administering an agent inhibiting the activation of HERVK locus or an agent inhibiting HERVK to the subject in need. In some embodiments, the agent inhibiting the activation of the HERVK locus increases the methylation level of the HERVK locus, reduces the transcript level of the HERVK locus, reduces the protein level of the HERVK locus, reduces the presence of the ERV particles in the cell and/or the release of the ERV particles from the cell.

In some embodiments, the administration is topical administration, intradermal administration or subcutaneous administration.

In the seventh aspect, the invention provides a method for treating progeroid diseases, e.g., Hutchinson-Gilford progeria syndrome (HGPS) or Werner syndrome (WS).

In some embodiments, the method comprises administering an agent inhibiting the activation of HERVK locus or an agent inhibiting HERVK to the subject in need. In some embodiments, the agent inhibiting the activation of the HERVK locus increases the methylation level of the HERVK locus, reduces the transcript level of the HERVK locus, reduces the protein level of the HERVK locus, reduces the presence of the ERV particles in the cell and/or the release of the ERV particles from the cell.

In some embodiments, the method comprises administering a retrovirus inhibitor to the subject in need. In some embodiments, the retrovirus inhibitor comprises a reverse transcriptase inhibitor, an integrase inhibitor and/or a protease inhibitor.

### Description of drawings

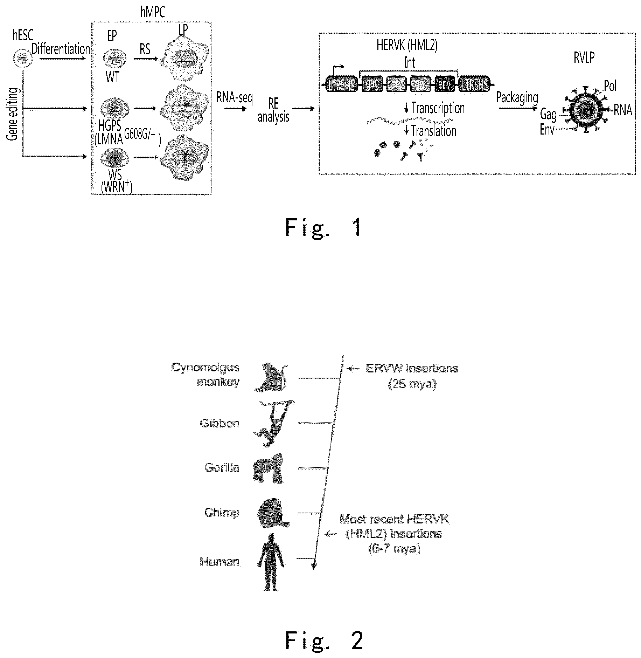

Fig. 1: the left panel is a schematic diagram of prematurely senescent cell model; the right panel is a schematic diagram of HERVK activation (including transcription, translation, package and release).

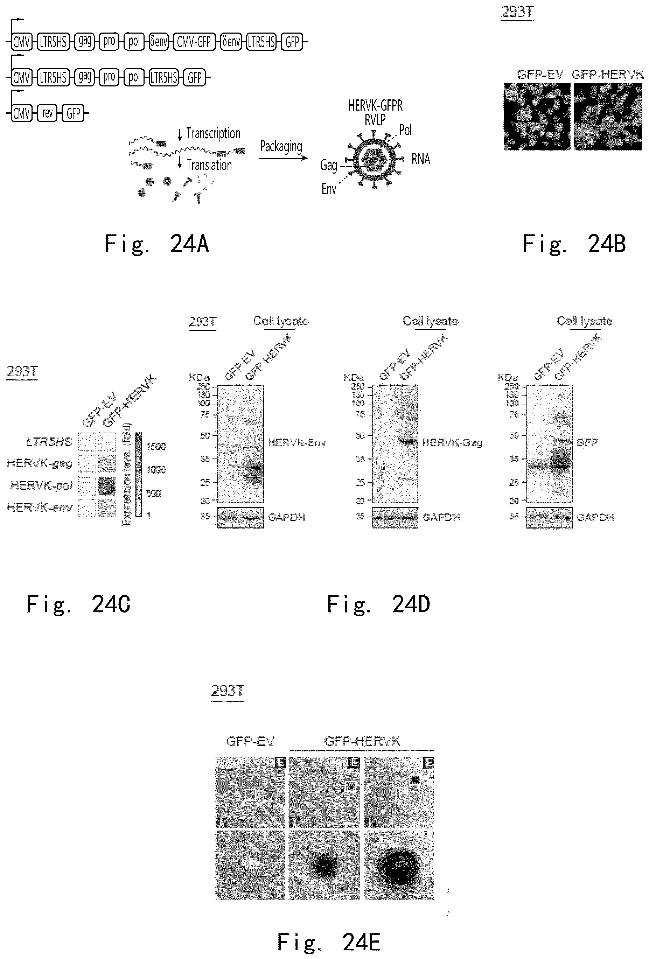

Fig. 2: a schematic diagram illustrating the incorporation of ERVW family and ERVK family into the genome during the evolution of primates.

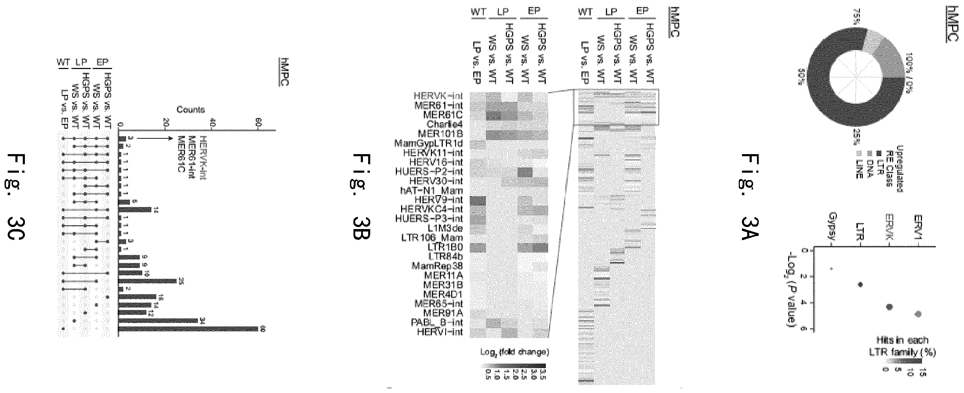

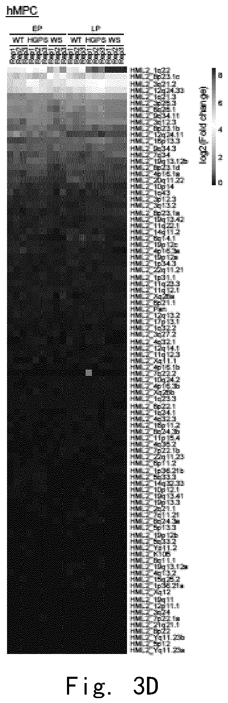

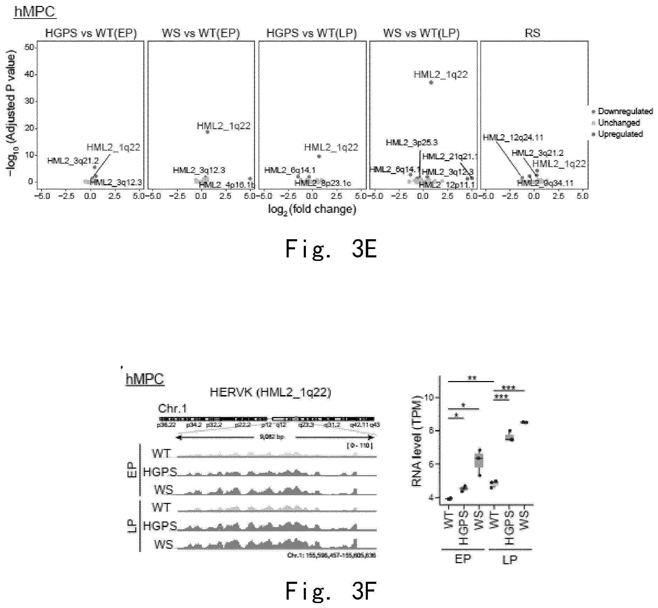

Fig. 3A, the left panel: ring plot, showing repetitive elements (RE) co-up-regulated in premature aging and the composition of replicatively senescent (RS) hMPCs classified by each repetitive element category. At least three up-regulated REs were considered in comparison (see Fig. 3C for the magnified RE). The right panel: family enrichment analysis of co-up-regulated RE of LTR type in prematurely senescent and RS hMPCs. Fig. 3B is a heat map showing the relative expression level (log2(fold-change)) of repetitive elements annotated by RepeatMasker in prematurely senescent and RS hMPCs. Fig. 3C is a bar diagram showing overlapping graph of repetitive elements annotated by RepeatMasker in prematurely senescent and RS hMPCs. Fig. 3D is a heat map showing the relative expression level of all provirus sequences of HERVK in prematurely senescent hMPC, color keys from blue to red represent expression levels from low to high. Fig. 3E is a volcano plot showing the differential expression of all provirus sequences of HERVK in prematurely senescent hMPCs. Fig. 3F shows up-regulation in RNA level of HERVK in provirus HML2_1q22 in prematurely senescent hMPCs.

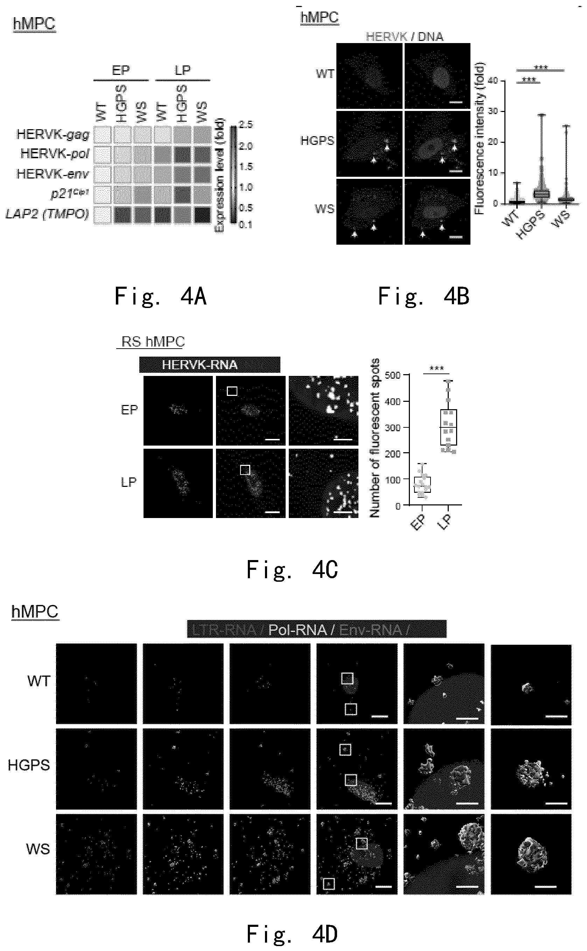

Fig. 4Ais a heat map showing the RNA levels of HERVK detected by RT-qPCR; Fig. 4B, the left panel is a representative image of HERVK RNA-FISH, the right panel is a quantitative analysis of fluorescence signal intensity.

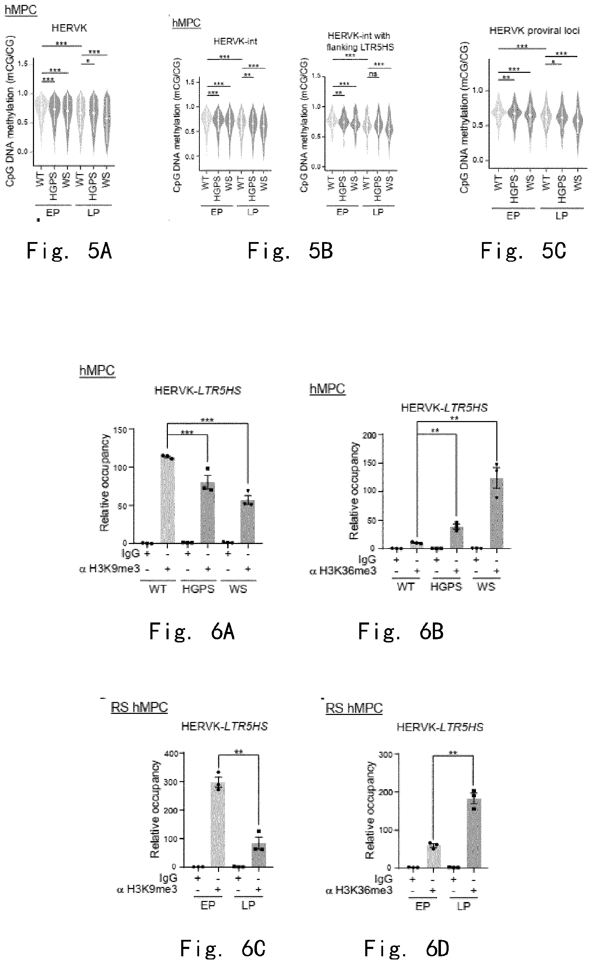

Fig. 5 is a violin plot showing the CpG DNA methylation level in HERVK.
Figs. 6A and B are the results of H3K9me3 and H3K36me3 enrichments of HERVK-LTR5HS region in WT, HGPS and WS hMPCs evaluated by ChIP-qPCR, n=3, data are presented as mean ± SEM; Figs. 6C and D are the results of H3K9me3 and H3K36me3 enrichments in HERVK-LTR5HS region in RS evaluated by ChIP-qPCR, n=3, data are presented as mean ± SEM.

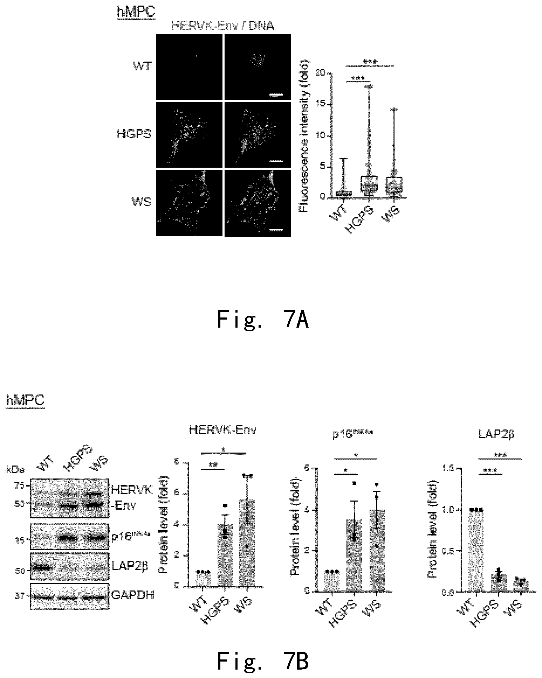

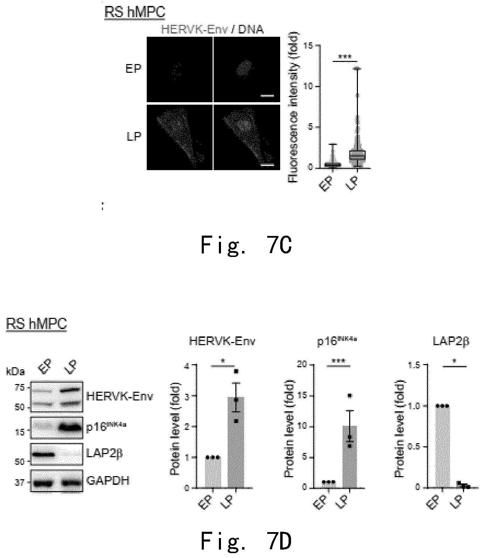

Fig. 7A is the result of immunofluorescent staining of HERVK-Env in WT, HGPS and WS hMPCs; Fig. 7B is the result of Western blot of BERVK-Env, p16^{INK4a} and LAP2 in WT, HGPS and WS hMPCs. Fig. 7C is the result of immunofluorescent staining of HERVK-Env in RS hMPC; Fig. 7D is the result of Western blot of HERVK-Env, p16INK4a and LAP2 in RS hMPCs.

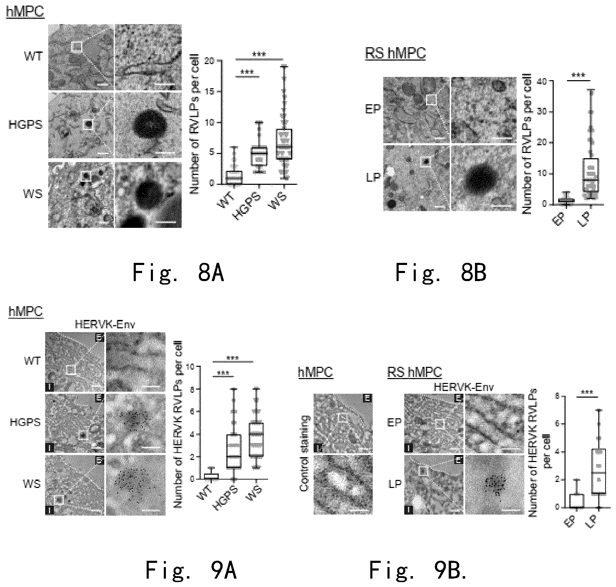

Fig. 8 shows the RVLP in WT, HGPS and WS hMPCs with heavy metal staining, the left panel is a TEM image, the right panel is the counting result of RVLP, RVLPs in HGPS, WS and RS hMPCs are significantly increased.
Fig. 9 shows the result of TEM analysis for immuno-gold labeled HERVK-Env. Fig. 9A, the left panel is a TEM image of WT, HGPS and WS hMPCs with a scale bar of 200 nm (left) and 100 nm (right), dotted lines indicate cytomembrane, E: extracellular, I: intracellular; the right panel is the result of statistical analysis. Fig. 9B, the left panel is a control TEM without adding primary antibody with a scale bar of 200 nm (top) and 100 nm (bottom), dotted lines indicate cytomembrane, E: extracellular, I: intracellular; the middle panel is a TEM image of RS hMPCs with a scale bar of 200 nm (left) and 100 nm (right), dotted lines indicate cytomembrane, E: extracellular, I: intracellular; the right panel is the result of statistical analysis.

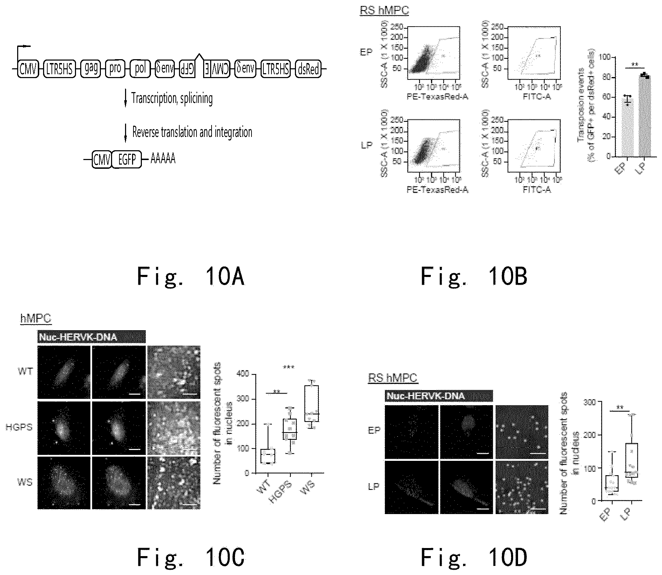

Fig. 10 shows the analysis of the transposition probability occurred for HERVK. Fig. 10A is a schematic diagram of constructing a vector for detecting HERVK transposition probability; Fig. 10B shows HERVK transposition probability in RS hMPCs with flow cytometry, the ratio of green fluorescence to red fluorescence represents transposition probability; Fig. 10C shows HERVK copy number in the genome detected in WT HGPS and WS hMPCs by single-molecule DNA-FISH; Fig. 10D shows HERVK copy number in the genome detected in RS hMPCs by single-molecule DNA-FISH.

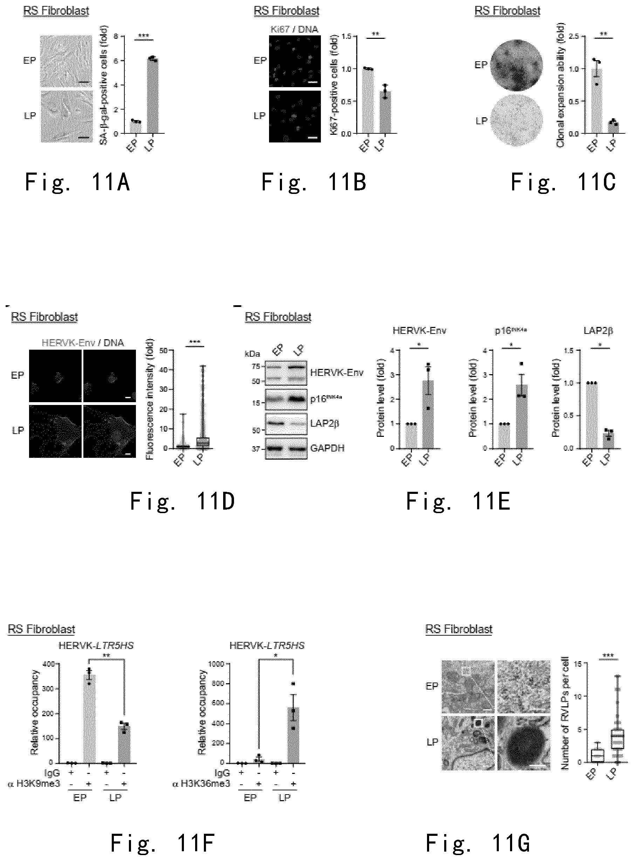

Fig. 11 shows the epigenetic derepression of HERVK in RS fibroblast.

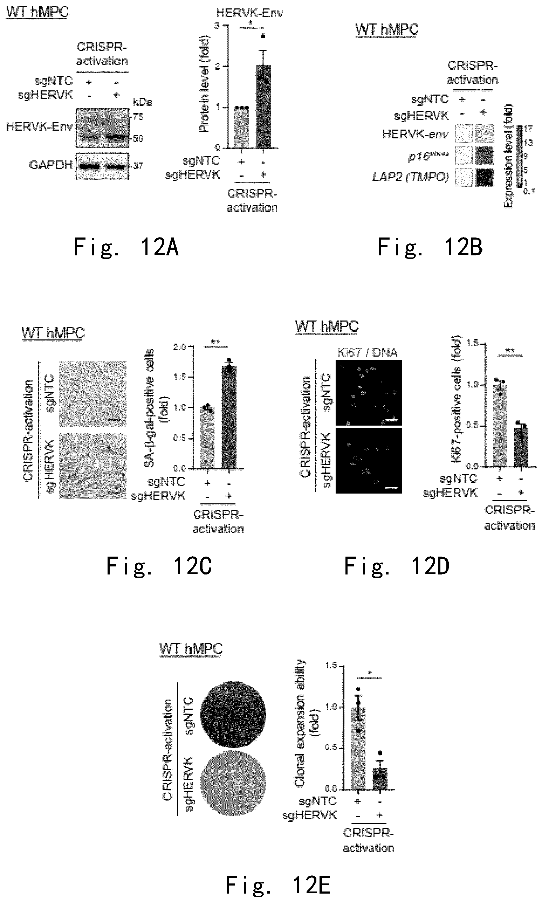

Fig. 12 shows the increase of HERVK-Env protein level upon systematic activation by CRISPR-dCas (12A); and that heat map of qPCR (12B) shows that HERVK-Env expression is up-regulated, and the expression of a positive mark for senescence p16INK4a is up-regulated and the expression of a negative mark LAP2 is down-regulated; SA-β-gal staining (12C), Ki67 staining (12D) and clonal amplification ability (12E) reveal cell senescence.

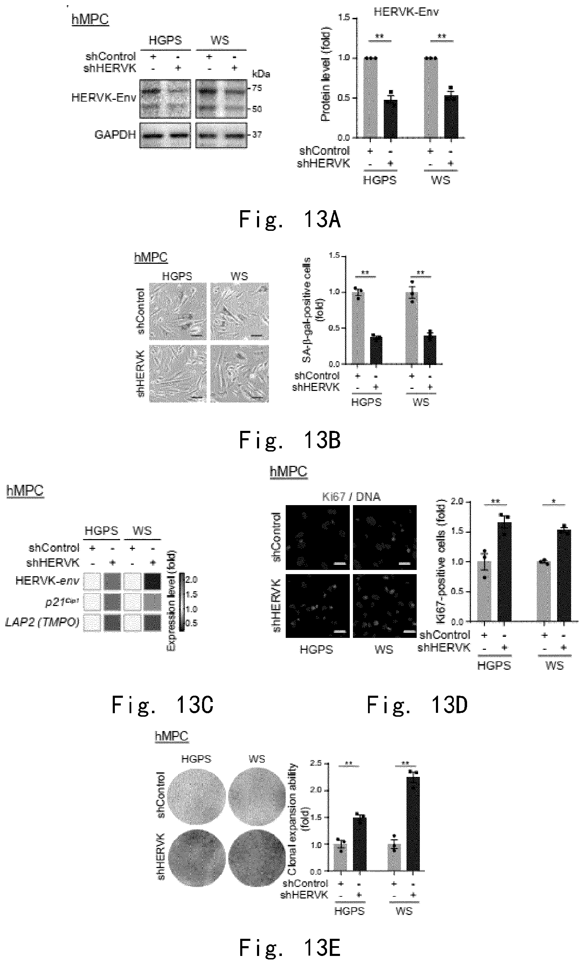

Fig. 13 shows HERVK knockdown by shRNA in HGPS and WS hMPCs reduces senescence phenotype.

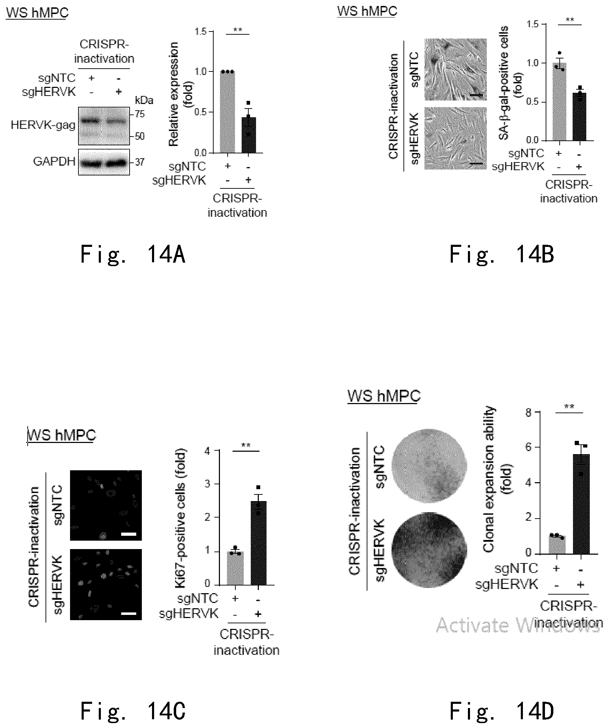

Fig. 14 shows the decrease of HERVK-Env protein level upon systematic suppression by Cas9KRAB (14A), and SA-β-gal staining (14B), Ki67 staining (14C) and clonal amplification ability (14D) reveal that cell senescence is delayed.

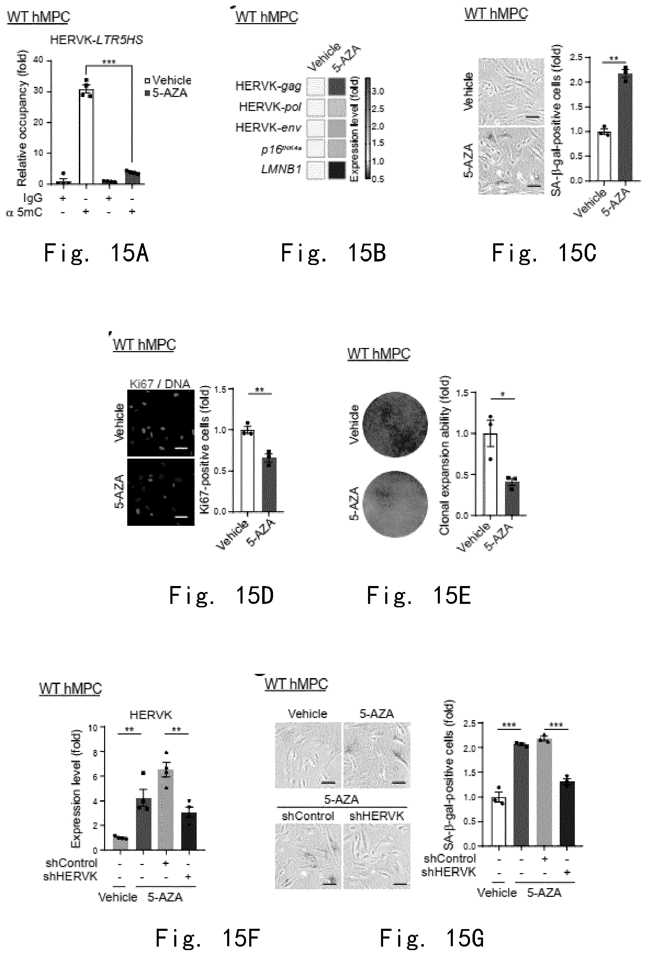

Fig. 15 shows that 5-AZA treatment leads to decrease in HERVK-LTR5HS methylation (14A), causes HERVK activation (increase in HERVK RNA level, 14B), induces senescence (14B-E), while knockdown of HERVK (14F) effectively reduces 5-AZA induced senescence phenotype (decrease in SA-β-gal stained cells, 14G).

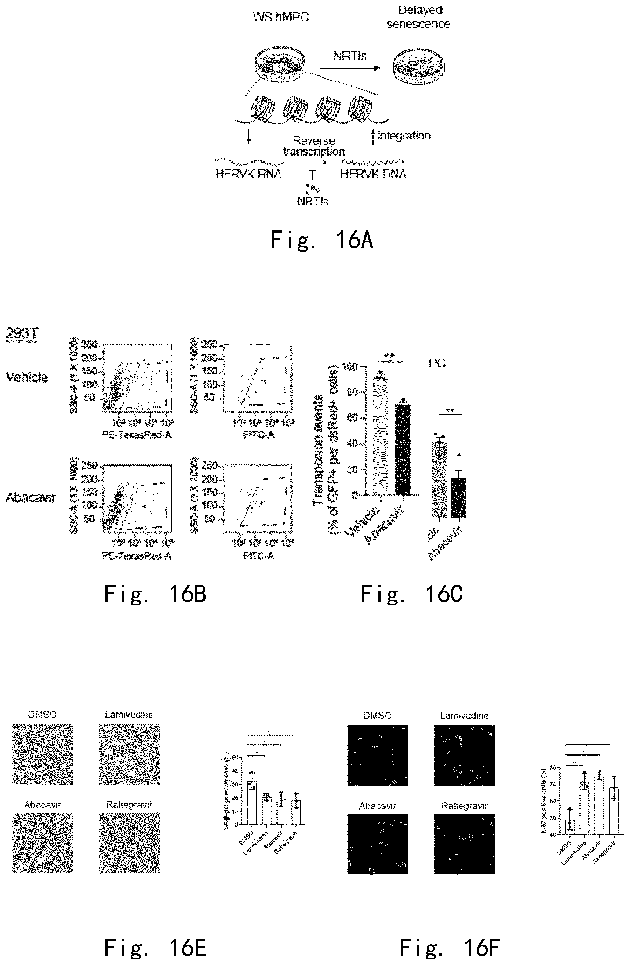

Fig. 16 shows HERVK reverse transcriptase inhibitor and integrase inhibitor delay cell senescence. Fig. 16A is a schematic diagram of HERVK life cycle, in which reverse transcriptase and integrase can be suppressed by small molecules; Fig. 16B is a flow cytometry showing that reverse transcriptase inhibitor Abacavir reduces transposition efficiency of HERVK; Fig. 16C shows that reverse transcriptase inhibitor Abacavir decreases DNA copy number of HERVK; reverse transcriptase inhibitors Abacavir, Lamivadine and integrase inhibitor Ratelgravir reduce the number of cells positive for SA-β-gal staining (Fig. 16C), and increase Ki67 positive cell number (Fig. 16D).

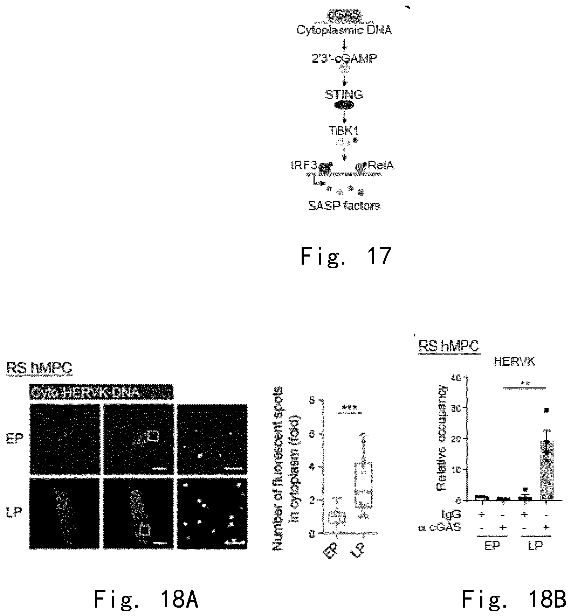

Fig. 17 is a schematic diagram of cGAS pathway of induced innate immune response.
Fig. 18 shows cGAS enrichment on cytosol HERVK DNA analyzed by immunoprecipitation and qPCR.

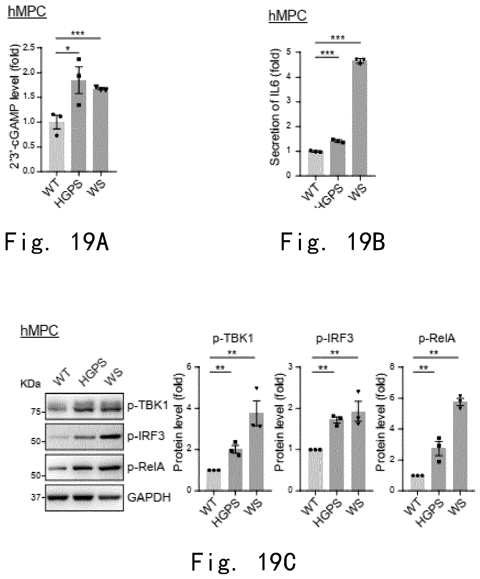

Fig. 19 shows the activation of cGAS pathway in prematurely senescent hMPC, inducing innate immune response. Fig. 19A shows ELISA on 2'3'-cGAMP level in WT, HGPS and WS hMPCs; Fig. 19B shows ELISA analysis of IL-6 level in media for WT, HGPS and WS hMPCs; Fig. 19C shows Western blot of p-TBK1, p-IRF3 and p-RelA in WT, HGPS and WS hMPCs.

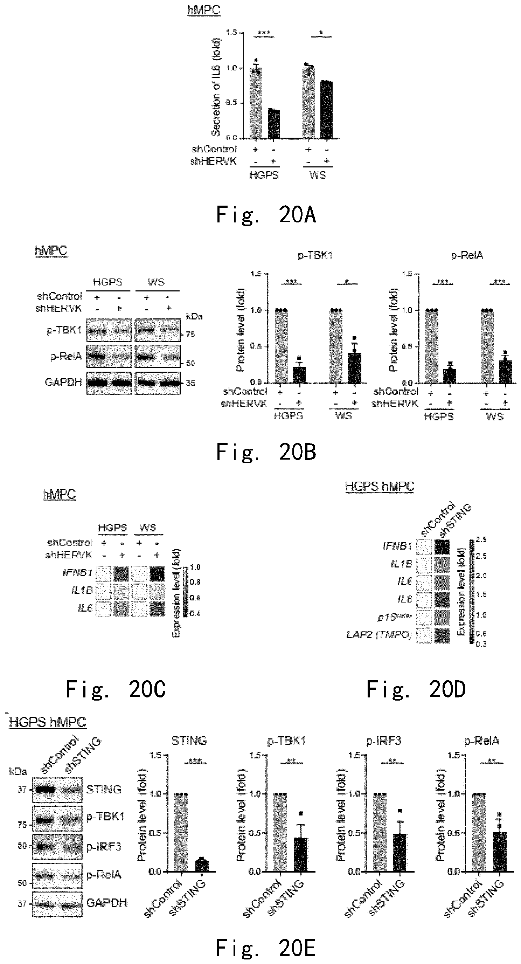

Fig. 20 shows that the knockdown of HERVK inhibits cGAS pathway in HGPS and WS hMPCs as well as the innate immune response. The result of HERVK knockdown (Figs. 20A and B) exhibits consistent trend with that of STING knockdown (Figs. 20C-E).

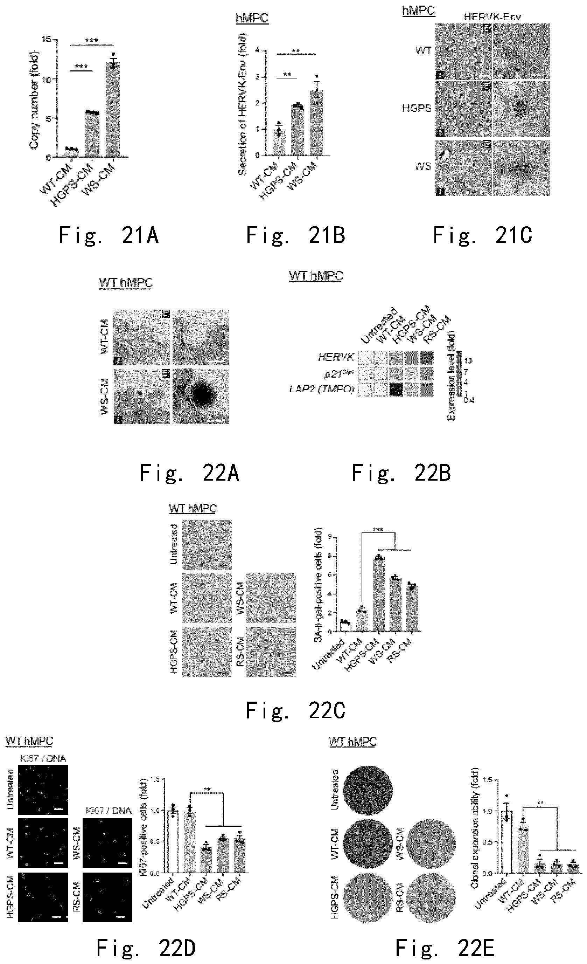

Fig. 21A shows HERVK DNA copy number in WT, HGPS and WS hMPCs by ddPCR; Fig. 21B shows HERVK-Env level in media from WT, HGPS and WS hMPCs by ELISA; Fig. 21C shows immuno-gold labeled TEM images of WT, HGPS and WS hMPCs (scale bar of 200 nm (left) and 100 nm (right), dotted lines indicate cytomembrane, E: extracellular, I: intracellular).
Fig. 22 shows that incubation with old CM induces senescence of young hMPCs.

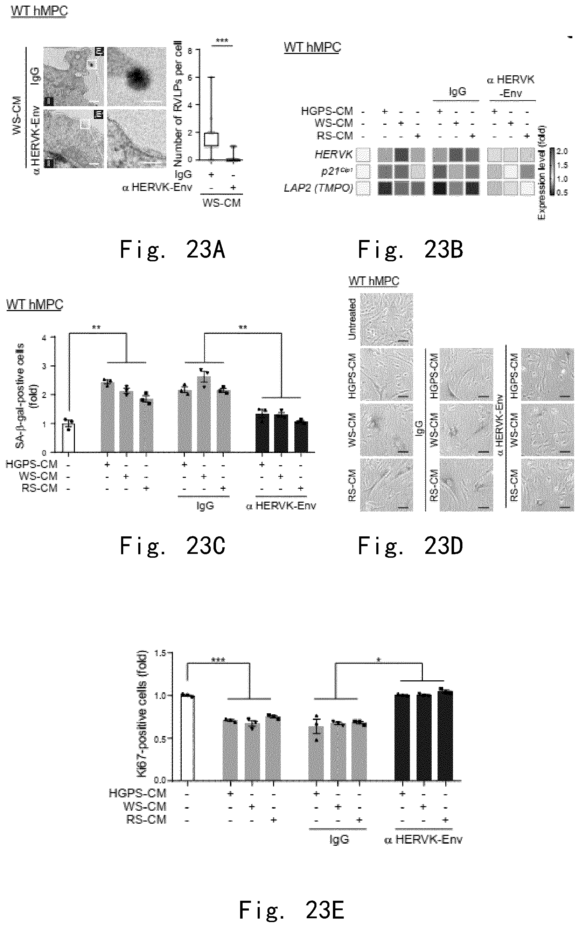

Fig. 23 shows that the ability of old CM to induce senescence is decreased after removing HERVK particles therein.
Fig. 24 shows the *in vitro* synthesis and purification of HERVK live virus carrying GFP. 293T cells are transfected with HERVK vector carrying GFP, and all of the HERVK viral RNA, viral protein and viral particle can be detected (Figs. 24B-E).

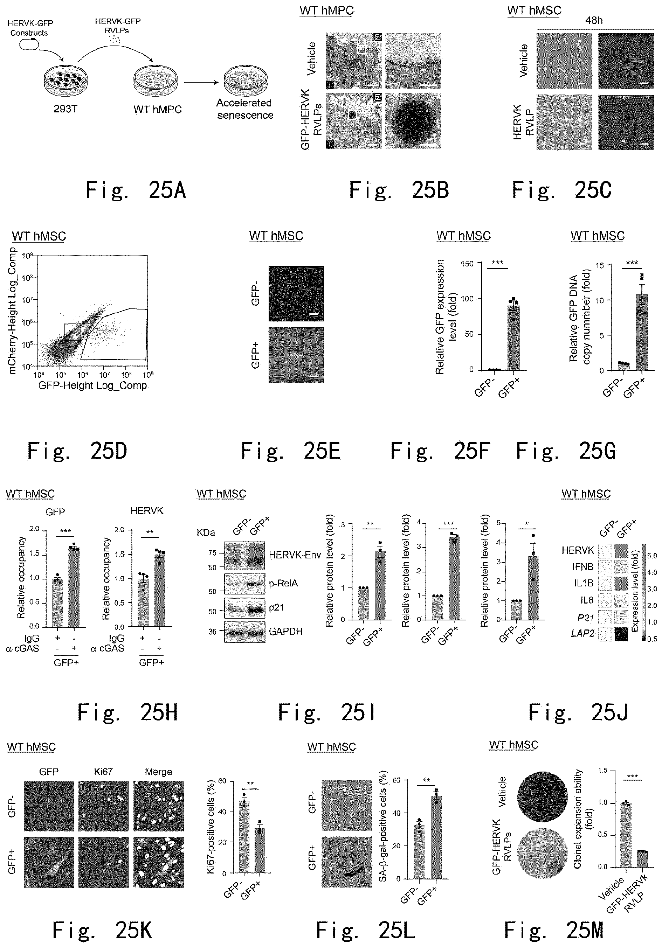

Fig. 25 shows that upon the infection of hMPCs with purified HERVK live virus carrying GFP, HERVK virus can enter the target cell (Figs. 25B-G), activate innate immune pathway and the up-retulation of the expression of inflammatory factors (Figs. 25H-J), and lead to senescence of hMPCs (Figs. 25K-M).

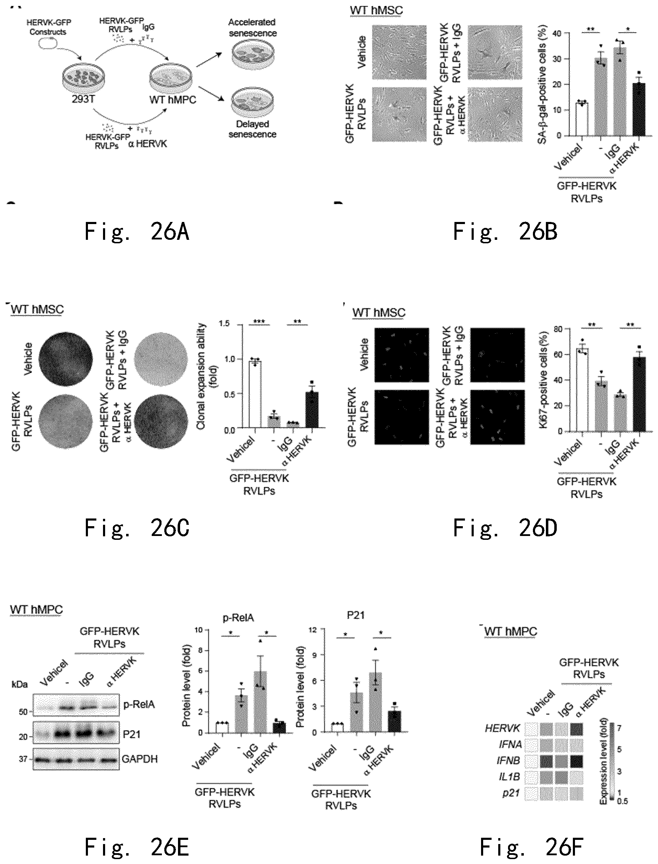

Fig. 26 shows that the innate immune response and the expression of inflammatory factors (Figs. 26E-F) can be reduced by neutralizing HERVK live virus with HERVK antibody, alleviating senescence of hMPCs (Figs. 26B-D).

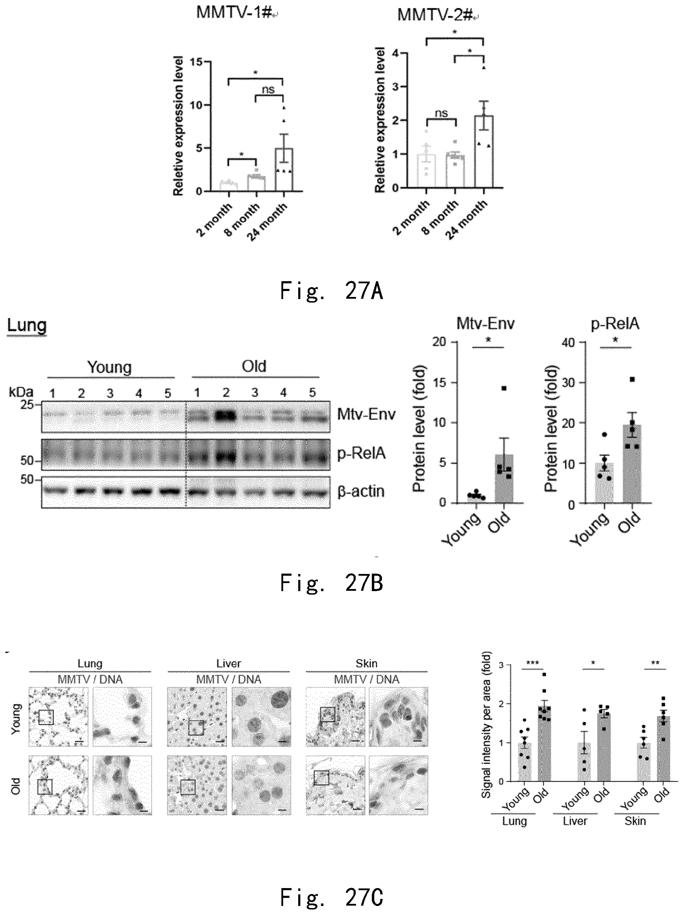

Fig. 27A shows the RT-qPCR analysis of MMTV in liver tissues from young and old mice; Fig. 27B shows the Western blot of MMTV-Env and p-RelA in liver tissues from young and old mice; Fig. 27C shows the quantitative analysis of immunohistochemical staining of MMTV-Env in liver tissues from young and old mice.

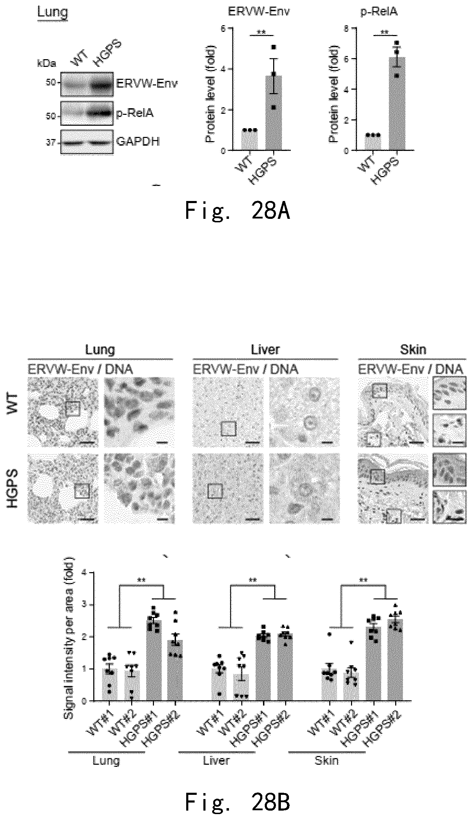

Fig. 28A shows the Western blot of ERVW-Env and p-RelA in lung tissues from HGPS and WT cynomolgus monkeys; Fig. 22B shows the quantitative analysis of immunohistochemical staining of ERVW-Env in lung, skin and liver tissues from HGPS and WT cynomolgus monkeys; Fig. 22C shows the quantitative results of Western blot of ERVW-Env in lung tissues from young and old cynomolgus monkeys; Fig. 22D shows the quantitative analysis of immunohistochemical staining of ERVW-Env in lung, skin and liver tissues from young and old cynomolgus monkeys.

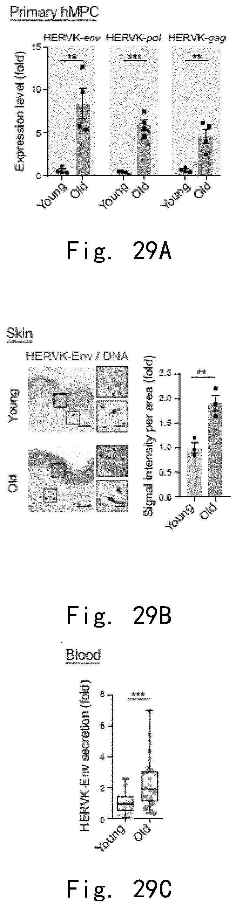

Fig. 29A shows the RT-qPCR results of HERVK gene in primary hPMC from young and old people; Fig. 29B the shows immunohistochemical staining of HERVK-Env in skin tissues from young and old people, the left panel is a representative image, the right panel is a statistical analysis; Fig. 29C shows ELISA of HERVK in sera from young and old people.

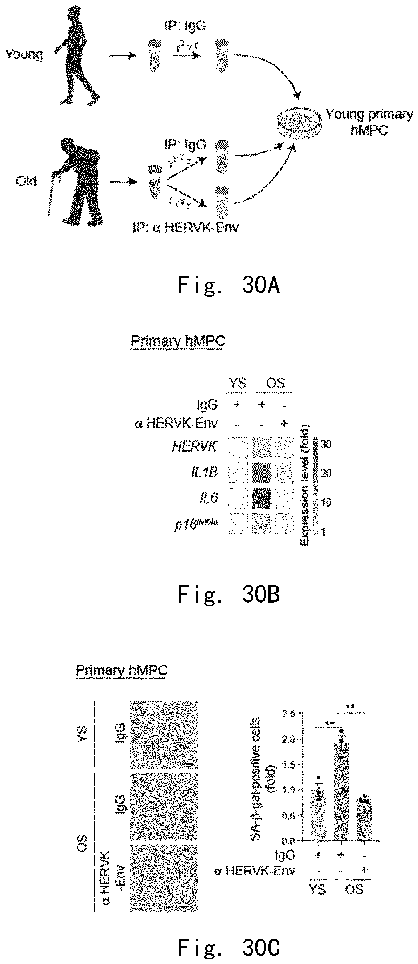

Fig. 30A shows a schematic diagram of culturing primary hPMC from young people with sera from young and old people as well as serum from old people with HERVK removed; Fig. 30B shows the RT-qPCR of inflammatory factors in primary hPMC from young people cultured with sera from young and old people as well as serum from old people with HERVK removed; Fig. 30C shows the SA-β-gal analysis of primary hPMC from young people cultured with sera from young and old people as well as serum from old people with HERVK removed.

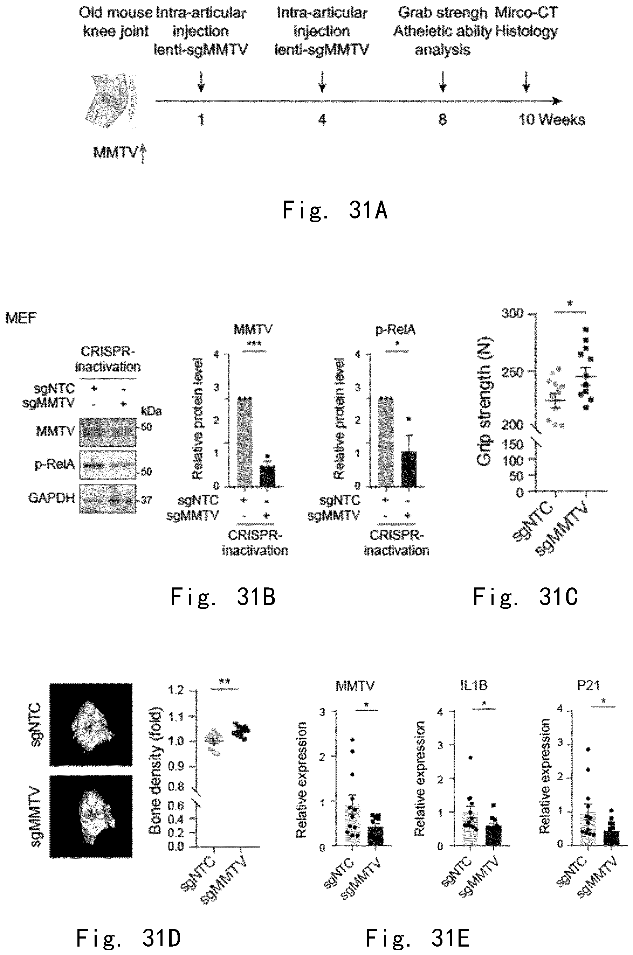

Fig. 31A shows an experimental flowchart of injecting lentivirus inhibiting MMTV (the lentivirus encodes sgRNA targeting MMTV) to the joint of old mice; Fig. 31B shows that upon systematic suppression by Cas9KRAB, MMTV protein level is decreased; Fig. 31C shows the limb tension level test of mice 8 weeks after injection; Fig. 31D shows the bone density of joint detected by mini-NMR 10 weeks after injection; Fig. 31E shows the RT-qPCR of inflammatory factors in the joint 10 weeks after injection.

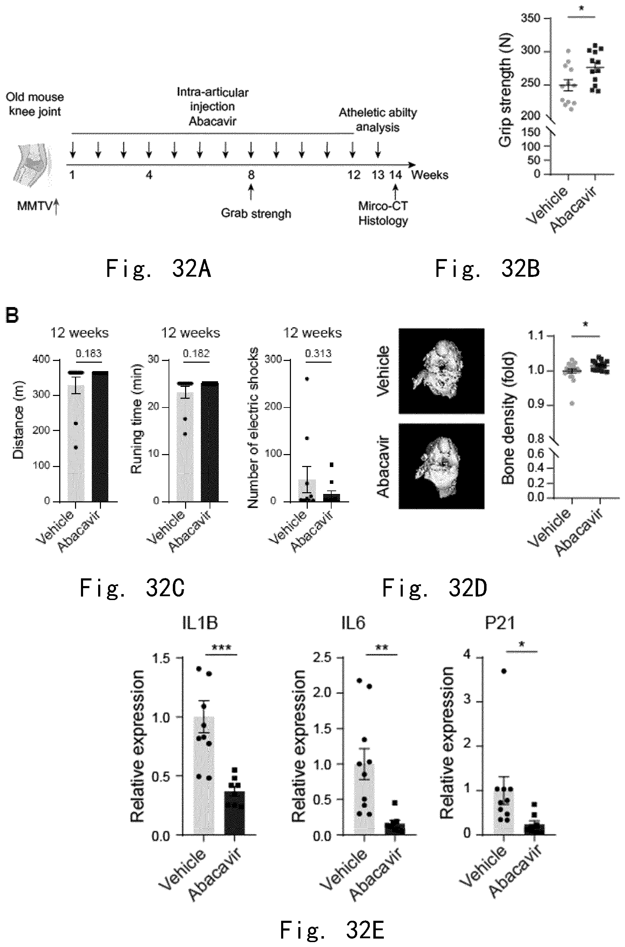

Fig. 32A shows an experimental flowchart of injecting reverse transcriptase inhibitor Abacavir to the joint of old mouse; Fig. 32B shows the limb tension level test of mice 8 weeks after injecting reverse transcriptase inhibitor Abacavir; Fig. 3C shows the running ability test of mice13 weeks after injecting reverse transcriptase inhibitor Abacavir; Fig. 32D shows the bone density of joint is detected by mini-NMR 14 weeks after injecting reverse transcriptase inhibitor Abacavir; Fig. 32D shows the RT-qPCR analysis of inflammatory factors in the joint after injecting reverse transcriptase inhibitor Abacavir.

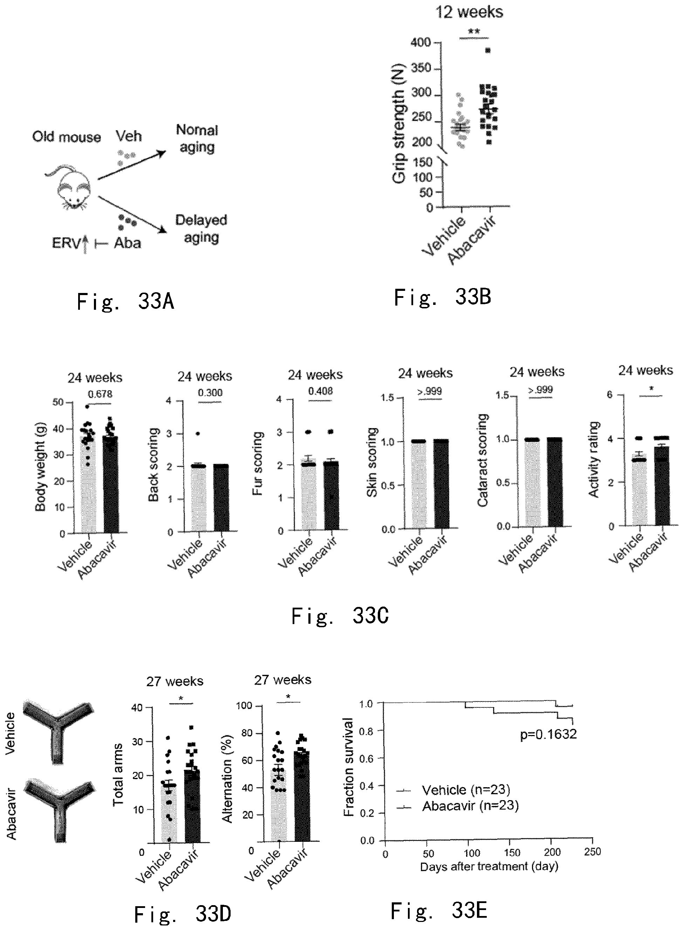

Fig. 33A shows a schematic diagram of daily administration of reverse transcriptase inhibitor Abacavir to old mice by dissolving the same in drinking water; Fig. 33B shows the limb tension level test of mice 12 weeks after administering reverse transcriptase inhibitor Abacavir; Fig. 33C shows the weight, skin, hair, hollow back, cataract and mobility test of mice 24 weeks after administering reverse transcriptase inhibitor Abacavir; Fig. 33D shows the survival curve after administering reverse transcriptase inhibitor to old mice.

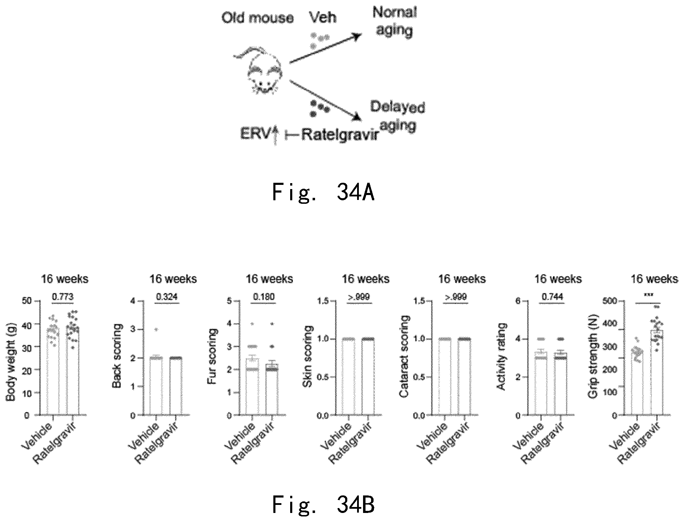

Fig. 34A shows a schematic diagram of administering integrase inhibitor Ratelgravir to old mice; Fig. 34B shows the limb tension level test of mice, as well as the weight, skin, hair, hollow back, cataract and mobility test of mice 16 weeks after administering reverse transcriptase inhibitor Abacavir.

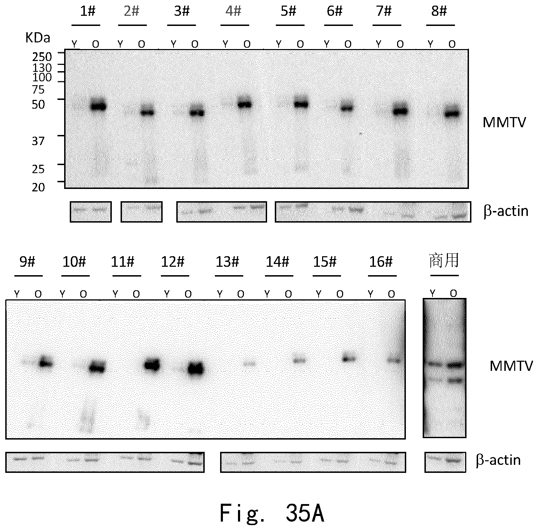

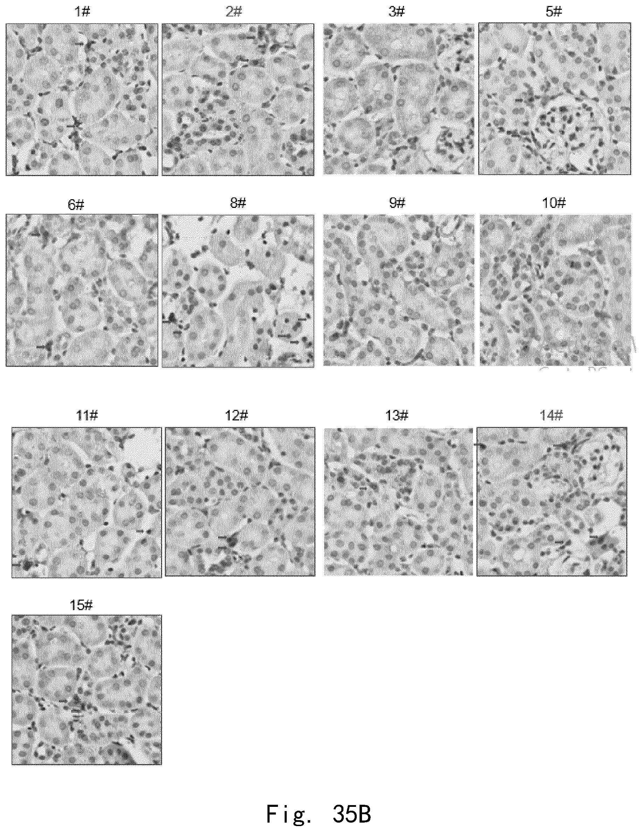

Fig. 35 shows results of western blot of MMTV-Env protein in mice (Fig. 35A) and immunohistochemical staining experiment of MMTV (Fig. 35B) with MMTV antibody.

### Detailed Description of the Invention

Although the invention is to be illustrated in conjuction with the following embodiments, it is understood that they are not meant to limit the invention to those embodiments. Instead, the invention is intended to encompass all replacements, modifications and equivalent forms within the scope of the invention defined by the claims. Those skilled in the art will recognize that many methods and materials similar or equivalent to the methods and materials described herein may be used to practice the invention. The invention is not limited to the described methods and materials. If one or more of the recited literature, patents and similar materials are different from or contradictory to the application, including but not limited to the defined terms, terminology, the described technology and the like, the application shall prevail. Unless otherwise defined, all technical and scientific terms used herein have the same meanings normally understood by ordinary skill in the field belonged to the invention. Although suitable methods and materials are described as follows, methods and materials similar or equivalent to those described herein are also used for practice or test of the invention.

### 1. Definition

As used herein, the term "and/or" encompasses all combinations of the items connected by the term, it shall be considered that each combination is individually listed herein. For example, "A and/or B" encompasses "A", "A and B" and "B". For example, "A, B and/or C" encompasses "A", "B", "C", "A and B", "A and C", "B and C" and "A, B and C".

Herein, the term "subject" means a non-human animal subject, e.g., non-human mammal, birds, reptiles, fishs, etc., including livestock, poultry, pet, racing animal, or a human subject.

"Aging" of a subject generally means the phenomenon that an organism is progressively decreased in the physical and/or mental adaptive ability to the environment of and gradually tends to be dead. Aging may be divided into physiological and pathological aging in which physiological aging means the physiological degeneration process occurred after mature, while pathological aging is senile changes caused by various external factors. However, aging is the result of combined effects of many pathological, physiological and psychological processes, in fact, physiological aging and pathological aging cannot be entirely distinguished in most instances.

The "degree of aging" of a subject is typically related to "physical age", means that the subject exhibits the level reflected by the physiology and the function thereof at a certain natural age, i.e., degree of performance of the physiology and the function thereof corresponding to a certain natural age.

Premature aging of a subject means physical age of a subject is significantly greater than the natural age, usually caused by factors such as environment, disease, and overwork. In some cases, premature aging includes hereditary progeria, e.g., Hutchinson-Gilford progeria syndrome (HGPS) and Werner syndrome (WS).

"Senescence" of a cell is regarded as the important factor for senescence of organism, and generally means a cell keeps active and metabolic activity, but loses proliferation capacity. Notable features for senescent cells include: (i) arrest of growth, (ii) enlargement and flattening in cell morphology, (iii) DNA damage lesion in cell nucleus, (iv) senescence-associated secretory phenotype (SASP), (v) senescence-associated elevated activity of β-galactosidase(SA-β-gal), (vi) increase in expression of tumor suppressor p16^{INK4a} and (vii) increase in number and size of PMI, nuclear bodies, etc.

The pathway led to senescence phenotype generally includes replicative senescence (RS), premature senescence and senescence after differentiation (SAD). "Replicative senescence" is the type of senescence occurred after many cell divisions. For example, when growing in culture, primary cell suffers from cell senescence after about 50 cell divisions. It is considered that such obstacle for further proliferation is due to the telomere shortening caused by each successive cell division, causing the cell to reach the point triggering DNA damage response (so called "Hayflick limit"), eventually led to induction of proliferation arrest and senescence. "Premature senescence" of cell means cell senescence without telomere deletion or dysfunction. Premature senescence can be caused by a variety of stimuli, including e.g., chemotherapy, radiotherapy, DNA damage, oxidative stress, inflammation, strong mitotic signaling and ribosome stress. In addition, a genetic deficiency in the cell will also lead to premature senescence of cell, for example, premature senescence will happen in cells in above-mentioned HGPS or WS patients. SAD means senescence-like phenotype exhibited by cells after the mitotic phase of terminal differentiation (including SASP), and may be induced by various stressors, including genetic toxicity, proteotoxicity, oxidation and ribosome stressor. Researches have shown that SAD are observed in some diseases. Herein, "the degree of senescence" of cell means senescence-associated phenotype thereof is comparable to the corresponding phenotype of cell with particular passage number.

An "endogenous retrovirus (ERV)" is a genomic sequence originated from retrovirus, which is generally regarded as the relic of retrovirus integrated into germline chromosome during evolution (such as millions of years ago). Although most ERVs are defective, and inactive in most instances, they may be activated under certain physiological and pathological conditions. For example, ERVW family was inserted into primate germline chromosome about 25 million years ago, while HERVK family was inserted into human germline chromosome about 6 million -7 million years ago. Researches have shown that in some cases, MMTV keeps active in rodents such as mouse. ERVW family still keeps active in non-human primates, such as an Old-World monkey, while HERVK family keeps active in human. ERV locus often includes flanking LTR zone and coding sequence.

Herein, "reference value" means index constant of normal subject or cell, tissue or organ thereof in a particular state (e.g., age or passage number), or representative physiological and biochemical characteristics such as morphology, function and metabolites for normal isolated cell or cell population, also called normal value. A reference value may be a predefined value, and also can be a value obtained by detecting reference subject or reference cell.

Herein, "reference subject" or "reference cell" means a subject or cell having above-mentioned representative characteristics. For example, a reference subject in the invention means e.g., a subject having representative characteristics for specific degree of aging/senescence or physical age, reference cell in the invention means e.g., the cell having representative characteristics for a specific passage number.

Herein, "locus" means a region on chromosome, including one or more genes, gene parts and/or regulatory sequences.

Herein, "homolog" of an ERV is an ERV that is phylogenetically homologous to the ERV in sequence or a homologous ERV of the same virus family originated from other species. For example, according to Stockinga & Kozak, Cell. Mol. Life Sci. 65 (2008) 3383-3398, HERVK is the homolog of MMTV in mice.

Herein, the "activation of an ERV locus" means the conversion of an ERV locus from silent state to a state capable of being transcribed, translated and even packaged and secreted as viral particles. An agent inhibiting/promoting the activation of an ERV locus encompasses any substance capable of inhibiting/promoting (including specifically inhibiting/promoting and non-specifically inhibiting/promoting) the activation of the ERV locus.

Herein, "non-specific retrovirus inhibitor" means a retrovirus inhibitor that is not specifically directed to a certain ERV locus.

As used herein, term "polynucleotide" or "nucleic acid molecule" includes DNA molecules (e.g., cDNA or genomic DNA) and RNA molecules (e.g., mRNA) as well as DNA or RNA analogs generated with nucleotide analogs. The nucleic acid molecule may be single-stranded or double-stranded, preferably double-stranded DNA. The nucleic acid can be synthesized with nucleotide analogs or derivatives (e.g., inosine or phosphorothioate nucleotide). Such nucleotide may be used to, for example, produce nucleic acid with changed base pairing ability or enhanced resistance to nuclease.

As used herein, the term "encode" means polynucleotides directly designate amino acid sequences of protein products thereof. The boundary of a coding sequence is generally defined by open reading frame, which is often started at ATG initiation codon or additional initiation codon such as GTG and TTG, and terminated at the stop codons TAA, TAG and TGA. The coding sequence may be a DNA, cDNA or recombinant nucleotide sequence.

As used herein, "antisense nucleic acid" means a nucleic acid molecule having complementary sequence with target nucleic acid (e.g., mRNA), which is involved in the regulation of gene expression by binding to the target nucleic acid in a base pairing manner. Antisense nucleic acid can include RNA or DNA molecules which are exactly complementary to particular mRNA, and specifically block its translation.

As used herein, "interfering nucleic acid" means RNA molecules encoded for RNA interference (RNAi), including siRNA, shRNA, miRNA and other nucleic acid molecules.

Herein, term "protein" means biomacromolecule consisting of one or more "polypeptide" chains. Polypeptide means a chain of amino acid residues connected by ten or more peptide bonds. All chemical formula or sequences for peptides and polypeptides herein are written from left to right, represent the direction from the amino terminal to the carboxyl terminal.

In the context of peptides, terms "amino acid", "residue" and "amino acid residue" can be used interchangeably, including naturally occurring amino acids in protein and non-natural amino acids. Naturally occurring amino acids in protein are named by single letter and three letters with common names in the art, see Sambrook, et al. (Molecular Cloning: A Laboratory Manual, 2nd, ed. Cold Spring Harbor Laboratory, Cold Spring Harbor Laboratory Press, Cold Spring Harbor, N.Y, 1989).

As used herein, "identity percent" means comparing amino acids of two polypeptides or nucleotides of two nucleic acid molecules, when in optimal alignment, the two polypeptides or two nucleic acid molecules have approximately specified the same percentage of amino acid or nucleotide. For example, "95% amino acid/nucleotide identity" means comparing amino acids of two polypeptides or nucleotides of two nucleic acid molecules, when in optimal alignment, the two polypeptides have 95% amino acid/nucleotide in common.

As used herein, an "isolated" cell means a cell separated from the original organism, generally refers to cells cultured *in vitro,* including but not limited to cells in adherent culture, cells in suspension culture or cells in 3D culture.

CRISPR/Cas system means clustered regularly interspaced short palindromic repeats and its related system, which can cut DNA chain in a specific position under the guidance of the guide RNA.

CRISPR/dCas system relates to the loss of nuclease activity by the modification of Cas, which is thus unable to cut DNA chain, meanwhile, connecting it to transcription activating domain to achieve the effect of site-specific transcriptional activation.

Also, CRISPR/dCas system can be used to regulate methylation level in the region of interest to regulate transcription. Specifically, dCas may be connected to a sequence promoting methylation, such as amino acid sequence with methyltransferase activity (e.g., CRISPR-dCas-SunTag-DNMT3A), to increase the methylation level in the region of interest, thus reducing transcription in the region of interest. dCas may also be connected with a sequence inhibiting methylation, such as Tet (ten-eleven translocation) catalytic domain (CRISPR-dCas-Tet), to reduce methylation level in the region of interest, thus reducing the transcription in the region of interest.

### 2. Method for identifying the degree of aging

The invention provides a method for identifying the degree of aging of a subject or a cell, tissue or organ thereof, or evaluating the physical age of a subject, or diagnosing progeroid diseases, comprising
a) detecting the activation level of endogenous retrovirus (ERV) in a sample from the subject; and
b) comparing the result of the detection with the reference value of the activation level of the ERV

Organisms often regulate ERV by, e.g., epigenetics. However, in some cases, ERV can bypass biological monitoring, so that its locus is activated due to changes in epigenetic regulation (e.g.,DNA methylation), and thus, the retroviral elements start to be transcribed, undergo retrotransposition, and Retrovirus-like particles (RVLP), which can infect cells, can even be generated and released.

The inventors surprisingly found that some ERV loci are activated during aging. In some embodiments, the ERV is selected from the group comprising families HERVK (including HERVK11), MER61, MER61C, Charlie4, MER101B, MamGyp, HERV16, HUERS-P2 and ERVW and homologs thereof. In some embodiments, the ERV is selected from the group comprising families HERVK (including HERVK11), MER61, MER61C, Charlie4, MER101B, MamGyp, HERV16, HUERS-P2, ERVW and MMTV and homologs thereof. In some embodiments, the ERV includes at least an ERV from HERVK family (including HERVK11), optionally, the ERV also includes ERV from the group comprising families MER61, MER61C, Charlie4, MER101B, MamGyp, HERV16, HUERS-P2 and ERVW and homologs thereof.

In some embodiments, the "activation level" of an ERV locus includes the methylation level of the ERV locus, the level of an ERV transcript, the level of an ERV protein, and/or the presence, localization of the ERV particles in the cell and/or the release of the ERV particles from the cell. A lower methylation level, a higher transcript level, a higher protein level and/ or a higher presence of the ERV particles in the cell and/or the release of the ERV particles from the cell represent a higher activation level of the ERV locus. In some embodiments, the activation level of an ERV locus includes the transposition probability of the ERV locus. A higher transposition probability represents a higher activation level.

In some embodiments, the reference value is obtained by detecting reference subject. In the invention, reference subject means a subject with a specific biological age. Desired subjects can be determined by detecting representative characteristics related with physical age (e.g., selected from characteristics such as brain activity, skin elasticity, reflex action and balance). Due to individual differences, preferably, the reference value is obtained by detecting a population of reference subjects.

According to the invention, a statistically significant number of normal subjects in a specific natural age (e.g., 20, 30, 40, 50, 60, 70, 80, 90 years old or elder) or a range of natural ages (e.g., 18-22 age, 28-32 age, 38-42 age, 48-52 age, 58-62 age, 68-72 age, 78-82 age, 88-92 age, etc.) can also be selected as reference subjects.

The reference value may be expressed as "mean ± SEM", and can also be expressed as ranges. In the case that the reference value is determined by detecting the population, the highest and lowest parts of the measured values may be removed, for example, the highest 2.5% and/or lowest 2.5% are removed.

When the measured result (i.e., activation level of the ERV locus) is higher than the reference value, the degree of aging of the subject or a cell, tissue or organ thereof or physical age of the subject is identified to be higher than the degree of aging or physical age corresponding to the reference value; when the measured result (i.e., activation level of the ERV locus) is lower than reference value, the degree of aging of the subject or a cell, tissue or organ thereof or physical age of the subject is identified to be lower than the degree of aging or physical age corresponding to the reference value.

In some embodiments, the reference value is obtained by detecting a reference subject or a population reference subjects. When the measured result is higher than reference value, the degree of aging of the subject or a cell, tissue or organ thereof or physical age of the subject is identified to be higher than the degree of aging of the reference subject or the population of reference subjects or a cell, tissue or organ thereof or the physical age of the reference subject or the population of reference subjects; when the measured result is lower than reference value, the degree of aging of the subject or a cell, tissue or organ thereof or physical age of the subject is identified as lower than the degree of aging of the reference subject or the population of reference subjects or a cell, tissue or organ thereof or the physical age of the reference subject or the population of reference subjects.

In some embodiments, the reference value represents a value or range for the activation level of the ERV locus of a reference subject or a population of reference subjects, including a value or range for the methylation level of the ERV locus, a value or range for the level of an ERV transcript, a value or range for the level of an ERV protein, a value or range for the presence of the ERV particles in the cell and/or the release of the ERV particles from the cell, and/or a value or range for the transposition probability of the ERV locus. In some embodiments, the reference value represents a value or range for the activation level of the ERV locus in a reference subject or a population of reference subjects, including a value or range for the methylation level of the ERV locus, a value or range for the level of an ERV transcript, a value or range for the level of an ERV protein, and/or a value or range for the presence of the ERV particles in the cell and/or the release of the ERV particles from the cell. In some embodiments, the reference value represents a value or range for activation level of the ERV locus of reference subject or population of reference subjects, including a value or range for the transposition probability of the ERV locus.

In some embodiments, the detecting the activation level of an ERV locus comprises detecting the methylation level of the ERV locus, e.g., the methylation level of the flanking LTR region. The methylation levels of specific regions may be tested by methods known in the art. For example, enrichment level of histone labeling (e.g., H3K9me3 or H3K36me3) may be detected by chromatin immunoprecipitation (ChIP)-qPCR method. In some embodiments, the method comprises the use of primer pairs comprising SEQ ID NOs: 9 and 10.

In some embodiments, the level of a transcript of the coding sequence of the ERV, or the level of the encoded protein thereof is detected. In some embodiments, the coding sequence comprises Env, Pol, Pro and/or Gag genes of the ERV In some embodiments, the level of the transcript of the coding sequence is detected by gene specific primer pair or probe. In some embodiments, the level of protein encoded by the coding sequence is detected by protein-binding molecule. Protein-binding molecule includes any molecule capable of binding to a given protein, such as but not limited to an antibody, the antigen-binding fragment and antibody derivative of an antibody. In some embodiments, the antigen-binding fragment of an antibody includes but is not limited to a Fv fragment (e.g., single stranded Fv and disulfide bonded Fv) and a Fab-like fragment (e.g., Fab fragment, Fab' fragment and F(ab')₂ fragment).

In some embodiments, the subject or animal is a mammal. In some embodiments, the subject or animal is a non-human mammal. For example, the subject or animal is a mouse, and optionally, the ERV is from MMTV family; or the subject or animal is a non-human primate, and optionally, the ERV is from ERVW family. Preferably, the subject or animal is a human, and optionally, the ERV is from HERVK family.

In some embodiments, the subject is a human. Preferably, the ERV is from MMTV family. In some embodiments, the detection is performed for HERVK coding region (HERVK-int)), e.g., its transcript. In some embodiments, the detection is performed for MER61 coding region (MER61-int), e.g., its transcript. In some embodiments, the detection is performed for LTR region in MamGyp, e.g., its transcript.

In some embodiments, the transcript or translated protein of coding sequence of HERVK is detected, including e.g., the transcript or translated protein of Env, Pol, Pro and/or Gag genes of HERVK. It's known in the art that there are many HERVK loci in human genome, there are slight differences among distinct HERVK loci, among which Env, Pol, Pro and Gag genes in HERVK locus HML2_1q22 are shown as SEQ ID NOs: 5, 6, 7 and 8,respectively, encoded proteins thereof are shown as SEQ ID NOs: 1, 2, 3 and 4. Therefore, in some embodiments, the transcript comprises a transcript of one of SEQ ID NOs: 5-8, or a nucleotide sequence having a percent identity of at least 80%, 85%, 90%, 95%, 96%, 97%, 98%, 99%, 99.5%, 99.6%, 99.7%, 99.8% or 99.9% to one of SEQ ID NOs: 5-8. In some embodiments, the protein is a protein comprising one of SEQ ID NOs: 1-4, or an amino acid sequence having a percent identity of at least 80%, 85%, 90%, 95%, 96%, 97%, 98%, 99%, 99.5%, 99.6%, 99.7%, 99.8% or 99.9% to one of SEQ ID NOs: 1-4.

In some embodiments, the method comprises the use of primer pairs comprising SEQ ID NOs: 11 and 12, SEQ ID NOs: 13 and 14, SEQ ID NOs: 15 and 16, SEQ ID NOs: 17 and 18, SEQ ID NOs: 19 and 20, or SEQ ID NOs: 21 and 22. In some embodiments, the method comprises the use of a probe comprising a nucleotide sequence of SEQ ID NO: 60.

The presence of the ERV particles in the cell and/or the release of the ERV particles from the cell may be detected by imaging technique, e.g., transmission electron microscopy (TEM) technique.

The release of ERV particle from cell may also be detected by measuring ERV protein, preferably Env protein, e.g., the protein comprising SEQ ID NO: 1 or an amino acid sequence having a percent identity of at least 80%, 85%, 90%, 95%, 96%, 97%, 98%, 99%, 99.5%, 99.6%, 99.7%, 99.8% or 99.9% to SEQ ID NO: 1.

ERV specific antibody may also be detected. In some embodiments, the method comprises the use of a protein comprising one of SEQ ID NOs: 1-4 or an amino acid sequence having a percent identity of at least 80%, 85%, 90%, 95%, 96%, 97%, 98%, 99%, 99.5%, 99.6%, 99.7%, 99.8% or 99.9% to one of SEQ ID NOs: 1-4. Preferably, the method comprises use of a protein comprising SEQ ID NO: 1 or amino acid sequence having a percent identity of at least 80%, 85%, 90%, 95%, 96%, 97%, 98%, 99%, 99.5%, 99.6%, 99.7%, 99.8% or 99.9% to SEQ ID NO: 1.

Samples that can be used for the method of the invention comprise samples of cells or tissues from the subject, e.g., biopsy samples of various tissues. Samples that can be used for the method of the invention may also be body fluid samples, e.g., urine, saliva and blood, including whole blood, plasma or serum.

The invention also provides a method for assessing the degree of senescence of an isolated animal cell or cell population, including
a) detecting the activation level of an ERV in the cell; and
b) comparing the result of the detection with the reference value of the activation level of the ERV

In some embodiments, the ERV is selected from the group comprising families HERVK (including HERVK11), MER61, MER61C, Charlie4, MER101B, MamGyp, HERV16, HUERS-P2 and ERVW and homologs thereof. In some embodiments, the ERV is selected from the group comprising families HERVK (including HERVK11), MER61, MER61C, Charlie4, MER101B, MamGyp, HERV16, HUERS-P2, ERVW and MMTV and homologs thereof. In some embodiments, the ERV includes at least an ERV from HERVK family (including HERVK11), optionally, the ERV also includes ERV from the group comprising families MER61, MER61C, Charlie4, MER101B, MamGyp, HERV16, HUERS-P2 and ERVW and homologs thereof.

In some embodiments, the "activation level" of an ERV locus includes the methylation level of the ERV locus, the level of an ERV transcript, the level of an ERV protein, and/or the presence, localization of the ERV particles in the cell and/or the release of the ERV particles from the cell. A lower methylation level, a higher transcript level, a higher protein level and/ or a higher presence of the ERV particles in the cell and/or the release of the ERV particles from the cell represent a higher activation level of the ERV locus. In some embodiments, the activation level of an ERV locus includes the transposition probability of the ERV locus. A higher transposition probability represents a higher activation level.

In some embodiments, the reference value is obtained by detecting a reference cell. In the invention, the reference cell is a cell having specific passage number or within specific range of passage numbers. In some embodiments, the reference value is obtained by detecting a population of reference cells.

The reference value may be expressed as "mean ± SEM", also can be expressed as range. In the case that the reference value is determined by detecting the population, the highest and lowest parts of the measured values may be removed, for example, the highest 2.5% and/or lowest 2.5% are removed.

When the measured result (i.e., activation level of the ERV locus) is higher than reference value, the degree of senescence of the cell or cell population is identified to be higher than the degree of senescence corresponding to the reference value; when the measured result (i.e., activation level of the ERV locus) is lower than reference value, the degree of senescence of the cell or cell population is identified to be lower than the degree of senescence corresponding to the reference value.

In some embodiments, the reference value is obtained by detecting a reference cell or a population of reference cells. When the measured result is higher than the reference value, the degree of senescence of the cell or cell population is identified as higher than the degree of senescence corresponding to passage number of the reference cell or the population of reference cells; when the measured result is lower than reference value, the degree of senescence of the cell or cell population is identified as lower than the degree of senescence corresponding to passage number of the reference cell or the population of reference cells.

In some embodiments, the reference value represents a value or range for the activation level of the ERV locus of a reference subject or a population of reference subjects, including a value or range for the methylation level of the ERV locus, a value or range for the level of an ERV transcript, a value or range for the level of an ERV protein, a value or range for the presence of the ERV particles in the cell and/or the release of the ERV particles from the cell, and/or a value or range for the transposition probability of the ERV locus. In some embodiments, the reference value represents a value or range for the activation level of the ERV locus in a reference subject or a population of reference subjects, including a value or range for the methylation level of the ERV locus, a value or range for the level of an ERV transcript, a value or range for the level of an ERV protein, and/or a value or range for the presence of the ERV particles in the cell and/or the release of the ERV particles from the cell. In some embodiments, the reference value represents a value or range for activation level of the ERV locus of reference subject or population of reference subjects, including a value or range for the transposition probability of the ERV locus.

In some embodiments, the detecting the activation level of an ERV locus comprises detecting the methylation level of the ERV locus, e.g., the methylation level of the flanking LTR region. The methylation levels of specific regions may be tested by methods known in the art. For example, enrichment level of histone labeling (e.g., H3K9me3 or H3K36me3) may be detected by chromatin immunoprecipitation (ChIP)-qPCR method. In some embodiments, the method comprises the use of primer pairs comprising SEQ ID NOs: 9 and 10.

In some embodiments, the level of a transcript of the coding sequence of the ERV, or the level of the encoded protein thereof is detected. In some embodiments, the coding sequence comprises Env, Pol, Pro and/or Gag genes of the ERV In some embodiments, the level of the transcript of the coding sequence is detected by gene specific primer pair or probe. In some embodiments, the level of protein encoded by the coding sequence is detected by protein-binding molecule. Protein-binding molecule includes any molecule capable of binding to a given protein, such as but not limited to an antibody, the antigen-binding fragment and antibody derivative of an antibody. In some embodiments, the antigen-binding fragment of an antibody includes but is not limited to a Fv fragment (e.g., single stranded Fv and disulfide bonded Fv) and a Fab-like fragment (e.g., Fab fragment, Fab' fragment and F(ab')₂ fragment).

Cells detected by the method of the invention may be any animal cell, for example, animal cell for the industrial production of recombinant protein or vaccine, or stem cell.

In some embodiments, the cell is a non-human mammal cell. In some embodiments, the cell is a murine cell, such as CHO and mouse cell. In some embodiments, the cell is a mouse cell, and the ERV is from MMTV family.

In some embodiments, the cell is a non-human primate cell. Preferably, the non-human primate is an Old-World monkey. For example, the cell is vero cell. Preferably, the ERV is from ERVW family.

In some embodiments, the cell is a human cell. Preferably, the ERV is from HERVK family. In some embodiments, the detection is performed for the HERVK coding region (HERVK-int)), e.g., its transcript. In some embodiments, the detection is performed for the MER61 coding region (MER61-int), e.g., its transcript. In some embodiments, the detection is performed for the LTR region in MamGyp, e.g., its transcript.

In some embodiments, the transcript or translated protein of including e.g., the transcript or translated protein of Env, Pol, Pro and/or Gag genes of HERVK. It's known in the art that there are many HERVK loci in human genome, there are slight differences among distinct HERVK loci, among which Env, Pol, Pro and Gag genes in HERVK locus HML2_1q22 are shown as SEQ ID NOs: 5, 6, 7 and 8,respectively, encoded proteins thereof are shown as SEQ ID NOs: 1, 2, 3 and 4. Therefore, in some embodiments, the transcript comprises a transcript of one of SEQ ID NOs: 5-8, or a nucleotide sequence having a percent identity of at least 80%, 85%, 90%, 95%, 96%, 97%, 98%, 99%, 99.5%, 99.6%, 99.7%, 99.8% or 99.9% to one of SEQ ID NO: 5-8. In some embodiments, the protein is a protein comprising one of SEQ ID NO: 1-4, or an amino acid sequence having a percent identity of at least 80%, 85%, 90%, 95%, 96%, 97%, 98%, 99%, 99.5%, 99.6%, 99.7%, 99.8% or 99.9% to one of SEQ ID NO: 1-4.

In some embodiments, the method comprises the use of primer pairs comprising SEQ ID NOs: 11 and 12, SEQ ID NOs: 13 and 14, SEQ ID NOs: 15 and 16, SEQ ID NOs: 17 and 18, SEQ ID NOs: 19 and 20, or SEQ ID NOs: 21 and 22. In some embodiments, the method comprises the use of a probe comprising a nucleotide sequence of SEQ ID NO: 60.

The presence of the ERV particles in the cell and/or the release of the ERV particles from the cell may be detected by imaging technique, e.g., transmission electron microscopy (TEM) technique.

The release of ERV particle from cell may also be detected by measuring ERV protein, preferably Env protein, e.g., the protein comprising SEQ ID NO: 1 or an amino acid sequence having a percent identity of at least 80%, 85%, 90%, 95%, 96%, 97%, 98%, 99%, 99.5%, 99.6%, 99.7%, 99.8% or 99.9% to SEQ ID NO: 1.

### 3. A method for regulating senescence

The invention provides a method for regulating senescence, contacting the cell with an agent regulating the activation of an ERV locus. The method of the invention regulates cell senescence by regulating the endogenous retrovirus.

In some embodiments, the ERV is selected from the group comprising families HERVK (including HERVK11), MER61, MER61C, Charlie4, MER101B, MamGyp, HERV16, HUERS-P2 and ERVW and homologs thereof. In some embodiments, the ERV is selected from the group comprising families HERVK (including HERVK11), MER61, MER61C, Charlie4, MER101B, MamGyp, HERV16, HUERS-P2, ERVW and MMTV and homologs thereof. In some embodiments, the ERV includes at least an ERV from HERVK family (including HERVK11), optionally, the ERV also includes ERV from the group comprising families MER61, MER61C, Charlie4, MER101B, MamGyp, HERV16, HUERS-P2 and ERVW and homologs thereof.

The regulating agent may be an inhibiting or promoting agent, and the method also comprises using an inhibiting agent against ERV particle. Cell senescence is delayed by inhibiting endogenous retrovirus, and is promoted by activating endogenous retrovirus.

Herein, an agent inhibiting the activation of an ERV locus relates to specific inhibition (e.g., knockout or knockdown of the locus, knockout or knockdown of genes in the locus, enhancing methylation level of the locus) and non-specific inhibition (e.g., enhancing the methylation level of whole genome, thus promoting methylation of the locus, or non-specific retrovirus inhibitor, suppressing virus packaging, etc.). The agent inhibiting ERV means an agent inhibiting ERV viral particles.

In some embodiments, the agent inhibiting the activation of an ERV locus promotes the methylation of the ERV locus by e.g., increasing the activity of transmethylase. The inhibiting agent includes but is not limited to transmethylase or the coding polynucleotide thereof.

In some embodiments, the agent inhibiting the activation of an ERV locus is a non-specific retrovirus inhibitor, which includes e.g., a reverse transcriptase inhibitor, an integrase inhibitor and/or a protease inhibitor. In some embodiments, the reverse transcriptase inhibitor includes nucleoside reverse transcriptase inhibitor such as enofovir, abacavir, stavudine (D4T), lamivudine (3TC) and/or zidovudine, and/or non-nucleoside reverse transcriptase inhibitor such as efavirenz, etravirine and/or nevirapine. In some embodiments, the integrase inhibitor includes Raltegravir. In some embodiments, the protease inhibitor includes Lopinavir and/or Darunavir. In some embodiments, the retrovirus inhibitor includes lamivudine (3TC), abacavir and/or Raltegravir.

In some embodiments, the agent inhibiting the activation of an ERV locus is a gene therapy system targeting the ERV locus.

In some embodiments, the agent inhibiting the activation of an ERV locus is a CRISPR-dCas system targeting the ERV locus and increasing the methylation.

In some embodiments, the agent inhibiting the activation of an ERV locus is a gene editing system for the knockout or knockdown of the ERV locus, including but not limited to meganuclease, zinc finger nuclease (ZFN), transcription activator-like effector nuclease (TALEN) and CRISPR/Cas system.

In some embodiments, the agent inhibiting the activation of an ERV locus is an antisense nucleic acid or interfering nucleic acid (including but not limited to siRNA, shRNA and miRNA) for knockout or knockdown of Env, Pol, Pro and/or Gag genes in the ERV

In some embodiments, the agent inhibiting ERV is a binding molecule which can bind to the ERV protein such as Env protein, e.g., an antibody or the antigen-binding fragment and antibody derivative thereof, Preferably a neutralizing antibody or antigen-binding fragment or antibody derivative thereof against the ERV

In some embodiments, the subject is a non-human mammal. In some embodiments, the subject is a rodent. Preferably, the rodent is mouse. Preferably, the ERV is from MMTV family.

In some embodiments, the subject is a non-human mammal. In some embodiments, the subject is a non-human primate. Preferably, the non-human primate is an Old-World monkey. Preferably, the ERV is from ERVW family.

In some embodiments, the subject is a human. Preferably, the ERV is from HERVK family.

In some embodiments, the agent inhibiting the activation of an ERV locus is a gene editing system for the knockout or knockdown of the ERV locus, including but not limited to HERVK and MMTV

In some embodiments, the agent inhibiting the activation of an ERV locus is a gene editing system for the knockout or knockdown of a HERVK locus. In some embodiments, the agent inhibiting the activation of an ERV locus is an antisense nucleic acid or interfering nucleic acid (including but not limited to siRNA, shRNA and miRNA) for the knockdown or knockout of a nucleic acid molecule comprising one of SEQ ID NOs: 5-8, or a nucleotide sequence having a percent identity of at least 80%, 85%, 90%, 95%, 96%, 97%, 98%, 99%, 99.5%, 99.6%, 99.7%, 99.8% or 99.9% to one of SEQ ID NOs: 5-8. In some embodiments, the agent inhibiting the activation of the ERV locus encodes a shRNA of SEQ ID NO: 51 or 52.

In some embodiments, the agent inhibiting ERV is a binding molecule which binds to ERV-Env, including but not limited to HERVK-Env, HERVW-Env and MMTV-Env, e.g., an antibody or the antigen-binding fragment or antibody derivative thereof. In some embodiments, the agent inhibiting ERV is a binding molecule which binds to HERVK-Env (e.g., the protein comprising SEQ ID NO: 1 or an amino acid sequence having a percent identity of at least 80%, 85%, 90%, 95%, 96%, 97%, 98%, 99%, 99.5%, 99.6%, 99.7%, 99.8% or 99.9% to SEQ ID NO: 1), for example, an antibody or the antigen-binding fragment or antibody derivative thereof.

In some embodiments, the regulating agent is an agent promoting the activation of an ERV locus in the cell. In the invention, the agent promoting the activation of the ERV locus may be a specific promoting agent or non-specific promoting agent.

In some embodiments, the specific promoting agent is a transcriptional activation system targeting the ERV locus. In some embodiments, the transcriptional activation system is a CRISPR-dCas transcriptional activation system. In some embodiments, the specific promoting agent is a demethylation system targeting the ERV locus. In some embodiments, the demethylation system is a demethylation system based on CRISPR-dCas, e.g., CRISPR-dCas-Tet.

In some embodiments, the non-specific promoting agent is a DNA methyltransferase inhibitor, e.g., decitabine, Lomeguatrib, SGI-1027 or 5-azacytidine.

In some embodiments, the cell is a non-human mammal cell. In some embodiments, the cell is a rodent cell. In some embodiments, the cell is a mouse cell. In some embodiments, the ERV is from MMTV family.

In some embodiments, the cell is a non-human mammal cell. In some embodiments, the cell is a non-human primate cell. In some embodiments, the non-human primate is An Old-World monkey. In some embodiments, the ERV is from ERVW family.

In some embodiments, the cell is a human cell, e.g., tumor cell, infected cell or cells in damaged tissues. Preferably, the ERV is from HERVK family. In some embodiments, the sgRNA in the CRISPR-dCas transcriptional activation system comprises SEQ ID NO: 59. In some embodiments, the method comprises contacting the human cell with HERVK particles.

The invention provides a method for treating premature aging in a subject in need or a cell, tissue of organ thereof, or preventing or delaying the aging of the subject or a cell, tissue of organ thereof, including administering an agent inhibiting the activation of an ERV locus or an agent inhibiting ERV to the subject.

In some embodiments, the ERV is selected from the group comprising families HERVK (including HERVK11), MER61, MER61C, Charlie4, MER101B, MamGyp, HERV16, HUERS-P2 and ERVW and homologs thereof. In some embodiments, the ERV is selected from the group comprising families HERVK (including HERVK11), MER61, MER61C, Charlie4, MER101B, MamGyp, HERV16, HUERS-P2, ERVW and MMTV and homologs thereof. In some embodiments, the ERV includes at least an ERV from HERVK family (including HERVK11), optionally, the ERV also includes ERV from the group comprising families MER61, MER61C, Charlie4, MER101B, MamGyp, HERV16, HUERS-P2 and ERVW and homologs thereof.

In some embodiments, the agent inhibiting the activation of an ERV locus promotes the methylation of an ERV locus by e.g., increasing the activity of transmethylase. The inhibiting agent includes but is not limited to transmethylase or its coding polynucleotide.

In some embodiments, the agent inhibiting the activation of an ERV locus is a non-specific retrovirus inhibitor, which includes e.g., a reverse transcriptase inhibitor, an integrase inhibitor and/or a protease inhibitor. In some embodiments, the reverse transcriptase inhibitor includes nucleoside type reverse transcriptase inhibitor such as enofovir, abacavir, stavudine (D4T), lamivudine (3TC) and/or zidovudine, and/or non-nucleoside type reverse transcriptase inhibitor such as efavirenz, etravirine and/or nevirapine. In some embodiments, the integrase inhibitor includes Raltegravir. In some embodiments, the protease inhibitor includes Lopinavir and/or Darunavir. In some embodiments, the retrovirus inhibitor includes lamivudine (3TC), abacavir and/or Raltegravir.

In some embodiments, the agent inhibiting the activation of an ERV locus is a gene therapy system targeting the ERV locus.

In some embodiments, the agent inhibiting the activation of an ERV locus is a CRISPR-dCas system targeting the ERV locus and increasing the methylation.

In some embodiments, the agent inhibiting the activation of an ERV locus is a gene editing system for the knockout or knockdown of the ERV locus, including but not limited to meganuclease, zinc finger nuclease (ZFN), transcription activator-like effector nuclease (TALEN) and CRISPR/Cas system.

In some embodiments, the agent inhibiting the activation of an ERV locus is an antisense nucleic acid or interfering nucleic acid (including but not limited to siRNA, shRNA and miRNA) used for the knockout or knockdown of Env, Pol, Pro and/or Gag genes in ERV.

In some embodiments, the agent inhibiting the ERV is a binding molecule which can bind with the ERV protein such as Env protein, e.g., an antibody or the antigen-binding fragment and antibody derivative thereof, preferably a neutralizing antibody against ERV or the antigen-binding fragment or antibody derivative thereof.

In some embodiments, the subject is a non-human mammal. In some embodiments, the subject is a rodent. Preferably, the rodent is mouse. Preferably, the ERV is from MMTV family.

In some embodiments, the subject is a non-human mammal. In some embodiments, the subject is a non-human primate. Preferably, the non-human primate is an Old-World monkey. Preferably, the ERV is from ERVW family.

In some embodiments, the subject is a human. Preferably, the ERV is from HERVK family.

In some embodiments, the agent inhibiting the activation of an ERV locus is a gene editing system for the knockout or knockdown of HERVK locus. In some embodiments, the agent inhibiting the activation of an ERV locus is an antisense nucleic acid or interfering nucleic acid (including but not limited to siRNA, shRNA and miRNA) for the knockdown or knockout of a nucleic acid molecule comprising one of SEQ ID NOs: 5-8, or a nucleotide sequence having a percent identity of at least 80%, 85%, 90%, 95%, 96%, 97%, 98%, 99%, 99.5%, 99.6%, 99.7%, 99.8% or 99.9% to one of SEQ ID NOs: 5-8. In some embodiments, the agent inhibiting the activation of the ERV locus encodes a shRNA of SEQ ID NO: 51 or 52.

In some embodiments, the agent inhibiting ERV is a binding molecule which binds to HERVK-Env (e.g., the protein comprising SEQ ID NO: 1 or an amino acid sequence having a percent identity of at least 80%, 85%, 90%, 95%, 96%, 97%, 98%, 99%, 99.5%, 99.6%, 99.7%, 99.8% or 99.9% to SEQ ID NO: 1), for example, an antibody or the antigen-binding fragment or antibody derivative thereof.

Isolated animal cells are used for industrial production of recombinant protein or vaccine, while the activity and proliferation ability of the used cells are very important for production. Cell senescence will make cell activity and proliferation ability reduce, thus adversely affecting productivity. In the process of culturing cells, preventing or delaying cell senescence is beneficial to industrial production.

Therefore, the invention provides a method for culturing isolated animal cell or cell population, including contacting the cell with an agent inhibiting the activation of an ERV locus of the cell or an agent inhibiting ERV.

In some embodiments, the ERV is selected from the group comprising families HERVK (including HERVK11), MER61, MER61C, Charlie4, MER101B, MamGyp, HERV16, HUERS-P2 and ERVW and homologs thereof. In some embodiments, the ERV is selected from the group comprising families HERVK (including HERVK11), MER61, MER61C, Charlie4, MER101B, MamGyp, HERV16, HUERS-P2, ERVW and MMTV and homologs thereof. In some embodiments, the ERV includes at least an ERV from HERVK family (including HERVK11), optionally, the ERV also includes ERV from the group comprising families MER61, MER61C, Charlie4, MER101B, MamGyp, HERV16, HUERS-P2 and ERVW and homologs thereof.

In some embodiments, the agent inhibiting the activation of an ERV locus promotes the methylation of the ERV locus by e.g., increasing activity of transmethylase. The inhibiting agent includes but is not limited to transmethylase or its coding polynucleotide.

In some embodiments, the agent inhibiting the activation of an ERV locus is a non-specific retrovirus inhibitor, which includes e.g., a reverse transcriptase inhibitor, an integrase inhibitor and/or a protease inhibitor. In some embodiments, the reverse transcriptase inhibitor includes nucleoside type reverse transcriptase inhibitor such as enofovir, abacavir, stavudine (D4T), lamivudine (3TC) and/or zidovudine, and/or non-nucleoside type reverse transcriptase inhibitor such as efavirenz, etravirine and/or nevirapine. In some embodiments, the integrase inhibitor includes Raltegravir. In some embodiments, the protease inhibitor includes Lopinavir and/or Darunavir. In some embodiments, the retrovirus inhibitor includes lamivudine (3TC), abacavir and/or Raltegravir.

In some embodiments, the agent inhibiting the activation of an ERV locus is a gene therapy system targeting the ERV locus.

In some embodiments, the agent inhibiting the activation of an ERV locus is a CRISPR-dCas system targeting the ERV locus and increasing the methylation.

In some embodiments, the agent inhibiting the activation of an ERV locus is a gene editing system for the knockout or knockdown of the ERV locus, including but not limited to meganuclease, zinc finger nuclease (ZFN), transcription activator-like effector nuclease (TALEN) and CRISPR/Cas system.

In some embodiments, the agent inhibiting the activation of an ERV locus is antisense nucleic acid or interfering nucleic acid (including but not limited to siRNA, shRNA and miRNA) used for the knockout or knockdown of Env, Pol, Pro and/or Gag genes in ERV.

In some embodiments, the agent inhibiting ERV is a binding molecule which can bind to an ERV protein such as Env protein, e.g., an antibody or the antigen-binding fragment and antibody derivative thereof, preferably a neutralizing antibody or antigen-binding fragment or antibody derivative thereof against the ERV

In some embodiments, the cell is a non-human mammal cell. In some embodiments, the cell is a murine cell, such as CHO and mouse cell. In some embodiments, the cell is a mouse cell, and the ERV is from MMTV family.

In some embodiments, the cell is a non-human primate cell. Preferably, the non-human primate is an Old-World monkey. For example, the cell is vero cell. Preferably, the ERV is from ERVW family.

In some embodiments, the cell is a human cell. Preferably, the ERV is from HERVK family.

In some embodiments, the agent inhibiting the activation of an ERV locus is a gene editing system for the knockout or knockdown of HERVK locus. In some embodiments, the agent inhibiting the activation of an ERV locus is an antisense nucleic acid or interfering nucleic acid (including but not limited to siRNA, shRNA and miRNA) for the knockdown or knockout of a nucleic acid molecule comprising one of SEQ ID NOs: 5-8, or a nucleotide sequence having a percent identity of at least 80%, 85%, 90%, 95%, 96%, 97%, 98%, 99%, 99.5%, 99.6%, 99.7%, 99.8% or 99.9% to one of SEQ ID NOs: 5-8. In some embodiments, the agent inhibiting the activation of the ERV locus encodes a shRNA of SEQ ID NO: 51 or 52.

In some embodiments, the agent inhibiting ERV is a binding molecule which binds to HERVK-Env (e.g., the protein comprising SEQ ID NO: 1 or an amino acid sequence having a percent identity of at least 80%, 85%, 90%, 95%, 96%, 97%, 98%, 99%, 99.5%, 99.6%, 99.7%, 99.8% or 99.9% to SEQ ID NO: 1), for example, an antibody or the antigen-binding fragment or antibody derivative thereof.

The invention provides a method for preventing or delaying cell senescence, including contacting the cell with an agent inhibiting the activation of an ERV locus of the cell or an agent inhibiting ERV.

In some embodiments, the ERV is selected from the group comprising families HERVK (including HERVK11), MER61, MER61C, Charlie4, MER101B, MamGyp, HERV16, HUERS-P2 and ERVW and homologs thereof. In some embodiments, the ERV is selected from the group comprising families HERVK (including HERVK11), MER61, MER61C, Charlie4, MER101B, MamGyp, HERV16, HUERS-P2, ERVW and MMTV and homologs thereof. In some embodiments, the ERV includes at least an ERV from HERVK family (including HERVK11), optionally, the ERV also includes ERV from the group comprising families MER61, MER61C, Charlie4, MER101B, MamGyp, HERV16, HUERS-P2 and ERVW and homologs thereof.

In some embodiments, the agent inhibiting the activation of an ERV locus promotes the methylation of the ERV locus by e.g., increasing activity of transmethylase. The inhibitor includes but is not limited to transmethylase or its coding polynucleotide.

In some embodiments, the agent inhibiting the activation of an ERV locus is a non-specific retrovirus inhibitor, which includes e.g., reverse transcriptase inhibitor, integrase inhibitor and/or protease inhibitor. In some embodiments, the reverse transcriptase inhibitor includes nucleoside type reverse transcriptase inhibitor such as enofovir, abacavir, stavudine (D4T), lamivudine (3TC) and/or zidovudine, and/or non-nucleoside type reverse transcriptase inhibitor such as efavirenz, etravirine and/or nevirapine. In some embodiments, the integrase inhibitor includes Raltegravir. In some embodiments, the protease inhibitor includes Lopinavir and/or Darunavir. In some embodiments, the retrovirus inhibitor includes lamivudine (3TC), abacavir and/or Raltegravir.

In some embodiments, the agent inhibiting the activation of an ERV locus is a gene therapy system targeting the ERV locus.

In some embodiments, the agent inhibiting the activation of an ERV locus is a CRISPR-dCas system targeting the ERV locus and increasing the methylation.

In some embodiments, the agent inhibiting the activation of an ERV locus is a gene editing system for the knockout or knockdown of the ERV locus, including but not limited to meganuclease, zinc finger nuclease (ZFN), transcription activator-like effector nuclease (TALEN) and CRISPR/Cas system.

In some embodiments, the agent inhibiting the activation of an ERV locus is antisense nucleic acid or interfering nucleic acid (including but not limited to siRNA, shRNA and miRNA) used for the knockout or knockdown of Env, Pol, Pro and/or Gag genes in the ERV

In some embodiments, the agent inhibiting ERV is a binding molecule which can bind with the ERV protein such as Env protein, e.g., an antibody or the antigen-binding fragment and antibody derivative thereof, Preferably ERV neutralizing antibody or antigen-binding fragment thereof or antibody derivative.

In some embodiments, the cell is a non-human mammal cell. In some embodiments, the cell is a murine cell, such as CHO and mouse cell. In some embodiments, the cell is a mouse cell, and the ERV is from MMTV family.

In some embodiments, the cell is a non-human primate cell. Preferably, the non-human primate is an Old-World monkey. For example, the cell is vero cell. Preferably, the ERV is from ERVW family.

In some embodiments, the cell is a human cell. Preferably, the ERV is from HERVK family.

In some embodiments, the agent inhibiting the activation of an ERV locus is a gene editing system for the knockout or knockdown of HERVK locus. In some embodiments, the agent inhibiting the activation of an ERV locus is an antisense nucleic acid or interfering nucleic acid (including but not limited to siRNA, shRNA and miRNA) for the knockdown or knockout of a nucleic acid molecule comprising one of SEQ ID NOs: 5-8, or a nucleotide sequence having a percent identity of at least 80%, 85%, 90%, 95%, 96%, 97%, 98%, 99%, 99.5%, 99.6%, 99.7%, 99.8% or 99.9% to one of SEQ ID NOs: 5-8. In some embodiments, the agent inhibiting the activation of the ERV locus encodes a shRNA of SEQ ID NO: 51 or 52.

In some embodiments, the agent inhibiting the ERV is a binding molecule which binds to HERVK-Env (e.g., the protein comprising SEQ ID NO: 1 or an amino acid sequence having a percent identity of at least 80%, 85%, 90%, 95%, 96%, 97%, 98%, 99%, 99.5%, 99.6%, 99.7%, 99.8% or 99.9% to SEQ ID NO: 1), for example, an antibody or the antigen-binding fragment or antibody derivative thereof.

The invention also provides a method for promoting cell senescence, including contacting the cell with an agent promoting the activation of an ERV locus of the cell or the ERV particles.

In some embodiments, the ERV is selected from the group comprising families HERVK (including HERVK11), MER61, MER61C, Charlie4, MER101B, MamGyp, HERV16, HUERS-P2 and ERVW and homologs thereof. In some embodiments, the ERV is selected from the group comprising families HERVK (including HERVK11), MER61, MER61C, Charlie4, MER101B, MamGyp, HERV16, HUERS-P2, ERVW and MMTV and homologs thereof. In some embodiments, the ERV includes at least an ERV from HERVK family (including HERVK11), optionally, the ERV also includes ERV from the group comprising families MER61, MER61C, Charlie4, MER101B, MamGyp, HERV16, HUERS-P2 and ERVW and homologs thereof. In the invention, the agent promoting the activation of an ERV locus may be a specific promoting agent or non-specific promoting agent.

In some embodiments, the specific promoting agent is a transcriptional activation system targeting the ERV locus. In some embodiments, the transcriptional activation system is a CRISPR-dCas transcriptional activation system. In some embodiments, the specific promoting agent is a demethylation system targeting the ERV locus. In some embodiments, the demethylation system is a demethylation system based on CRISPR-dCas, e.g.,CRISPR-dCas-Tet. In some embodiments, the ERV locus includes but is not limited to HERVK, HERVW and MMTV loci.

In some embodiments, the non-specific promoting agent is a DNA methyltransferase inhibitor, e.g., decitabine, Lomeguatrib, SGI-1027 or 5-azacytidine.

In some embodiments, the cell is a non-human mammal cell. In some embodiments, the cell is a rodent cell. In some embodiments, the cell is a mouse cell. In some embodiments, the ERV is from MMTV family.

In some embodiments, the cell is a non-human mammal cell. In some embodiments, the cell is a non-human primate cell. In some embodiments, the non-human primate is an Old-World monkey. In some embodiments, the ERV is from ERVW family.

In some embodiments, the cell is a human cell, e.g., tumor cell, infected cell or cells in damaged tissues. Preferably, the ERV is from HERVK family. In some embodiments, the sgRNA in the CRISPR-dCas transcriptional activation system comprises SEQ ID NO: 59. In some embodiments, the method comprises contacting the human cell with HERVK particles.

The invention provides a method for preventing or delaying tissue and organ degeneration, treating aging-related diseases, delaying the aging of body, for example, a therapeutic method for preventing or delaying skin aging, treating or preventing osteoarthritis and frailty, comprising administering an agent inhibiting the activation of an ERV locus or an agent inhibiting an ERV to the subject in need. In some embodiments, the ERV locus includes but is not limited to HERVK, HERVW and MMTV loci.

In some embodiments, the agent inhibiting the activation of an ERV locus promotes the methylation of the ERV locus by e.g., increasing the activity of transmethylase. The inhibiting agent includes but is not limited to transmethylase or its coding polynucleotide.

In some embodiments, the agent inhibiting the activation of an ERV locus is a non-specific retrovirus inhibitor, which includes e.g., a reverse transcriptase inhibitor, an integrase inhibitor and/or a protease inhibitor. In some embodiments, the reverse transcriptase inhibitor includes nucleoside type reverse transcriptase inhibitor such as enofovir, abacavir, stavudine (D4T), lamivudine (3TC) and/or zidovudine, and/or non-nucleoside type reverse transcriptase inhibitor such as efavirenz, etravirine and/or nevirapine. In some embodiments, the integrase inhibitor includes Raltegravir. In some embodiments, the protease inhibitor includes Lopinavir and/or Darunavir. In some embodiments, the retrovirus inhibitor includes lamivudine (3TC), abacavir and/or Raltegravir.

In some embodiments, the agent inhibiting the activation of an ERV locus is a gene therapy system targeting the ERV locus.

In some embodiments, the agent inhibiting the activation of an ERV locus is a CRISPR-dCas system targeting the ERV locus and increasing the methylation.

In some embodiments, the agent inhibiting the activation of an ERV locus is a gene editing system for the knockout or knockdown of the ERV locus, including but not limited to meganuclease, zinc finger nuclease (ZFN), transcription activator-like effector nuclease (TALEN) and CRISPR/Cas system.

In some embodiments, the agent inhibiting the activation of an ERV locus is a gene editing system for the knockout or knockdown of the ERV locus.

In some embodiments, the Agent inhibiting ERV is a binding molecule which can bind with the ERV-Env, e.g., an antibody or the antigen-binding fragment and antibody derivative thereof.

The invention also provides a method for preventing or delaying skin aging, comprising administering an agent inhibiting the activation of HERVK locus or an agent inhibiting HERVK to the subject in need.

In some embodiments, the agent inhibiting the activation of HERVK locus promotes the methylation of HERVK locus by e.g., increasing activity of transmethylase. The inhibitor includes but is not limited to transmethylase or its coding polynucleotide.

In some embodiments, the agent inhibiting the activation of HERVK locus is a non-specific retrovirus inhibitor, which includes e.g., a reverse transcriptase inhibitor, an integrase inhibitor and/or a protease inhibitor. In some embodiments, the reverse transcriptase inhibitor includes nucleoside type reverse transcriptase inhibitor such as enofovir, abacavir, stavudine (D4T), lamivudine (3TC) and/or zidovudine, and/or non-nucleoside type reverse transcriptase inhibitor such as efavirenz, etravirine and/or nevirapine. In some embodiments, the integrase inhibitor includes Raltegravir. In some embodiments, the protease inhibitor includes Lopinavir and/or Darunavir. In some embodiments, the retrovirus inhibitor includes lamivudine (3TC), abacavir and/or Raltegravir.

In some embodiments, the agent inhibiting the activation of an ERV locus is a gene therapy system targeting the ERV locus.

In some embodiments, the agent inhibiting the activation of an ERV locus is a CRISPR-dCas system targeting the ERV locus and increasing the methylation.

In some embodiments, the agent inhibiting the activation of HERVK locus is a gene editing system for the knockout or knockdown of the HERVK locus, including but not limited to meganuclease, zinc finger nuclease (ZFN), transcription activator-like effector nuclease (TALEN) and CRISPR/Cas system.

In some embodiments, the agent inhibiting the activation of an ERV locus is a gene editing system for the knockout or knockdown of HERVK locus. In some embodiments, the agent inhibiting the activation of an ERV locus is an antisense nucleic acid or interfering nucleic acid (including but not limited to siRNA, shRNA and miRNA) for the knockdown or knockout of a nucleic acid molecule comprising one of SEQ ID NOs: 5-8, or a nucleotide sequence having a percent identity of at least 80%, 85%, 90%, 95%, 96%, 97%, 98%, 99%, 99.5%, 99.6%, 99.7%, 99.8% or 99.9% to one of SEQ ID NOs: 5-8. In some embodiments, the agent inhibiting the activation of the ERV locus encodes a shRNA of SEQ ID NO: 51 or 52.

In some embodiments, the agent inhibiting the ERV is a binding molecule which binds to HERVK-Env (e.g., the protein comprising SEQ ID NO: 1 or an amino acid sequence having a percent identity of at least 80%, 85%, 90%, 95%, 96%, 97%, 98%, 99%, 99.5%, 99.6%, 99.7%, 99.8% or 99.9% to SEQ ID NO: 1), for example, an antibody or the antigen-binding fragment or antibody derivative thereof.

In some embodiments, the administration is topical administration, intradermal administration or subcutaneous administration.

The invention also provides a method for treating progeroid diseases, e.g., HGPS or WS and aging-related diseases, including but not limited to arthritis, premature ovarian failure, hepatic fibrosis, pulmonary fibrosis and angiocardiopathy, comprising administering an agent inhibiting the activation of an ERV locus or an agent inhibiting an ERV to the subject in need. In some embodiments, the ERV is selected from HERVK, HERVW and MMTV

In some embodiments, the agent inhibiting the activation of an ERV locus promotes the methylation of the ERV locus by e.g., increasing activity of transmethylase. The inhibitor includes but is not limited to transmethylase or its coding polynucleotide.

In some embodiments, the agent inhibiting the activation of an ERV locus is a non-specific retrovirus inhibitor, which includes e.g., a reverse transcriptase inhibitor, an integrase inhibitor and/or a protease inhibitor. In some embodiments, the reverse transcriptase inhibitor includes nucleoside type reverse transcriptase inhibitor such as enofovir, abacavir, stavudine (D4T), lamivudine (3TC) and/or zidovudine, and/or non-nucleoside type reverse transcriptase inhibitor such as efavirenz, etravirine and/or nevirapine. In some embodiments, the integrase inhibitor includes Raltegravir. In some embodiments, the protease inhibitor includes Lopinavir and/or Darunavir. In some embodiments, the retrovirus inhibitor includes lamivudine (3TC), abacavir and/or Raltegravir.

In some embodiments, the agent inhibiting the activation of ERV locus is a gene therapy system targeting the ERV locus.

In some embodiments, the agent inhibiting the activation of ERV locus is a CRISPR-dCas system targeting the ERV locus and increasing the methylation.

In some embodiments, the agent inhibiting the activation of HERVK locus is a gene editing system for the knockout or knockdown of the ERV locus, including but not limited to meganuclease, zinc finger nuclease (ZFN), transcription activator-like effector nuclease (TALEN) and CRISPR/Cas system.

In some embodiments, the agent inhibiting the activation of an ERV locus is a gene editing system for the knockout or knockdown of HERVK locus. In some embodiments, the agent inhibiting the activation of an ERV locus is an antisense nucleic acid or interfering nucleic acid (including but not limited to siRNA, shRNA and miRNA) for the knockdown or knockout of a nucleic acid molecule comprising one of SEQ ID NOs: 5-8, or a nucleotide sequence having a percent identity of at least 80%, 85%, 90%, 95%, 96%, 97%, 98%, 99%, 99.5%, 99.6%, 99.7%, 99.8% or 99.9% to one of SEQ ID NOs: 5-8. In some embodiments, the agent inhibiting the activation of the ERV locus encodes a shRNA of SEQ ID NO: 51 or 52.

In some embodiments, the agent inhibiting the ERV is a binding molecule which binds to HERVK-Env (e.g., the protein comprising SEQ ID NO: 1 or an amino acid sequence having a percent identity of at least 80%, 85%, 90%, 95%, 96%, 97%, 98%, 99%, 99.5%, 99.6%, 99.7%, 99.8% or 99.9% to SEQ ID NO: 1), for example, an antibody or the antigen-binding fragment or antibody derivative thereof.

The invention also provides a method for treating progeroid diseases, e.g., HGPS or WS, including administering an agent inhibiting the activation of HERVK locus or an agent inhibiting HERVK to the subject in need.

In some embodiments, the agent inhibiting the activation of HERVK locus promotes the methylation of HERVK locus by e.g., increasing activity of transmethylase. The inhibitor includes but is not limited to transmethylase or its coding polynucleotide.

In some embodiments, the agent inhibiting the activation of ERV locus is a non-specific retrovirus inhibitor, which includes e.g., a reverse transcriptase inhibitor, an integrase inhibitor and/or a protease inhibitor. In some embodiments, the reverse transcriptase inhibitor includes nucleoside type reverse transcriptase inhibitor such as enofovir, abacavir, stavudine (D4T), lamivudine (3TC) and/or zidovudine, and/or non-nucleoside type reverse transcriptase inhibitor such as efavirenz, etravirine and/or nevirapine. In some embodiments, the integrase inhibitor includes Raltegravir. In some embodiments, the protease inhibitor includes Lopinavir and/or Darunavir. In some embodiments, the retrovirus inhibitor includes lamivudine (3TC), abacavir and/or Raltegravir.

In some embodiments, the agent inhibiting the activation of ERV locus is a CRISPR-dCas system targeting the ERV locus and increasing the methylation.

In some embodiments, the agent inhibiting the activation of HERVK locus is a gene editing system for the knockout or knockdown of the HERVK locus, including but not limited to meganuclease, zinc finger nuclease (ZFN), transcription activator-like effector nuclease (TALEN) and CRISPR/Cas system.

In some embodiments, the agent inhibiting the activation of an ERV locus is a gene editing system for the knockout or knockdown of HERVK locus. In some embodiments, the agent inhibiting the activation of an ERV locus is an antisense nucleic acid or interfering nucleic acid (including but not limited to siRNA, shRNA and miRNA) for the knockdown or knockout of a nucleic acid molecule comprising one of SEQ ID NOs: 5-8, or a nucleotide sequence having a percent identity of at least 80%, 85%, 90%, 95%, 96%, 97%, 98%, 99%, 99.5%, 99.6%, 99.7%, 99.8% or 99.9% to one of SEQ ID NOs: 5-8. In some embodiments, the agent inhibiting the activation of the ERV locus encodes a shRNA of SEQ ID NO: 51 or 52.

In some embodiments, the agent inhibiting the HERVK is a binding molecule which binds to HERVK-Env (e.g., the protein comprising SEQ ID NO: 1 or an amino acid sequence having a percent identity of at least 80%, 85%, 90%, 95%, 96%, 97%, 98%, 99%, 99.5%, 99.6%, 99.7%, 99.8% or 99.9% to SEQ ID NO: 1), for example, an antibody or the antigen-binding fragment or antibody derivative thereof.

### 4. A kit for detecting aging

The invention provides a kit for identifying the degree of aging of a subject or a cell, tissue or organ thereof, or evaluating the physical age of a subject, or diagnosing progeroid diseases, comprising a detecting agent for detecting the activation level of an endogenous retrovirus (ERV) in the sample from the subject.

The invention also provides use of a detecting agent for detecting the activation level of an endogenous retrovirus (ERV) in the sample from a subject in the preparation of a kit for identifying the degree of aging of the subject or a cell, tissue or organ thereof, or evaluating the physical age of the subject, or diagnosing progeroid diseases.

In some embodiments, the ERV is selected from the group comprising families HERVK (including HERVK11), MER61, MER61C, Charlie4, MER101B, MamGyp, HERV16, HUERS-P2 and ERVW and homologs thereof. In some embodiments, the ERV is selected from the group comprising families HERVK (including HERVK11), MER61, MER61C, Charlie4, MER101B, MamGyp, HERV16, HUERS-P2, ERVW and MMTV and homologs thereof. In some embodiments, the ERV includes at least an ERV from HERVK family (including HERVK11), optionally, the ERV also includes ERV from the group comprising families MER61, MER61C, Charlie4, MER101B, MamGyp, HERV16, HUERS-P2 and ERVW and homologs thereof.

In some embodiments, the detecting agent is used for detecting the methylation level of the ERV locus, e.g., the methylation level of the flanking LTR region thereof. In some embodiments, the detecting agent comprises primer pairs comprising polynucleotides of SEQ ID NOs: 9 and 10.

In some embodiments, the detecting agent is used for detecting the transcript level of coding sequence of the ERV, or the level of the encoded protein thereof. In some embodiments, the coding sequence comprises Env, Pol, Pro and/or Gag genes in the ERV In some embodiments, the detecting agent is gene specific primer pair or probe for detecting the level of the transcript of the coding sequence. In some embodiments, the detecting agent is a protein-binding molecule for detecting the level of the protein encoded by the coding sequence. Protein-binding molecule includes any molecule capable of binding to a given protein, such as but not limited to an antibody, the antigen-binding fragment of an antibody and antibody derivative. In some embodiments, the antigen-binding fragment of an antibody includes but is not limited to a Fv fragment (e.g., single stranded Fv and disulfide bonded Fv) and a Fab-like fragment (e.g., Fab fragment, Fab' fragment and F(ab')₂ fragment).

In some embodiments, the subject is a non-human mammal. In some embodiments, the subject is a rodent. Preferably, the rodent is mouse. Preferably, the ERV is from MMTV family.

In some embodiments, the subject is a non-human mammal. In some embodiments, the subject is a non-human primate. Preferably, the non-human primate is an Old-World monkey. Preferably, the ERV is from ERVW family.

In some embodiments, the subject is a human. Preferably, the ERV is from HERVK family. In some embodiments, the detection is performed for HERVK coding region (HERVK-int)), e.g., its transcript. In some embodiments, the detection is performed for MER61 coding region (MER61-int), e.g.,its transcript. In some embodiments, the detection is performed for LTR region in MamGyp, e.g.,its transcript.

In some embodiments, the detecting agent is used for detecting the transcript or translated protein of the coding sequence in HERVK, including e.g., the transcript or translated protein of Env, Pol, Pro and/or Gag genes in HERVK. In some embodiments, the the transcript comprises a transcript of one of SEQ ID NOs: 5-8, or a nucleotide sequence having a percent identity of at least 80%, 85%, 90%, 95%, 96%, 97%, 98%, 99%, 99.5%, 99.6%, 99.7%, 99.8% or 99.9% to one of SEQ ID NOs: 5-8. In some embodiments, the protein is a protein comprising one of SEQ ID NOs: 1-4, or an amino acid sequence having a percent identity of at least 80%, 85%, 90%, 95%, 96%, 97%, 98%, 99%, 99.5%, 99.6%, 99.7%, 99.8% or 99.9% to one of SEQ ID NOs: 1-4.

In some embodiments, the kit comprises primer pairs comprising polynucleotides of SEQ ID NOs: 11 and 12, SEQ ID NOs: 13 and 14, SEQ ID NOs: 15 and 16, SEQ ID NOs: 17 and 18, SEQ ID NOs: 19 and 20, or SEQ ID NOs: 21 and 22. In some embodiments, the kit comprises a probe comprising a nucleotide sequence of SEQ ID NO: 60.

In some embodiments, the kit comprises a binding partner comprising one of SEQ ID NOs: 1-4, or a protein with an amino acid sequence having a percent identity of at least 80%, 85%, 90%, 95%, 96%, 97%, 98%, 99%, 99.5%, 99.6%, 99.7%, 99.8% or 99.9% to one of SEQ ID NOs: 1-4. Preferably, the binding partner comprises SEQ ID NO: 1, or a protein of amino acid sequence having a percent identity of at least 80%, 85%, 90%, 95%, 96%, 97%, 98%, 99%, 99.5%, 99.6%, 99.7%, 99.8% or 99.9% to SEQ ID NO: 1.

Samples which can be detected by the kit of the invention are the samples from cells or tissues of the subject, e.g.,, biopsy samples of various tissues . Samples which can be detected by the kit of the invention may also be body fluid samples, e.g., urine, saliva and blood, including whole blood, plasma or serum.

The invention also provides a kit for assessing the degree of senescence of an isolated cell or cell population comprising a detecting agent for detecting the activation level of an ERV in the cell.

The invention also provides use of a detecting agent for detecting the activation level of an ERV in a cell in the preparation of a kit for assessing the degree of senescence of an isolated cell or cell population.

In some embodiments, the ERV is selected from the group comprising families HERVK (including HERVK11), MER61, MER61C, Charlie4, MER101B, MamGyp, HERV16, HUERS-P2 and ERVW and homologs thereof. In some embodiments, the ERV is selected from the group comprising families HERVK (including HERVK11), MER61, MER61C, Charlie4, MER101B, MamGyp, HERV16, HUERS-P2, ERVW and MMTV and homologs thereof. In some embodiments, the ERV includes at least an ERV from HERVK family (including HERVK11), optionally, the ERV also includes ERV from the group comprising families MER61, MER61C, Charlie4, MER101B, MamGyp, HERV16, HUERS-P2 and ERVW and homologs thereof.

In some embodiments, the activation level of the ERV includes the methylation level of the ERV locus, the level of an ERV transcript, the level of an ERV protein, the presence, localization of the ERV particles in the cell and/or the release of the ERV particles from the cell, and/or the transposition probability of the ERV locus. In some embodiments, the activation level of the ERV includes the methylation level of the ERV locus, the level of an ERV transcript, the level of an ERV protein, and the presence, localization of the ERV particles in the cell and/or the release of the ERV particles from the cell. In some embodiments, the activation level of the ERV includes the transposition probability of the ERV locus. In some embodiments, the activation level of the ERV is presented by the localization of ERV particles in the cell.

In some embodiments, the detecting agent is used for detecting the methylation level of the ERV locus, e.g., the methylation level of the flanking LTR region thereof. In some embodiments, the detecting agent comprises primer pairs comprising polynucleotides of SEQ ID NOs: 9 and 10.

In some embodiments, the detecting agent is used for detecting the transcript level of coding sequence of the ERV, or the level of the encoded protein thereof. In some embodiments, the coding sequence comprises Env, Pol, Pro and/or Gag genes in the ERV In some embodiments, the detecting agent is gene specific primer pair or probe for detecting the level of the transcript of the coding sequence. In some embodiments, the detecting agent is a protein-binding molecule for detecting the level of the protein encoded by the coding sequence. Protein-binding molecule includes any molecule capable of binding to a given protein, such as but not limited to an antibody, the antigen-binding fragment of an antibody and antibody derivative. In some embodiments, the antigen-binding fragment of an antibody includes but is not limited to a Fv fragment (e.g., single stranded Fv and disulfide bonded Fv) and a Fab-like fragment (e.g., Fab fragment, Fab' fragment and F(ab')₂ fragment).

In some embodiments, the cell is a non-human mammal cell. In some embodiments, the cell is a murine cell, such as CHO and mouse cell. In some embodiments, the cell is a mouse cell, and the ERV is from MMTV family.

In some embodiments, the cell is a non-human primate cell. Preferably, the non-human primate is an Old-World monkey. For example, the cell is vero cell. Preferably, the ERV is from ERVW family.

In some embodiments, the cell is a human cell. Preferably, the ERV is fromHERVK family. In some embodiments, the detection is performed for HERVK coding region (HERVK-int)), e.g., its transcript. In some embodiments, the detection is performed for MER61 coding region (MER61-int), e.g., its transcript. In some embodiments, the detection is performed for LTR region in MamGyp, e.g., its transcript.

In some embodiments, the detecting agent is used for detecting transcript or translated protein of the coding sequence of HERVK, including e.g., the transcript or translated protein of Env, Pol, Pro and/or Gag genes in HERVK. In some embodiments, the transcript comprises a transcript of one of SEQ ID NOs: 5-8, or a nucleotide sequence having a percent identity of at least 80%, 85%, 90%, 95%, 96%, 97%, 98%, 99%, 99.5%, 99.6%, 99.7%, 99.8% or 99.9% to one of SEQ ID NOs: 5-8. In some embodiments, the protein is a protein comprising one of SEQ ID NOs: 1-4, or an amino acid sequence having a percent identity of at least 80%, 85%, 90%, 95%, 96%, 97%, 98%, 99%, 99.5%, 99.6%, 99.7%, 99.8% or 99.9% to one of SEQ ID NOs: 1-4.

In some embodiments, the kit comprises primer pairs comprising polynucleotides of SEQ ID NOs: 11 and 12, SEQ ID NOs: 13 and 14, SEQ ID NOs: 15 and 16, SEQ ID NOs: 17 and 18, SEQ ID NOs: 19 and 20, or SEQ ID NOs: 21 and 22. In some embodiments, the kit comprises a probe comprising a nucleotide sequence of SEQ ID NO: 60.

### 5. Pharmaceutical composition for regulating aging

The invention provides a pharmaceutical composition for treating the premature aging of a subject in need or a cell, tissue of organ thereof, or preventing or delaying aging of the subject or a cell, tissue of organ thereof, comprising an agent inhibiting the activation of an ERV locus or an agent inhibiting an ERV and a pharmaceutically acceptable excipient.

The invention also provides use of an agent inhibiting the activation of an ERV locus or an agent inhibiting an ERV in the preparation of a pharmaceutical composition for treating the premature aging of a subject in need or a cell, tissue of organ thereof, or preventing or delaying aging of a subject or a cell, tissue of organ thereof.

In some embodiments, the ERV is selected from the group comprising families HERVK (including HERVK11), MER61, MER61C, Charlie4, MER101B, MamGyp, HERV16, HUERS-P2 and ERVW and homologs thereof. In some embodiments, the ERV is selected from the group comprising families HERVK (including HERVK11), MER61, MER61C, Charlie4, MER101B, MamGyp, HERV16, HUERS-P2, ERVW and MMTV and homologs thereof. In some embodiments, the ERV includes at least an ERV from HERVK family (including HERVK11), optionally, the ERV also includes ERV from the group comprising families MER61, MER61C, Charlie4, MER101B, MamGyp, HERV16, HUERS-P2 and ERVW and homologs thereof. In some embodiments, the agent inhibiting the activation of the ERV locus includes but is not limited to transmethylase or its coding polynucleotide.

In some embodiments, the agent inhibiting the activation of an ERV locus is a non-specific retrovirus inhibitor, which includes e.g., a reverse transcriptase inhibitor, an integrase inhibitor and/or a protease inhibitor. In some embodiments, the reverse transcriptase inhibitor includes nucleoside type reverse transcriptase inhibitor such as enofovir, abacavir, stavudine (D4T), lamivudine (3TC) and/or zidovudine, and/or non-nucleoside type reverse transcriptase inhibitor such as efavirenz, etravirine and/or nevirapine. In some embodiments, the integrase inhibitor includes Raltegravir. In some embodiments, the protease inhibitor includes Lopinavir and/or Darunavir. In some embodiments, the retrovirus inhibitor includes lamivudine (3TC), abacavir and/or Raltegravir.

In some embodiments, the agent inhibiting the activation of an ERV locus is a CRISPR-dCas system targeting the ERV locus and increasing the methylation.

In some embodiments, the agent inhibiting the activation of an ERV locus is a gene editing system for the knockout or knockdown of the ERV locus, including but not limited to meganuclease, zinc finger nuclease (ZFN), transcription activator-like effector nuclease (TALEN) and CRISPR/Cas system.

In some embodiments, the agent inhibiting the activation of an ERV locus is an antisense nucleic acid or interfering nucleic acid (including but not limited to siRNA, shRNA and miRNA) for the knockout or knockdown of Env, Pol, Pro and/or Gag genes in ERV.

In some embodiments, the agent inhibiting ERV is a binding molecule which can bind to an ERV protein such as Env protein, e.g., an antibody or the antigen-binding fragment and antibody derivative thereof, preferably a neutralizing antibody or the antigen-binding fragment or antibody derivative thereof against the ERV

In some embodiments, the subject is a non-human mammal. In some embodiments, the subject is a rodent. Preferably, the rodent is mouse. Preferably, the ERV is from MMTV family.

In some embodiments, the subject is a non-human mammal. In some embodiments, the subject is a non-human primate. Preferably, the non-human primate is an Old-World monkey. Preferably, the ERV is from ERVW family.

In some embodiments, the subject is a human. Preferably, the ERV is from HERVK family.

In some embodiments, the agent inhibiting the activation of ERV locus is a gene editing system for the knockout or knockdown of HERVK locus. In some embodiments, the agent inhibiting the activation of an ERV locus is an antisense nucleic acid or interfering nucleic acid (including but not limited to siRNA, shRNA and miRNA) for the knockdown or knockout of a nucleic acid molecule comprising one of SEQ ID NOs: 5-8, or a nucleotide sequence having a percent identity of at least 80%, 85%, 90%, 95%, 96%, 97%, 98%, 99%, 99.5%, 99.6%, 99.7%, 99.8% or 99.9% to one of SEQ ID NOs: 5-8. In some embodiments, the agent inhibiting the activation of the ERV locus encodes a shRNA of SEQ ID NO: 51 or 52.

In some embodiments, the agent inhibiting the ERV is a binding molecule which binds to HERVK-Env (e.g., the protein comprising SEQ ID NO: 1 or an amino acid sequence having a percent identity of at least 80%, 85%, 90%, 95%, 96%, 97%, 98%, 99%, 99.5%, 99.6%, 99.7%, 99.8% or 99.9% to SEQ ID NO: 1), for example, an antibody or the antigen-binding fragment or antibody derivative thereof.

In some embodiments, the pharmaceutical composition is used for preventing or delaying tissue and organ degeneration, treating related diseases, delaying the aging of body, for example, preventing or delaying skin aging, treating or preventing osteoarthritis and frailty. In some embodiments, the pharmaceutical composition is used for topical administration, intradermal administration or subcutaneous administration.

In some embodiments, the pharmaceutical composition is used for treating progeroid diseases, e.g., HGPS or WS and aging-related diseases, including but not limited to arthritis, premature ovarian failure, hepatic fibrosis, pulmonary fibrosis and angiocardiopathy.

In some embodiments, the pharmaceutical composition is used for preventing or delaying skin aging. In some embodiments, the pharmaceutical composition is used for topical administration, intradermal administration or subcutaneous administration.

In some embodiments, the pharmaceutical composition is used for treating progeroid diseases, e.g., HGPS or WS.

The invention also provides a pharmaceutical composition for promoting cell senescence, comprising an agent promoting the activation of an ERV locus in the cell or the ERV particles.

The invention also provides use of an agent promoting the activation of an ERV locus in a cell or the ERV particles in the preparation of a pharmaceutical composition for promoting cell senescence.

In some embodiments, the ERV is selected from the group comprising families HERVK (including HERVK11), MER61, MER61C, Charlie4, MER101B, MamGyp, HERV16, HUERS-P2 and ERVW and homologs thereof. In some embodiments, the ERV is selected from the group comprising families HERVK (including HERVK11), MER61, MER61C, Charlie4, MER101B, MamGyp, HERV16, HUERS-P2, ERVW and MMTV and homologs thereof. In some embodiments, the ERV includes at least an ERV from HERVK family (including HERVK11), optionally, the ERV also includes ERV from the group comprising families MER61, MER61C, Charlie4, MER101B, MamGyp, HERV16, HUERS-P2 and ERVW and homologs thereof. In some embodiments, the promoting agent is a transcriptional activation system targeting the ERV locus. In some embodiments, the transcriptional activation system is a CRISPR-dCas transcriptional activation system. In some embodiments, the specific promoting agent is a demethylation system targeting the ERV locus. In some embodiments, the demethylation system is a demethylation system based on CRISPR-dCas, e.g., CRISPR-dCas-Tet.

In some embodiments, the promoting agent is a DNA methyltransferase inhibitor, e.g., decitabine, Lomeguatrib, SGI-1027 or 5-azacytidine.

In some embodiments, the cell is a non-human mammal cell. In some embodiments, the cell is a rodent cell. In some embodiments, the cell is a mouse cell. In some embodiments, the ERV is from MMTV family.

In some embodiments, the cell is a non-human mammal cell. In some embodiments, the cell is a non-human primate cell. In some embodiments, the non-human primate is an Old-World monkey. In some embodiments, the ERV is from ERVW family.

In some embodiments, the cell is a human cell, e.g., tumor cell, infected cell or cells in damaged tissues. Preferably, the ERV is from HERVK family. In some embodiments, the sgRNA in the CRISPR-dCas transcriptional activation system comprises SEQ ID NO: 59.

### Examples

### Materials and Methods

Unless otherwise specified, experimental operations in the Examples of the application are performed according to the conventional methods in the art. The following materials and methods are intended to illustrate, rather than to limit the invention.

### Animal and Human Samples

C57BL/6J mice were purchased from SPF (Beijing) BIOTECHNOLOGY Co., Ltd., raised in cages with ventilation in the animal room in the Institute of Biophysics and Institute of Zoology, Chinese Academy of Sciences, and fed with standard laboratory feed and water, under 12h/12h light and dark cycle.

All mice experiments followed "Formal Principles for the Application of the Ethical Approval of Animal Research", and were approved by the Animal Care and Use Committee, Institute of Zoology, Chinese Academy of Sciences in advance.

Mice were sacrificed by CO₂ for euthanasia, followed by cervical dislocation.

All cynomolgus monkey experiments were performed according to the principles for the humane treatment of non-human primates, and approved of the Animal Care and Use Committee, Institute of Zoology, Chinese Academy of Sciences.

Young cynomolgus monkeys and old cynomolgus monkeys used in the experiments were all raised in Beijing Institute of Xieerxin Biological Resource, which was approved by Laboratory Animal Care accredited facility in Beijing, and complied with all local and national laws regarding animal researches.

Lung, liver and skin samples were collected from 8 young and old cynomolgus monkeys without clinical or experimental history.

HGPS cynomolgus monkeys were raised in Yunnan Key Laboratory of Primate Biomedical Research (LPBR), and ethical animal experiments were conducted according to the regulations of International Association for Assessment and Accreditation of Laboratory Animal Care (AAALAC) (68).

Primary hMPCs were isolated from gums of individual with specified age with the approval by the Ethics Committee of the 306th Hospital of PLA in Beijing.

Human eyelid skin samples were obtained by blepharoplasty from specified healthy donor with the ethical approval of the Peking Union Medical College Hospital Institutional Review Board.

Human serum samples were collected with the approval of the Research Ethics Committee of the First Affiliated Hospital of Kunming Medical University.

### Mice Experiments

### 1. Knockdown of MMTV

To assess whether the knockdown of MMTV alleviates joint degeneration related with mice aging, lentiviruses expressing Cas9KRAB empty vector (n=12) or Cas9KRAB-sgMMTV (n=12) were injected to the articular cavities of mice of 21 months old, and supplementarily injected after 4 weeks. Grip strength test was perrformed 4 weeks and 8 weeks after the first injection. Motor coordination and balance, motor ability and Micro-CT (micro computed tomography) were performed 10 weeks after the first injection, samples were taken, i.e., joints of mice hind limbs were taken out, one was frozen with liquid nitrogen and the other was fixed in 4% PFA.

### 2. Treating Joint degeneration Associated with aging with Abacavir

10µl Abacavir (in PBS with 2% DMSO) at the concentration of 10 mg/ml was injected to knee joint cavities of mice of 22 months old (n=12), injected once a week. Grip strength test was performed 4 weeks, 8 weeks and 12 weeks after the first injection. Motor coordination and balance, motor ability and Micro-CT were performed 14 weeks after the first injection, samples were taken, i.e., joints of mice hind limbs were taken out, one was frozen in liquid nitrogen and the other was fixed in 4% PFA.

### 3. Long-term oral administration of Abacavir

Abacavir was dissolved in drinking water containing 0.4% DMSO at 2 mg/ml, mice of 18 months old were administered by feeding the water daily (n=23). The weight, hollow back, hair, skin, eye (cataract) and activity score of mice were measured 6 weeks, 12 weeks and 24 weeks after administration, grip strength was measured and a survival curve was plotted. Motor coordination and balance, motor ability and Y Maze behavior test were performed 24 weeks after administration.

**Table 1. Mice phenotype scoring criteria**

| | Hair | Skin | Hollow back | Eye | Motor ability |
|---|---|---|---|---|---|
| 1 | Smooth and glossy hair | Intact skin without erythema | Normal | Normal | Drooping, closed eyes |
| 2 | Bristled and white hair | Very slight erythema, barely visible | Visible hollow back at rest | Pinpoint white spots in the lens | No response to capture |
| 3 | Periocular or face unhairing | Apparent erythema | Visible hollow back in a relaxed state | Completely covered lens by white spots | Decreased activity during capture |
| 4 | Back unhairing with visible skin | Moderate to severe erythema | Visible hollow back at activation | Completely white eyes | Decreased spontaneous activity |
| 5 | Extensive back unhairing with bare skin | Formation of skin damage scab | Dying with hollow back | Completely white eyes and the release of milky fluid | Free and brisk movement |

### 4. Long-term injection of Ratelgravir

Ratelgravir was dissolved at 50 mg/ml in 30%PEG300+ 2% Tween80 + 2%DMSO, and intraperitoneally injected to mice at 18 months old, 200µl each time, twice a week (n=23). The weight, hollow back, hair, skin, cataract and activity score of mice were measured 8 weeks and 16 weeks after first injection, grip strength was measured and a survival curve was plotted.

### 5. Grip strength test

Grip strength of the limbs was measured by Grip strength meter (Panlab Grip Strength Meter, LE902) in accordance with the manufacturer's instructions. In brief, mice were put on the top of grip strength meter and pulled along the grid at a constant rate, until mice loosened its grip. The peak tension in each of the 10 repeats was recorded, and the mean value was calculated as grip strength for each mouse.

### 6. Forced treadmill testing

Mice were placed on a running machine (SANS Bio Instrument, SA101) to test the motor ability of mice in accordance with the manufacturer's instructions. In particular, slope of runway was set as 5°, electrical stimulation was 2 mA, the running speed was set as follows: an initial velocity of 5 m/min for 5 min; then accelerating to final velocity of 25 m/min with an accelerated rate of 1 m/min², 20 mins in total. First, mice were trained for three consecutive days, and tested on the fourth day. The times of electrical stimulation, and the run distance in 25 min were recorded.

### 7. Rotor rod test

Motor coordination and balance of mice was measured by Rota Rod system (Yiyan Tech, YLS-4C) in accordance with the manufacturer's instructions. In brief, mice were put into different channels on the rod, the initial velocity of the rotor rod was 4 rpm/min, which was then accelerated to 44 rpm/min with an accelerated rate 8 rpm/min², until mice fell down, each mouse was trained for 3 times. Mice were trained for three consecutive days and tested on the fourth day, each mouse was tested for 3 times, the times were recorded after mice fell down.

### 8. Micro-CT (micro computed tomography)

Micro-CT (PE Quantum FX) was performed in accordance with the manufacturer's instructions. In brief, mice were anesthetized and placed in the instrument, scanning parameters for Micro-CT were voltage 50Kvp, power 40W, 6-frame superposition, field 4cm×4cm. Three-dimensional images were screenshot after scan and reconstruction, and bone density was calculated by Ansys software.

### 9. Y maze test

Mice were placed in the end of any arm of Y maze (trisection radiation maze, consisting of 3 arms with equal length (50cm×18cm×35cm), the intersection angle between every two arms was 120 degree), allowed to explore freely for 5 min, animal behavior changes in 5 min were recorded by camera system, and the short-term spatial memory ability of mice was detected by recording the total times that the mice entered the arms and the rotations that the mice entered in turn the three arms of the Y maze.

### Cell Culture

HEK293T cells and human fibroblasts were cultured in Dulbecco Modified Eagle Medium (DMEM, Thermo Fisher Scientific), supplemented with 10% fetal calf serum (FBS, Gibco, Thermo Fisher Scientific), 2 mmol/L GlutaMAX(Thermo Fisher Scientific), 0.1 mmol/L non-essential amino acid (NEAA, Thermo Fisher Scientific), 1% penicillin/streptomycin (Thermo Fisher Scientific).

Human MPCs (hMPC) were grown in hMPCs medium contaning 90% MEM Alpha Medium (Thermo Fisher Scientific), 10%FBS, 2 mmol/L GlutaMAX, 0.1 mmol/L NEEA, 1% penicillin/streptomycin and 1 ng/mL bFGF2 (Joint Protein Central) in a plate (CORNING) coated with 0.1% gelatin (Sigma Aldrich).

Cells were cultured in an incubator (Thermo Fisher Scientific) at 37°C, 5%CO₂.

### Western Blot

Cells were lysed in 1×SDS buffer (100 mM Tris-HCl, pH 6.8, 10% glycerol, 2%SDS and 2%2-mercaptoethanol), boiled for 10 min at 105°C. Protein concentration was measured by BCA kit. Cell lysate was subjected to SDS-PAGE electrophoresis, then transferred to PVDF membrane (Millipore). The membrane was incubated with primary antibody, incubated with secondary antibody conjugated to HRP after washing, then blotted with substrate, the substrate was the mixture of 1000µL substrate A and 3µL substrate B (substrate A: 0.2 mM cumaric acid, 1.25 mM luminal, 0.1 M Tris-HCl, pH 8.5; substrate B: 3% H₂O₂) or Super Signal West Femto Maximum Sensitivity Substrate (Thermo Fisher Scientific). Images were taken by Image Lab software. Quantification was conducted by ImageJ.

### Immunoprecipitation (IP) of HERVK from Medium

Cells were grown in hMPC medium containing microbubble-depleted fetal calf serum (dFBS) for 48h. Conditioned medium was collected, filtered by 0.2 µm filter (Pall Corporation), and pre-rinsed with Protein A/G Agarose beads at 4°C for 2-3h. Supernatant was incubated with 1/1000 volume of HERVK-Env antibody and fresh Protein A/G Agarose beads at 4°C, followed by collecting supernatant. The beads were washed by PBS for western blot.

### Purification of Microbubble Particle from Medium

Before collecting medium, cells were grown in hMPC medium containing microbubble-depleted fetal calf serum (dFBS) for 48h. Medium was collected and the cells were counted. Medium from the same cell number was used for microbubble purification, the purification was performed as follows: centrifuged at 300×g for 10 min to remove cell contaminants, centrifuged at 15,000×g for 20 min, filtered by 0.2 µm filter (Pall Corporation), ultracentrifuged at 110,000×g for 2h. Precipitates, i.e., the microbubble were collected, and dissolved in PBS with the same volume for the isolation of viral RNA or western blot.

### Isolation of Cell DNA/RNA and (Reverse Transcription) Quantitative PCR((RT-)qPCR)

In accordance with the manufacturer's instructions, total genomic DNA of cell was extracted with DNA extraction kit (Tiangen).

In accordance with the manufacturer's instructions, total RNA of cell was extracted by TRIzol (Thermo Fisher Scientific); reverse transcribed into cDNA with GoScript reverse transcription system (Promega) for RT-qPCR; RT-qPCR was conducted with THUNDERBIRD qPCR Mix(TOYOBO) and CFX384 real-time system (Bio-Rad Laboratories, Inc.).

### Isolation of Viral RNA from Medium

In accordance with the manufacturer's instructions, RNA was extracted from 140µL microbubble with QIAamp viral RNA mini Kit (Qiagen).

In brief, samples were incubated with prepared buffer AVL and vector RNA. After adding ethanol, samples were purified by QIAamp Mini column, washed, eluted, then treated with DNase I (in 2.4µL reaction buffer at 37°C, 20µL virus RNA was treated by 2µL DNase I, 30 min). DNase I was inactivated by inactivating reagents provided in the kit and removed, afterwards, centrifuged at 10,000×g for 1.5 min, supernatant was heated at 70°C for 10 min to remove any residual activity of DNase.

### Droplet Digital PCR (ddPCR)

In accordance with the manufacturer's instructions, viral RNA purified from microbubble was reverse transcribed to cDNA with GoScript reverse transcription system (Promega) for ddPCR.

In brief, 20µL reactive solution containing template, primer and QX200 ddPCR EvaGreen Supermix (Bio-Rad Laboratories, Inc.) mixed with 70µL DG Oil (Bio-Rad Laboratories, Inc.) in DG8 cartridge (Bio-Rad Laboratories) by QX200 droplet generator (Bio-Rad Laboratories, Inc.). Droplets were transferred to 96-well plate, sealed by PX1^{™} PCR Plate Sealer (Bio-Rad Laboratories, Inc.). PCR amplification was performed in thermal cycler (Bio-Rad Laboratories, Inc.), then samples were placed in QX200 droplet reader (Bio-Rad Laboratories, Inc.) to analyze the absolute copy number of HERVK in microbubble.

### ELISA

For ELISA of IL6, medium was collected and filtered by 0.2 µm filter, then incubated in the plate (BioLegend, in accordance with the manufacturer's instructions, precoated overnight at 4°C before use) coated with IL6 antibody. Afterwards, the plate was incubated with detection antibody, avidin-HRP and fresh mixed TMB substrate. After adding termination solution, the plate was measured at 450 nm with Synergy H1 (BioTek).

To measure HERVK-Env protein level in medium or serum, first, medium or human serum was concentrated by 100 times or 10 times with Centricon Plus-70 (Millipore) or Amicon Ultra-15 (Millipore), then measurement was conducted with HERVK_7p22.1 Provirus Ancestral Env Polypretein (ERVK6) ELISA Kit in accordance with the manufacturer's instructions. In brief, concentrated medium or serum (100µL) was added to coated plate, incubated at 37°C for 2h, incubated with biotin antibody, avidin-HRP, then fresh mixed TMB substrate and termination solution was added, and measurement was conducted in 450 nm with Synergy H1 (BioTek) at 450 nm.

The level of 2'3'-cGAMP in cell was measured by 2'3'-cGAMP ELISA kit. In brief, 50µL cell lysate (lysed in RIPA buffer) from the same number of specified cell lines was add to the plate, then adding 2'3'-cGAMP HRP tracer agent, 2'3'-cGAMP polyclonal antiserum. Shaked at room temperature. After incubating with TMB substrate and termination solution, the plate was measured by Synergy H1(BioTek) at 450 nm.

### Immunofluorescence, Immunohistochemical Staining and Microscopy

Cells seeded on coverslip (Thermo Fisher Scientific) were washed by PBS, fixed in 4% paraformaldehyde (PFA), permeabilized in 0.4%Triton X-100, blocked by 10% donkey serum. The coverslip was incubated with primary antibody at 4°C in blocking buffer, then incubated with secondary antibody at room temperature for 1h. Nucleus was labelled by Hoechst 33342 (Invitrogen).

In accordance with the manufacturer's instructions, tissue sections were immunohistochemically stained with DAB Staining Kit (ZSGB-BIO). In brief, the section dewaxed with xylene and ethanol was incubated with 3% H₂O₂ solution together to block the activity of endogenous peroxidase, then antigen was recovered with 10 mM sodium citrate buffer (pH 6.0). Next, the section was permeabilized, blocked, incubated with primary antibody at 4°C overnight, then incubated with secondary antibody for 1h, and next, stained by DAB substrate solution and hematoxylin, dehydrated and fixed.

Images were taken with Leica SP5 confocal microscope or LEICA Aperio CS2.

### RNA/DNA-Fluorescence in situ Hybridization (FISH)

In accordance with the manufacturer's instructions (Thermo Fisher Scientific), RNA-FISH was conducted with QuantiGene^{®} ViewRNA ISH Cell Assay Kit.

In brief, cells seeded on coverslip (Thermo Fisher Scientific) were fixed in by fresh 4% formaldehyde solution, permeabilized by detergent solution QC, and digested by protease QC. Probe HERVK Alexa Fluor 488 (Thermo Fisher Scientific) was diluted in Probe Set Diluent QF, and added to wells, incubated in HB-1000 hybridizer (Gene Company Limited) at 41+1°C for 30 min. The coverslip was incubated with Working Pre-Amplifier Mix Solution in HB-1000 hybridizer at 40+ 1 °C for 30 min, incubated with Working Amplifier Mix Solution for 30 min, and incubated with Working Label Probe Mix Solution for 30 min, washed by PBS in each step. Cell nucleus was labelled by DAPI solution, the coverslip was fixed, images were taken with Leica SP5 confocal microscope.

The experiment for detecting DNA of HERVK gene by single molecule DNA-FISH was conducted by the National Key Laboratory of Agricultural Microbiology, Huazhong Agricultural University. Senescent cells were successively treated by RNase A (100µg/mL, dissolved in 2×SSC) at 37°C for 1h, treated by 70% formamide dissolved in 2×SSC at 72°C for 10 min, treated by precooled ethanol (concentration from 80%, 90% to 100%, each treament for 1 min). Then cells were incubated with HERVK 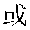 ACTIN specific probe pairs (each probe 10 µM) in hybridization solution (10% deionized formamide, 2×SSC, 10% dextran sulfate and 2 mM VRC) at 42°C overnight. Washed 3 times by washing buffer (2× SSC, 30% formamide, 0.1% Triton-X 100 and 2 mM VRC), cells were incubated with amplification probes (SFSP-001 and SFTP-001, Spatial FISH, Co., Ltd.) and fluorescent probes (SFFP-001, Spatial FISH, Co., Ltd) together, HERVK DNA was subjected to single molecule detection. Finally, cells were counterstained by DAPI and imaged by confocal microscope.

### Flow Cytometric Fluorescence Activated Cell Sortinge (FACS)

The proportion of GFP positive cells was analyzed, or GFP positive cells were sorted, by flow cytometer.

In brief, to assess transposition events occurred in HERVK during aging process, wild type mesenchymal stem cells of early passage and late passage (P7 generation and P14 generation) were transfected with HERVK reporter plasmid, and meanwhile co-transfected with dsRed, a vector expressing red fluorescence (obtained from Xu Xingzhi's Laboratory, School of Basic Medicine, Shenzhen University) to calculate transfection probability. After the confluence of cells, cells were digested into single cell, and resuspended by PBS, analyzed by flow cytometer BD Influx, the ratio of cells positive for green fluorescence to cells positive for red fluorescence was the probability of transposition events occurred in HERVK.

To sort cells infected with HERVK viral particles, wild type mesenchymal stem cells were infected with purified HERVK viral particles having green fluorescence label, after 48h, cells negative and positive for green fluorescence were sorted by overspeed flow sorter Astrios EQ.

### Transmission Electron Microscopy (TEM)

Cells were precipitated and fixed by 2.5% (v/v) glutaraldehyde dissolved in phosphate buffer (PB, 0.1 M, pH 7.4) for 20 min, placed at 4°C overnight. Conventional heavy metal staining was performed.

In brief, cells were fixed in 1% (w/v) osmium tetroxide dissolved in PB at 4°C for 2h, placed in pure acetone after serial dehydration by graded ethanol, soaked in graded mixture of acetone and resin, then embedded in pure resin containing 1.5% BDMA, and polymerized, 45°C for 12h, 60°C for 48h. Ultrathin sections (70 nm in thickness) were made by slicer (Leica EM UC6), double stained by uranyl acetate and lead citrate.

For Immuno-TEM, cell were seeded in 35 mm culture dish (CORNING), fixed in 4% formaldehyde at room temperature, 4°C overnight, then changed to 1% formaldehyde. Samples were dehydrated by an ethanol gradient, soaked by LR Gold resin gradient and embedded in LR Gold resin containing initiator. Afterwards, samples were polymerized using Leica AFS2 at -20°C by ultraviolet light for 24h, and sections were sliced by Leica EM UC7. For staining, sections were blocked in 2% BSA (Jackson ImmunoResearch), incubated with murine anit-HERVK-Env together at room temperature for 2h, washed by PB buffer containing 0.1% fresh cold gelatin for 5×2 min, incubated with 6 nm gold labelled anti-mouse secondary antibody (Jackson ImmunoResearch) together at room temperature for 1h, then fixed in 1% glutaraldehyde dissolved in ddH₂O. After washing by ddH₂O, sections were stained by 2% uranyl acetate at room temperature for 30 min, and then, imaged with TEM Spirit 120 kV (FEI Tecnai Spirit 120 kV).

### Plasmid Construction

To generate HERVK and STING knockdown vectors, specific shRNA was cloned to MluI/ClaI site in lentivirus vector pLVTHM plasmid (addgene# 12247).

To perform TBK1 knockout by CRISPR/Cas9, sgRNA targeting TBK1 was cloned to lenti-pMK1334 (addgene # 127965) through BstXI/BlpI site, and co-transfected by hSpCas9567 expression cassette with lenti-CRISPR v2 (addgene, # 52961).

To activate endogenous HERVK, non-targeted control or lentivirus construct with HERVK LTR targeted sgRNA was cloned to lenti-SAM v2 (addgene # 75112) through ESP3I site, and co-transfected with lenti-MPH v2 (addgene# 89308).

To inhibit endogenous HERVK, a vector targeting HERVK was constructed based on an inhibiting system of Cas9KRAB (Cas9KRABi). The vector expressing Cas9KRAB was from Dr. Didier Trono's laboratory (École Polytechnique Fédérale de Lausanne (EPFL), Lausanne, Swiss).

To inhibit endogenous MMTV, sgRNA clone targeting MMTV was cloned to an empty vector expressing Cas9KRAB.

To construct full length HERVK plasmid, according to previously published sequencing results (See Marie Dewannieux, Genome Reserch, 2006, Young Nam Lee, PLoS Pathogens, 2007), full length HERVK fragment (SEQ ID NO: 150) was synthesized, and cloned to mutant pEGFP-N3 vector having KpnI enzyme site through NheI/ApaI site (pEGFP-N3-KpnI, obtained from Xu Xingzhi's Laboratory, School of Basic Medicine, Shenzhen University), and vector pEGFP-N3-HERVK was obtained.

To construct HERVK with green fluorescence label, CMV-EGFP fragment (SEQ ID NO: 153) was PCR amplified from pEGFP-C1 (obtained from Xu Xingzhi Laboratory, School of Basic Medicine, Shenzhen University), and CMV-EGFP fragment was inserted to pEGFP-N3-HERVK through KpnI site, constructing GFP-HERVK. Using pEGFP-N3-HERVK as template, env, pol, gag, pro (SEQ ID NOs: 5-8) and rev (SEQ ID NO: 149) genes in HERVK were amplified by PCR, and inserted into pEGFP-N3-KpnI to obtain plasmid pEGFP-N3-gag-pro-pol (expressing gag, pro and pol genes), pEGFP-N3-Env (expressing env gene) and pEGFP-N3-Rev (expressing rev gene).

To construct HERVK transposition reporter vector, first, above-mentioned HERVK fragment was cloned to dsRed vector having KpnI enzyme site mutant (obtained from Xu Xingzhi's Laboratory, School of Basic Medicine, Shenzhen University). GFP reporter (SEQ ID NO: 152) was obtained by PCR amplification from L1 reporter (gifted by Prof. Wang Jichang, Sun Yat-sen University in Guangzhou, China), GFP reporter was inserted to dsRed - HERVK through KpnI site, contracting HERVK transposition reporter vector.

### Lentivirus Production

To package lentivirus construct, HEK293T cells were transfected by lentivirus vector and packaging plasmid pMD2.G(addgene, # 12260) and psPAX2(addgene, # 12259) using lipofectamine 3000(Invitrogen). Supernatant containing lentivirus was harvested 48h and 72h after transfection, filtered by 0.2 µm filter, concentrated by ultracentrifugation at 19,400 rpm for 2.5h. Suspended virus was precipitated and virus titer was evaluated.

### HERVK Virus Production

To package HERVK virus, HEK293T cells were transfected by GFP-HERVK as well as plasmids pEGFP-N3-gag-pro-pol, pEGFP-N3-Env and pEGFP-N3-Rev using lipofectamine 3000(Invitrogen). Supernatant containing HERVK virus was harvested 48h and 72h after transfection, filtered by 0.2 µm filter, concentrated by ultracentrifugation at 100,000g for 2h. Suspended virus was precipitated and virus titer was evaluated.

### Transposition Event

To assess transposition events occurred for HERVK during aging process, cells of early and late generations were transfected with HERVK reporter plasmid, and meanwhile co-transfected with a vector expressing red fluorescence to calculate transfection probability. After confluence of cells, cells were digested into single cell, and resuspended with PBS, analyzed by flow cytometer BD Influx, the ratio of cells positive for green fluorescence to cells positive for red fluorescence was the probability of transposition events occurred in HERVK.

### Cell Treatment

For 5-azacytidine (5-AZA) treatment, hMPCs were seeded (generation 0 (P0) after treatment), and treated by 500µg/µL 5-AZA for 4 days, followed by passage and culture in fresh medium. At P2 after treatment (6 days after 5-AZA treatment), cells was used for Ki67 immunofluorescence, SA-β-gal staining and RNA/DNA extraction.

For treatment by endogenous viral reverse transcriptase inhibitor and integrase inhibitor, hMPCs were treated by 10µM abacavir, lamivudine (3TC) and Raltegravir, respectively, medium was replaced every 3 days, followed by new treatment. After treatment of two cell passages, cells were used for Ki67 immunofluorescence, SA-β-gal staining and RNA/DNA extraction.

For HERVK RVLP infection, hMPCs were seeded (P0 after treatment) and incubated with HERVK RVLP in the presence of polybrene (Sigma-Aldrich) for 24h. To increase HERVK infection efficiency, hMPCs were centrifuged at 1200g for 2.5 h. Two days after infection, GFP positive cells were sorted by FACS. After infection of two-passage cells, cells were used for Ki67 immunofluorescence, SA-β-gal staining and RNA/DNA extraction.

For lentivirus transduction, hMPCs were seeded (P0 after treatment) and incubated with lentivirus in the presence of polybrene (Sigma-Aldrich) for 24h. After two passages, treated cells were used for Ki67 immunofluorescence, SA-β-gal staining and RNA/DNA extraction.

For conditioned medium treatment, 50% conditioned medium +50% fresh medium (in the presence of polybrene (Sigma-Aldrich)) were used to culture WT hMPCs in early passage, new conditioned medium was replaced every three days. Cells were passaged and used for Ki67, SA-β-gal staining and RNA/DNA extraction.

To detect whether retrovirus-like particles (RVLP) from CM attached to the surface of young cells, the cells were treated by CM for 12h, followed by TEM analysis.

To culture cells with combined human serum of young people (18-25 years old, n = 20) or old people (65-80 years old, n = 40), primary hMPCs were pre-cultured in normal hMPC medium for 24h, washed by PBS for 3 times, then cultured twice in the medium containing 10% human serum.

### Clonal Expansion Test

5 thousand cells were seeded in one well of 6-well plate (Corning) cover with gelatin (Sigma), and cultured for about 10 days. Cells were fixed by 4% PFA, stained by 10% crystal violet for 30 min. Relative cell integral density was calculated by ImageJ software.

### SA-β-gal staining

Cells were fixed by a buffer containing 2% (w/v) formaldehyde and 0.2% (w/v) glutaraldehyde for 5 min, and treated by staining buffer containing 1mg/mL X-gal at 37°C overnight. Stained cells were observed with optical microscope, and the percentage of positive cells was analyzed by ImageJ software.

### Chromatin Immunoprecipitation (ChIP)-qPCR

Cells were fixed in 1% formaldehyde dissolved in PBS at room temperature for 10 min, and then, quenched by 125 mM glycine. Cells were lysed on ice for 10 min, and subjected to ultrasonic treatment with Covaris S220. Collected supernatant was incubated with Dynabeads Protein A (Life Technologies, 10001D) pre-mixed with 2.3 µg specified antibody at 4°C overnight. After washing, samples were digested by Protease K(New England Biolabs), eluted and cross-link-reversed on thermal mixer at 68°C for 2h. DNA was extracted with phenol-chloroform-isoamylol, and eluted DNA was analyzed by qPCR.

To immunoprecipitate cGAS, cytoplasmic constituents of cells were extracted after fixing, which was incubated with cGAS antibody and Dynabeads Protein A at 4°C overnight. DNA was extracted as described above. qPCR analysis of 5S rDNA ( in principle not present in the cytoplasm) was used to exclude nuclear genome contamination.

### Construction and Sequencing of RNA-seq Library

Total RNA was extracted by TRIzol reagent, per repeated 1×10⁶ cells. genomic DNA was removed and mRNA was isolated. Then the preparation of library, quality control and high-throughput sequencing were conducted by Novogene Bioinformatics Technology Co.Ltd. Wherein, the library was constructed by NEBNext Ultra RNA Library Prep Kit for Illumina (NEB)) in accordance with the manufacturer's instructions; according to the manufacturer's instructions, high-throughput sequencing was conducted in HiSeq X 10 platform.

### RNA-seq Data Processing

The original reads of the end were trimmed by TrimGalore (version 0.4.5, Babraham Bioinformatics, https://github.com/FelixKrueger/TrimGalore), and mapped to human (Homo sapiens) hg19 or cynomolgus monkey (Macaca Fascicularis) MacFas5.0 reference genomes obtained from UCSC Genome Browser database by hisat2 (version 2.0.4). Then, reads mapped to each gene were counted by HTSeq (version 0.11.0) with reads of high quality mapping (mapping quality score above 20). Differentially expressed genes were calculated by DESeq2 R package (version 1.29.8), for hMPC, critical value was "|log2(fold change)|> 0.5, and adjusted P value<0.05"; for cynomolgus monkey tissue, critical value was "|log2(fold change)|> 0.5 and P value<0.05". GO and path enrichment analysis were performed in metascape. Senescence-associated secretory phenotype (SASP) was from previous studies. Gene enrichment analysis was performed by desktop GSEA application program (version 2.2.4).

### Analysis of expression level of repeat elements

To assess expression level of repetitive elements, Repenrich2 pipeline was conducted. Then, differentially expressed repetitive elements were calculated by R package edgeR (version 3.30.3), critical value was "| log2(fold change)|> 0.1 and FDR <0.05". The category of repetitive elements was noted in RepeatMasker, may be divided into LTR, LINE, SINE, DNA (also called DNA transposon), Satellite and some small RNA repeats, including tRNA, rRNA, scRNA and snRNA category. Differentially expressed repetitive elements were subjected to family enrichment analysis with Fisher exact test in R (version 4.0.2).

### Analysis of Expression Level of HERVK Provirus (HML2)

First, 91 HERVK provirus specific fasta files were obtained from NCBI database (GenBank ID: JN675007-JN675097), the files were annoted in previous studies. To evaluate the transcription levels of HERVK provirus in prematurely senescent and RS hMPC, 91 HERVK provirus fasta files were connected to hg19 (GRCh37.75) cDNA fasta file annoted in Ensembl database. Afterwards, mapping index was generated by Salmon (version 0.8.2) and cleaned RNA-seq read, the read was mapped to linking index file of hg19 and HERVK provirus by Salmon quantitative program with parameter "-1 A --gcBias". Next, read counts mapped to HERVK provirus sequence were obtained, differentially expressed HERVK provirus locus was calculated by R package DESeq2 (version 1.29.8), critical value was "| log2(fold change)| > 0.1, Benjamini-Hochberg adjusted P value<0.05". TPM (Transcripts per kilobase per million mapped reads) of HERVK provirus locus was calculated by Salmon quantitative program, visualized by ggpubr R package (version 0.4.0) in R (version 4.0.2), p value in each comparision was assessed by t test of unpaired two samples. Expression level was calculated for packing size of each 10 bp in the genome (Reads Per Kilobase per Million mapped reads, RPKM), visualized in IGV (version 2.7.2).

### Construction and Sequencing of Whole Genome Bisulfite Sequencing (WGBS) library

Genomic DNA was extracted from cells by asy Blood & Tissue Kits (QIAGEN), per repeated 2×10⁶ cells, cut to 100-300 bp by ultrasonic apparatus. Then the preparation of library, bisulfite treatment, quality control and sequencing were conducted by Novogene Bioinformatics Technology Co. Ltd.

### Data Processing of Whole Genome Bisulfite Sequencing

Original sequencing reads were trimmed by fastp software (version 0.19.10) using default parameters, using bsmap (version 2.90) and parameter " -v 0.1 -g 1 -R -u" (89), cleaned reads were mapped to human hg19 reference genome obtained from UCSC Genome Browser database. CpG DNA methylation level in each cytosine site was calculated by methratio program provided by bsmap. To ensure accuracy of methylation level, forward and reverse chain reads of each cytosine site were combined, and only CpG site with depth>5 was retained for downstream analysis. To calculate relative CpG DNA methylation level in repetitive element loci, genomic loci of repetitive element noted in each RepeatMasker were obtained from UCSC Genome Browser. Then average CpG DNA methylation level of repetitive element noted in each RepeatMasker was calculated, the statistic analysis was conducted by ggpubr R package (version 0.4.0) in R(version 4.0.2). P value was assessed by t test of two paired samples.

### Statistic Analysis

Statistic analysis was conducted to all data was with PRISM version 8 software (GraphPad software). The result was expressed as mean ± SEM. Comparision was performed with two-tailed Student's t test and one-way anova. P value <0.05 was regarded as having statistical significance (^{∗}), P value <0.01 was regarded as having high statistical significance (^{∗∗}), P value <0.001 was regarded as having high statistical significance (^{∗∗∗}).

### Primers, shRNAs and sgRNAs

Primer, shRNA, sgRNA sequences and probe sequences used in the Examples of the application were shown in the following Table 2.

**Table 2**

| **Primer** | | | | |
|---|---|---|---|---|
| **Amplicon** | **F (5'-->3')** | **R (5'-->3')** | **Use** | |
| LTR5HS | GGGCAGCAATACTGC TTTGT (SEQ ID NO: 9) | CAATAGTGGGGAGAGGGTC A (SEQ ID NO: 10) | RT-qPCR, Chip-qPCR | |
| HERVK-Env 1# | TCGAAGCATCAAAAG CCC (SEQ ID NO: 11) | GCAGACTAACAACAGACAA AAC (SEQ ID NO: 12) | RT-qPCR, Chip-qPCR | |
| HERVK-Env 2# | CCAGCCTCCCAAACTA TAACC (SEQ ID NO: 13) | AAGAGTGCAATGCAACTCC (SEQ ID NO: 14) | RT-qPCR | |
| HERVK-Gag 1# | AAATAAGACCCAACC GCCAGTAGC (SEQ ID NO: 15) | GAATTGCCATGCCTCAGTAT CTCC (SEQ ID NO: 16) | RT-qPCR, Chip-qPCR | |
| HERVK-Gag 2# | CCCAGAAGGAAACGG AAAC (SEQ ID NO: 17) | TGACCTGCTGAAAGAGGAC (SEQ ID NO: 18) | RT-qPCR | |
| HERVK-Pol 1# | GCCGATGAAAAAGCC CGTAAGG (SEQ ID NO: 19) | TTGACACTCAGGATTGGCGT TTTC (SEQ ID NO: 20) | RT-qPCR, Chip-qPCR | |
| HERVK-Pol 2# | TTTGCCACTGCCCATT CTCC (SEQ ID NO: 21) | AAGACCAATCTGCCATGCAC (SEQ ID NO: 22) | RT-qPCR | |
| p16^{INK4a} | TCATGATGATGGGCA GCGCC (SEQ ID NO: 23) | ACGGGTCGGGTGAGAGTGG C (SEQ ID NO: 24) | RT-qPCR | |
| p21^{Cip1} | TGTCCGTCAGAACCCA TGC (SEQ ID NO: 25) | AAAGTCGAAGTTCCATCGCT C (SEQ ID NO: 26) | RT-qPCR | |
| Lamin B | ACCAGAAGGAAGCAT GAAACGCG (SEQ ID NO: 27) | TCATCTCATGAAGGGCCTGA GCC (SEQ ID NO: 28) | RT-qPCR | |
| LAP2β | CCCCTCGGTCCTGACA AAAG (SEQ ID NO: 29) | CGCTCTTCGTCACTGGAGAA (SEQ ID NO: 30) | RT-qPCR | |
| IFNβ | ACGCCGCATTGACCAT CTAT (SEQ ID NO: 31) | GTCTCATTCCAGCCAGTGCT (SEQ ID NO: 32) | RT-qPCR | |
| IL1β | CGCCAGTGAAATGAT GGCTTAT | CTGGAAGGAGCACTTCATCT GT | RT-qPCR | |
| | (SEQ ID NO: 33) | (SEQ ID NO: 34) | | |
| IL6 | ACTGACCTCTTCAGAA CGAATTG (SEQ ID NO: 35) | CCATCTTTGGAAGGTTCAGG TTG (SEQ ID NO: 36) | | RT-qPCR |
| IL8 | ACTGAGAGTGATTGA GAGTGGAC (SEQ ID NO: 37) | AACCCTCTGCACCCATGGTT C (SEQ ID NO: 38) | | RT-qPCR |
| β-actin | AGAGCTACGAGCTGC CTGAC (SEQ ID NO: 39) | AGCACTGTGTTGGCGTACAG (SEQ ID NO: 40) | | RT-qPCR |
| GAPDH | TCGGAGTCAACGGAT TTGGT (SEQ ID NO: 41) | TTGCCATGGGTGGAATCATA (SEQ ID NO: 42) | | RT-qPCR |
| 5s rDNA | CTCGTCTGATCTCGGA AGCTAAG (SEQ ID NO: 43) | GCGGTCTCCCATCCAAGTAC (SEQ ID NO: 44) | | RT-qPCR |
| HERVK6 | GGAGAGAAGCTGTCC TGTGG (SEQ ID NO: 45) | TGACTGGACTTGCACGTAGG (SEQ ID NO: 46) | | ddPCR |
| MMTV-1 | GATGGTATGAAGCAGG ATGG (SEQ ID NO: 47) | CCTCTTTTCTCTATATCTATTA GCTGAGGTAATC (SEQ ID NO: 48) | | RT-qPCR |
| MMTV-2 | GATGGTATGAAGCAGG ATGG (SEQ ID NO: 49) | AAGGGTAAGTAACACAGGC AGATGTA (SEQ ID NO: 50) | | RT-qPCR |

| **shRNA** | | | | |
|---|---|---|---|---|
| Name | Sequence | | Use | |
| shHERVK-1 | CCTGAACATCCAGAATTAT (SEQ ID NO: 51) | | Knockdown of HERVK | |
| shHERVK-2 | ACATTATTAGATTCCATTGC (SEQ ID NO: 52) | | Knockdown of HERVK | |
| shSTING-2 | GCCCGGATTCGAACTTACAAT (SEQ ID NO: 53) | | Knockdown of STING | |

| **sgRNA** | | | | |
|---|---|---|---|---|
| Name | Sequence | | Use | |
| sgNTC-1 | ACGGAGGCTAAGCGTCGCAA (SEQ ID NO: 54) | | CRISRP/Cas9 control | |
| sgNTC-2 | ACGGGCGGCTATCGCTGACT (SEQ ID NO: 55) | | CRISRP/Cas9 control | |
| sgTBK1 | TCTCTCATTTGAACATCCAC (SEQ ID NO: 56) | | CRISRP/Cas9 knockdwon of TBK 1 | |
| sgNTC-act-1 | AAGATGAAAGGAAAGGCGTT (SEQ ID NO: 57) | | CRISRP/dCas9-activation control | |
| sgNTC-act-2 | CTGAAAAAGGAAGGAGTTGA (SEQ ID NO: 58) | | CRISRP/dCas9-activation control | |
| sgHERVK-act | GATAGGGAAAAACCGCCTTAGGG (SEQ ID NO: 59) | | CRISRP/dCas9-activation control | |
| sgHERVK-in act | ACCCCGTGCTCTCTGAAACAC (SEQ ID NO: 61) | | | CRISRP/Cas9 KRAB inhibiting HERVK |
| sgMMTV | TGTGAACCTTGCGGTTCCCAAGG (SEQ ID NO: 62) | | | CRISRP/Cas9 KRAB inhibiting HERVK |

| Probe | | | | |
|---|---|---|---|---|
| HERVK Alexa Fluor 488 | CGGCAGTGCAGCAAGTAGATTTATGTACTATACAAGCAGTCTCTCTG CTTCCAGGGGAGCCCCCACAAAAAACCCCCACAGGGGTATATGGAC CCCTGCCTAAGGGGACTGTAGGACTAATCTTGGGACGATCAAGTCT AAATCTAAAAGGAGTTCAAATTCATACTAGTGTGGTTGATTCAGACT ATAAAGGCGAAATTCAATTGGTTATTAGCTCTTCAATTCCTTGGAGT GCCAGTCCAAGAGACAGGATTGCTCAATTATTACTCCTGCCATACAT TAAGGGTGGAAATAGTGAAATAAAAAGAATAGGAGGGCTTGGAAG CACTGATCCAACAGGAAAGGCTGCATATTGGGCAAGTCAGGTCTCA GAGAACAGACCTGTGTGTAAGGCCATTATTCAAGGAAAACAGTTTG AAGGGTTGGTAGACACTGGAGCAGATGTCTCTATCATTGCTTTAAAT CAGTGGCCAAAAAATTGGCCTAAACAAAAGGCTGTTACAGGACTT GTCGGCATAGGCACAGCCTCAGAAGTGTATCAAAGTACGGAGATTT TACATTGCTTAGGGCCAGATAATCAAGAAAGTACTGTTCAGCCAATG ATTACTTCAATTCCTCTTAATCTGTGGGGTCGAGATTTATTACAACAA TGGGGTGCGGAAATCACCATGCCCGCTCCATCATATAGCCCCACGA GTCAAAAAATCATGACCAAGATGGGATATATACCAGGAAAGGGACT AGGGAAAAATGAAGATGGCATTAAAATTCCAGTTGAGGCTAAAATA AATCAAGAAAGAGAAGGAATAGGGAATCCTTGCCTAGGGGCGGCC ACTGTAGAGCCTCCTAAACCCATACCATTAACTTGGAAAACAGAAA AACCAGTGTGGGTAAATCAGTGGCCGCTACCAAAACAAAAACTGG AGGCTTTACATTTATTAGCAAATGAACAGTTAGAAAAGGGTCATATT GAGCCTTCGTTCTCACCTTGGAATTCTCCTGTGTTTGTAATTCAGAA GAAATCAGGCAAATGGCGTATGTTAACTGACTTAAG (SEQ ID NO: 60) | | | |

| **Probe pair (for single molecule DNA/RNA-FISH)** | | | | |
|---|---|---|---|---|
| **Target sequence** | | **Upper sequence** | **Lower sequence** | |
| HERVK-mRN A gag-1 | | CTAATCAAGCTATTTCAAACAAC AG (SEQ ID NO: 63) | ACAATTTTGCCCATGGTTTCCA GAA (SEQ ID NO: 64) | |
| HERVK-mRN A gag-2 | | ATTTAGATCTAGAAGATTGGAA AAG (SEQ ID NO: 65) | ATTGGTAAGGAACTAAAACAA GCAG (SEQ ID NO: 66) | |
| HERVK-mRN A gag-3 | | TCCACTTACAGTATGGAATGATT GG (SEQ ID NO: 67) | CCATTATTAAAGCAGCTTTAGA ACC (SEQ ID NO: 68) | |
| HERVK-mRN A gag-4 | | AAGATAGCGTTTCAGTTTCTGAT GC (SEQ ID NO: 69) | CCTGGAAGCTGTATAATAGATT GTA (SEQ ID NO: 70) | |
| HERVK-mRN A gag-5 | ATCCCAGAAGGAAACGGAAAC TTTA (SEQ ID NO: 71) | | ATTGCGAATATGTAGCAGAGC CGTT (SEQ ID NO: 72) | |
| HERVK-mRN A gag-6 | TGTTGACTATAATCAATTACAGG AG (SEQ ID NO: 73) | | TGATATATCCTGAAACATTAAA ATT (SEQ ID NO: 74) | |
| HERVK-mRN A gag-7 | AAGTCCTCTTTCAGCAGGTCAG GTG (SEQ ID NO: 75) | | CCGTAACATTACAACCTCAAG CGCA (SEQ ID NO: 76) | |
| HERVK-mRN A gag-8 | TCAGTATGGATATCTAGGAATGC CA (SEQ ID NO: 77) | | CAGCACACAGGACAGGGAGC CATA (SEQ ID NO: 78) | |
| HERVK-mRN Apol-1 | AGTGGAAAGTGTTACCTCAGGG AAT (SEQ ID NO: 79) | | CTTAATAGTCCAACTCTTTGTC AGA (SEQ ID NO: 80) | |
| HERVK-mRN A pol-2 | TTTCTGCAAGCAGAGGTTGCCA ATG (SEQ ID NO: 81) | | AGGACTGGCAATAGCATCTGA TAAG (SEQ ID NO: 82) | |
| HERVK-mRN A pol-3 | GGCATTCCTACTTATGCCATGTC AA (SEQ ID NO: 83) | | TTTGTTCTCTATCTTAAGAGGA GAC (SEQ ID NO: 84) | |
| HERVK-mRN A pol-4 | TACTGATCTTGTGGAGTGGTCAT TC (SEQ ID NO: 85) | | TTCCTCACAGTACAGTTAAGA CTTT (SEQ ID NO: 86) | |
| HERVK-mRN A pol-5 | TAGTTGTCCCTTTAACCAAGGA ACA (SEQ ID NO: 87) | | GTTAGACAAGCCTTTATCAATT CTG (SEQ ID NO: 88) | |
| HERVK-mRN A pol-6 | AATTGACTACTTGGATTCTACCT AA (SEQ ID NO: 89) | | ATTACCAGACGTGAACCTTTA GAAA (SEQ ID NO: 90) | |
| HERVK-mRN A pol-7 | TCAATCGGCTCAAAGAGCAGAG TTG (SEQ ID NO: 91) | | TTGCAGTCATTACAGTGTTACA AGA (SEQ ID NO: 92) | |
| HERVK-mRN A pol-8 | TCGGATTCTGCATATGTAGTACA GG (SEQ ID NO: 93) | | TACAAGGGATGTTGAGACAGC TCTA (SEQ ID NO: 94) | |
| HERVK-mRN A pol-9 | ATTGTACAACATTGCACCCAGT GTC (SEQ ID NO: 95) | | AGTCTTAGACCTGCCCACTCA AGAG (SEQ ID NO: 96) | |
| HERVK-mRN A pol-10 | TATGGCAAATGGATGTCACACAT GT (SEQ ID NO: 97) | | CCTTCATTTGGGAAGATTATCA TATG (SEQ ID NO: 98) | |
| HERVK-mRN Apol-11 | TTCACATTTCATGTGTGCAACTT GC (SEQ ID NO: 99) | | AAACAGGAGAAAGTACTTCCC ATGT (SEQ ID NO: 100) | |
| HERVK-mRN Apol-12 | CACATACAACAGGAATTCCTTAT AA (SEQ ID NO: 101) | | TCCCAAGGACAGGCCATAGTT GAAA (SEQ ID NO: 102) | |
| HERVK-mRN Apol-13 | GACAGTAAGGAGTGTACCACTC CTC (SEQ ID NO: 103) | | GATGCAACTTAATCTAGCACTC TAT (SEQ ID NO: 104) | |
| HERVK-mRN A env-1 | TTTCCATTGGGTATCATTATCCTC C (SEQ ID NO: 105) | | ATTTGCCTAGGGAGAGCACCA GGAT (SEQ ID NO: 106) | |
| HERVK-mRN A env-2 | TAGAAGTACCTACTGTCAGTCCT AA (SEQ ID NO: 107) | | AGTAGATTCACTTATCACATGG TAA (SEQ ID NO: 108) | |
| HERVK-mRN A env-3 | ATTCCTAAAGGATCAAAGAATA CAG (SEQ ID NO: 109) | | AGTTTTAGTTTGGGAAGAATG TGTG (SEQ ID NO: 110) | |
| HERVK-mRN A env-4 | AGATTGGGCACCTCGAGGTCAA TTC (SEQ ID NO: 111) | | ACCACAATTGCTCAGGACAAA CTCA (SEQ ID NO: 112) | |
| HERVK-mRN A env-5 | GAACATCCAGAATTGTGGAGGC TTA (SEQ ID NO: 113) | | TGTGGCCTCACACCACATTAG AATT (SEQ ID NO: 114) | |
| HERVK-mRN A env-6 | TTCCTTTACAAAGTTGCGTAAA GCC (SEQ ID NO: 115) | | CCTTATATGCTAGTTGTAGGAA ATA (SEQ ID NO: 116) | |
| HERVK-mRN A env-7 | CCGTATTCTGCTGGTGAGAGCA AGA (SEQ ID NO: 117) | | AAGGCATGTGGATCCCTGTGT CCAC (SEQ ID NO: 118) | |
| HERVK-mRN A env-8 | ACTTTAATTGCAGTGATTATGGG AT (SEQ ID NO: 119) | | AATTGCAGTCACAGCTACGGC TGCT (SEQ ID NO: 120) | |
| HERVK-mRN A env-9 | GATCTTAGACAAACTGTCATTTG GA (SEQ ID NO: 121) | | GGGAGACAGGCTCATGACCTT AGAA (SEQ ID NO: 122) | |
| HERVK-mRN A env-10 | GTCTGAGCATCACTGGGACATG GTT (SEQ ID NO: 123) | | GACGCCATCTACAGGGAAGAG AAGA (SEQ ID NO: 124) | |
| HERVK-mRN A env-11 | TTAGTCTGCAGGTGTACCCAAC AGC (SEQ ID NO: 125) | | CCGAAGAGACAGTGACATCG AGAAC (SEQ ID NO: 126) | |
| HERVK-mRN A ltr-1 | TCAGATTGTTACTGTGTCTGTAT AG (SEQ ID NO: 127) | | AAGAAGTAGACATAGGAGACT CCAT (SEQ ID NO: 128) | |
| HERVK-mRN A ltr-2 | GTGCTCTCTGAAACATGTGCTG TGT (SEQ ID NO: 129) | | AAACTCAGGGTTAAATGGATT AAGG (SEQ ID NO: 130) | |
| HERVK-mRN A ltr-3 | ACAGATGCTTGAAGGCAGCATG CTC (SEQ ID NO: 131) | | TTAAGAGTCATCACCACTCCCT AAT (SEQ ID NO: 132) | |
| HERVK-mRN A ltr-4 | CTGTGCTGAGGAGGATTAGTAT AAG (SEQ ID NO: 133) | | GGAAAGCATGCCTCTTGCAGT TGAG (SEQ ID NO: 134) | |
| HERVK-mRN A ltr-5 | CGATTGTACGTTCCACCTACTGA GA (SEQ ID NO: 135) | | AGGGAGAAACCACCTTAGGG CTGGA (SEQ ID NO: 136) | |
| HERVK-mRN A ltr-6 | TCCTTTACCTTGTCTATGATGCA AA (SEQ ID NO: 137) | | ACCTTTGTTCACGTGTTTGTCT GCT (SEQ ID NO: 138) | |
| HERVK-mRN A ltr-7 | TTAGTATAAGAGGAAAGCATGC CTC (SEQ ID NO: 139) | | TGCAGTTGAGAGAAGAGGAA GACAT (SEQ ID NO: 140) | |
| HERVK-mRN A ltr-8 | GTGGGCAGCAATACTGCTTCGT AAA (SEQ ID NO: 141) | | CATTGAGATGTTTATGTGTATG CAT (SEQ ID NO: 142) | |
| HERVK-mRN A ltr-9 | TACCTTGTCTATGATGCAAACAC CT (SEQ ID NO: 143) | | TGTTCACGTGTTTGTCTGCTG ACCC (SEQ ID NO: 144) | |
| HERVK-mRN A ltr-10 | TCTCGGAGAAACACCCACGAAT GAT (SEQ ID NO: 145) | | AATAAATACTAAGGGAACTCA GAGG (SEQ ID NO: 146) | |
| HERVK-mRN A ltr-11 | TCTCATTCCACCTTATGAGAAAC AC (SEQ ID NO: 147) | | CACAGGTGTGGAGGGGCAAC CCACC (SEQ ID NO: 148) | |

### Antibodies

Antibodies used in the Examples of the application are conventional commercial antibodies, antiserum or antibodies coming with the kit, e.g., antibodies shown in Table 3.

**Table 3**

| Antibody | Source | Use |
|---|---|---|
| Murine anti-HERVK-Env | Austral Biologicals | HERVK-Env primary antibody, treating HERVK |
| Murine IgG | Santa Cruz | Control |
| Rabbit anti-ERVW-1 | Abcam | Cynomolgus monkey ERVW primary antibody |
| Rabbit anti-phospho-NF-xB p65 (Ser536) | Cell Signaling Technolog | Detecting NF-xB phosphorylated primary antibody |
| Alexa Fluor^{®} 568 Donkey Anti-Rabbit IgG (H+L) | Invitrogen | Secondary antibody |
| Alexa Fluor^{®} 568 Donkey Anti-Mouse IgG (H+L) | Invitrogen | Secondary antibody |
| Alexa Fluor^{®} 488 Donkey Anti-Mouse IgG (H+L) | Invitrogen | Secondary antibody |
| HRP-conjugated Goat-anti-Mouse (H+L) | ZSGB-Bio | Secondary antibody |
| HRP-conjugated Goat-anti-Rabbit(H+L) | ZSGB-Bio | Secondary antibody |
| Rabbit anti-cGAS | Cell Signaling Technology | For cGAS of IP |
| Rabbit anit-Phospho-TBK1/NAK (Ser172) | Cell Signaling Technolog | Primary antibody for detecting TBK1 phosphorylation |
| Rabbit anti- Phospho-IRF-3 (Ser396) | Cell Signaling Technology | Primary antibody for detecting IRF3 phosphorylation |
| Rabbit anti-STING | R&D systems | Primary antibody for detecting STING |
| Murine anti-p 16 | BD Biosciences | Primary antibody for detecting p16 |
| Murine anti-LAP2β | BD Biosciences | Primary antibody for detecting LAP2 □ |
| Rabbit anti-GAPDH | Santa Cruz Biotechnology | Primary antibody for detecting GAPDH |
| Murine anti-Ki67 | Santa Cruz Biotechnology | Primary antibody for Ki67 immunofluorescence |
| Rabbit anti-H3K9me3 | Abcam | For H3K9me3 ChiP |
| Rabbit anti-H3K36me3 | Abcam | For 36me3 ChiP |
| Rabbit anti-MMTV | Novus | Primary antibody for detecting MMTV |
| Murine anti-MMTV1-16 | SinoBiological | Binding to MMTV |

### Example 1. ERV activation was associated with human cell senescence.

In the example, the relationship between HERVK activation and human cell senescence was studied with human mesenchymal progenitor cell (hMPC) of Hutchinson-Gilford progeria syndrome (HGPS) and Werner syndrome (WS) (LMNAG608G/+ hMPCs) or WS hMPCs (WRN-/- hMPCs) (as cell models of premature senescence), and wild type (WT) hMPCs with replicative senescence (RS).

### 1.1. RNA-seq analysis of senescent hMPCs

RNA-seq analysis was performed in WT RS hMPCs and prematurely senescent models (including HGPS and WS hMPC).

As shown by Figure 3, the expression of several transposable elements in senescent hMPC, such as LTR, DNA transposon and LINE (Long interspersed nuclear element, a kind of non-LTR). In these elements, HERVK in ERVK family both up-regulated in hMPCs in replicative senescence and premature senescence (Fig. 3B and Fig. 3C).

In particular, in Fig. 3B and Fig. 3C, the comparison by gene transcription levels of HGPS vs. WT and WS vs. WT in hMPCs at early passage (EP), the comparison by gene transcription levels of HGPS vs. WT and WS vs. WT at late passage (LP), and the comparison of gene transcription levels by hMPCs EP vs. LP, were shown.

Fig. 3B showed the comprehensive ranking according to above-mentioned comparison of gene expression levels, the results showed:
HERVK-int, MER61-int and MER61C represented significant differences in expression in all five items;
Charlie4 and MER101B showed significant differences in comparisons by EP HGPS vs. WT and WS vs. WT;
MamGypLTR (LTR region in MamGyp family) showed significant differences in comparisons by EP HGPS vs. WT and WS vs. WT, LP HGPS vs. WT and WT EP vs. LP ;
HERVK11 showed significant differences in comparisons of EP HGPS vs. WT and WS vs. WT, LP WS vs. WT, and WT EP vs. LP;
HERV16 showed significant differences in comparisons by EP HGPS vs. WT, , LP HGPS vs. WT and WS vs. WT, and WT EP vs. LP;
HUERS-P2 showed significant differences in comparisons by EP WS vs. WT, LP HGPS vs. WT and WS vs. WT, and WT EP vs. LP.

It can be seen that human ERV of HERVK (including HERVK11), MER61, MER61C, Charlie4, MER101B, MamGyp, HERV16, HUERS-P2, HERV30 loci may be used as biological marker for senescence.

In Fig. 3D and Fig. 3E, the comparisons of degrees, at different sites in hMPCs of early passage (EP), of whole virus HERVK activation by HGPS vs. WT and WS vs. WT, the comparisons of degrees, at different sites in hMPCs of late passage (LP), of whole virus HERVK activation by HGPS vs. WT and WS vs. WT, and the comparison of degree, at different sites in wild type hMPC, of whole virus HERVK activation by EP vs. LP, were shown.

Fig. 3E showed the comprehensive ranking according to of degree of whole virus HERVK activation at different sites, the results showed:
HML2_1q22 represented significant activation in comparisons of all five items;
HML2_3q12.3 showed significant activation in comparisons by EP HGPS vs. WT and WS vs. WT and LP WS vs. WT;
HML2_3q21.2 showed significant activation in comparisons by EP HGPS vs. WT and WT EP vs. LP;
HML2_4p16.1b showed significant activation in the comparison by EP WS vs. WT;
HML2_21q21.1 showed significant activation in the comparison by LP HGPS vs. WT;
HML2_12q11.1 showed significant activation in the comparison by LP HGPS vs. WT;

More particularly, as shown by Fig. 3F, HERVK corresponding to locus HML2_1q22 was highly activated in senescent hMPCs.

### 1.2. Levels of particular HERVK transcript in hMPC

RT-qPCR was performed with primers (shown in Table 2) for different regions of HERVK transcript (including Env, Pol and Gag), the result was shown in Fig. 4A.

As shown by Fig. 4, HERVK was highly expressed during cell senescence, showing the similar trend of expression with previously reported marker, e.g., positive marker for senescence, p21Cip1 (increased during senescence) and negative marker for senescence, Lamin associated protein LAP2 (TMPO) (decreased during senescence).

Furthermore, RNA-FISH and single molecule RNA-FISH analysis were conducted. The results were shown in Figs. 4B-C, cytoplasmic HERVK RNA signals increased in HGPS and WS hMPCs as well as RS hMPCs. Meanwhile, probes were labelled with different fluorescence for different regions of HERVK transcript (including Env, Pol and Gag), showing that different regions of HERVK were co-localized to the same viral particle (Fig. 4D).

### 1.3. The relationship between HERVK locus methylation and senescence in hMPC

A whole genome methylation analysis was conducted. The results showed that in line with activated HERVK transcription in hMPCs in senescence, methylation levels of global DNA in the whole HERVK family, HERVK coding sequence (HERVK-int) and flanking LTR5HS (human specific HERVK promoter) regions were all decreased in prematurely senescent hMPCs and RS hMPCs (as shown by Fig. 5).

Locus specific analysis also showed that CpG DNA methylation signal in provirus locus (including provirus) was decreased (as shown by Fig. 5C).

ChIP-qPCR analysis was conducted. The results showed that HERVK activation was related with decrease in inhibitory histone mark (H3K9me3) at HERVK-LTR5HS position in senescent hMPCs and increase in transcriptionally activated histone mark (H3K36me3) (as shown by Fig. 6).

It can be seen that epigenetic regulation of HERVK locus is associated with HERVK transcription, and involved in cell senescence.

### 1.4. The relationship between HERVK protein component, retrotransposition as well as RVLP formation and cell senescence in hMPC

Western blot and immunofluorescence assay were performed on each hMPCs. The results showed that HERVK-Env protein levels in both prematurely senescent and RS hMPCs were increased (as shown by Fig. 7).

TEM analysis showed that in line with increase in HERVK reverse transcribed RNA and protein levels, TEM results showed RVLP accumulation in cytoplasm of prematurely senescent and RS hMPCs (as shown by Fig. 8). In contrast, RVLP was rarely detected in WT hMPCs of early passage with young phenotype.

Immuno TEM analysis using anti-HERVK-Env antibody showed that HERVK formed RVLP with a diameter of 80-120 nm in senescent cells (as shown by Fig. 9).

In addition, HERVK transposition reporter vector was used to transform hMPCs.

By analyzing transformed cells by flow cytometer BD Influx, the ratio of cells positive for green fluorescence to cells positive for red fluorescence was the probability of transposition events occurred in HERVK. The results showed that the occurrence rate of retrotransposition events was increased in senescent hMPCs (as shown by Figs. 10A-B).

Single molecule DNA-FISH showed that HERVK DNA copy number in senescent hMPCs was increased (as shown by Figs. 10C-D), indicating increased retrotransposition events in senescent hMPCs.

### 1.5. The relationship between HERVK locus activation and senescence in other cells

Experiments similar to the above were conducted in human fibroblasts, the results showed that similar to senescent hMPCs, decrease in H3K9me3 and increase in H3K36me3 were observed in HERVK genomic sites, as well as the HERVK expression (in RNA and protein levels) was increased in senescent human fibroblasts (Figs. 11A-F). In addition, RVLP accumulation was increased in senescent fibroblasts (as shown by Fig. 11G).

It can be seen that compared with non-senescent cells, HERVK is activated, mobilized and packaged in senescent cells.

### Example 2. HERVK expression caused innate immune response and cell senescence.

In this example, the direct relationship between HERVK locus activation and cell senescence was studied.

### 2.1. The activation of HERVK transcription promoted cell senescence, while the inhibition of HERVK alleviated cell senescence

Using CRISPR-dCas9 transcriptional activation system to activate HERVK transcription, the effect of the activation of endogenous HERVK on cell senescence was determined. The transcriptional activation system contained activating protein complex (synergistic activation mediator, SAM) carrying a sgRNA targeting HERVK-LTR5HS promoter region (sgHERVK-act). CRISPR-dCas9 system containing non-targeting sgRNA was used as control.

HERVK-activated cell was analyzed by RT-qPCR and Western blot.

Consequently, it was proved at protein and RNA levels, that endogenous HERVK was activated by CRISPR-dCas9 transcriptional activation system (as shown by Fig. 12A and Fig. 12B).

Analysis of classical aging characteristics was performed on HERVK-activated cells, the results showed that HERVK activation caused hMPC senescence. In particular, relative percentage of cells positive for senescence associated β-galactosidase (SA-β-gal) was increased, the number of Ki67 positive cells was decreased and the clonal expansion ability was reduced (as shown by Figs. 12C-12E). Furthermore, RT-qPCR showed that the level of senescence mark p16^{INK4a} was increased, and the level of LAP2 (TMPO) was decreased (as shown by Fig. 12B).

To further assess whether endogenous HERVK activation was the driving force for cell senescence, HERVK in prematurely senescent hMPCs were koncked down by HERVK specific shRNA. The results showed that endogenous HERVK was decreased in prtein and RNA levels (as shown by Fig. 13A).

Above-mentioned analysis of classical aging characteristics was conducted on HERVK-konckdown hMPCs, and the results showed that inhibiting HERVK may alleviate hMPC senescence (as shown by Figs. 13B-E).

Meanwhile, Cas9KRAB inhibiting system was used to suppress HERVK. The results showed that endogenous HERVK was decreased in protein and RNA levels (as shown by Fig. 14A).

Above-mentioned analysis of classical aging characteristics was conducted on HERVK-inhibited hMPC, and the results showed that inhibiting HERVK may alleviate hMPC senescence (as shown by Fig. 14B-D).

### 2.2. Demethylation of HERVK locus

5-AZA (a DNA methyltransferase inhibitor (DNMTi)) was used to treat hMPCs of early passage. ChIP-qPCR and RT-qPCR analyses were performed on treated cells, cells not treated by 5-AZA were used as control.

The results showed that 5-AZA treatment may reduce 5mC occupation in HERVK-LTR5HS, meanwhile, HERVK RNA level and senescence marker p16^{INK4a} in hMPCs were increased (as shown by Figs. 15A-B).

The analysis of classical aging characteristics was conducted on treated cells. The results were consistent with HERVK activation experiments, and 5-AZA treatment caused premature senescence of cells. In particular, SA-β-gal positive cells were increased, the number of Ki67 positive cells was decreased and the clonal expansion ability was reduced (as shown by Figs. 15 C-E), while HERVK knockdown effectively alleviated 5-AZA-induced senescence (as shown by Figs. 15F-G). These data supported that activation of endogenous HERVK was the driving force for hMPC senescence.

### 2.3. Retrovirus inhibitor alleviated cell senescence

In the Example, the effect of non-specific retrovirus inhibitor on cell senescence was studied.

10 µM lamivudine (3TC), abacavir (0.2 µM) and Raltegravir were added to WS hMPC, respectively, incubated at 37°C, and fresh medium containing each drug was replaced every two days, solvents dissolving above-mentioned drug with DMSO added at a corresponding amount were used as control.

10 days after incubation, the reverse transcriptase inhibitor abacavir can effectively reduce transposition events and DNA copy number (as shown by Figs. 16B-C).

Above-mentioned cells were stained by Ki67 and SA-β-gal. The results were shown in Fig. 25. In WS hMPCs treated by lamivudine (3TC), abacavir or Raltegravir, Ki67 positive cells significantly were increased as compared with the control, while SA-β-gal positive cells significantly were decreased as compared with the control.

Above-mentioned results showed that the retrovirus inhibitors may reduce senescence phenotype of senescent cells.

### 2.4. HERVK DNA induced innate immune response

Pol protein encoded by HERVK had reverse transcription activity, can reverse transcribe HERVK RNA into DNA, thus generating other HERVK DNA outside the genome. These excessive cytoplasmic DNA may be recognized by key DNA sensor cGMP-AMP synthase (cGAS) and innate immune response was triggered (as shown by Fig. 17).

Single molecule DNA-FISH was performed on hMPCs of early passage and late passage, the results showed that in line with the increased HERVK RNA, HERVK DNA in senescent hMPCs cytoplasm significantly was increased (as shown by Fig. 18A).

Immunoprecipitation analysis confirmed that cGAS enrichment on cytoplasmic HERVK DNA in senescent hMPC was increased, and the enrichment was rare in hMPCs of early passage (as shown by Fig. 18B), indicating that cytoplasmic HERVK DNA in senescent hMPCs will activate cGAS-STING pathway.

To further study the activation of cGAS-STING DNA sensing pathway, the following key characteristics in different senescent hMPCs (including RS, HGPS and WS) and fibroblasts were detected: phosphorylation of second messenger 2'3'-cGAMP, RelA /NF-xB, the expression of TANK binding kinase 1(TBK1) and IFN regulating factor 3 (IRF3) as well as proinflammatory cytokines (e.g., IL1B andIL6), these factors were regarded as SASP factors.

In prematurely senescent hMPCs, 2'3'-cGAMP content was increased (as shown by Fig. 19A); the phosphorylation of TBK1, RelA and IRF3 were up-regulated (as shown by Fig. 17B) and the inflammatory cytokines IL6 was up-regulated (as shown by Fig. 19C). Similar phenomenon was observed in RS hMPCs and different senescent fibroblasts.

Knockdown of HERVK or STING (shSTING-2) both reduced phosphorylation levels of TBK1, RelA and IRF3, and alleviated cell senescence and SASP (as shown by Fig. 20).

By contrast, CRISPR-dCas9 system or 5-AZA treatment, activating endogenous HERVK, caused increased levels of phosphorylated TBK1, RelA and IRF3, and caused the up-regulated expression of SASP cytokine in hMPCs.

It can be seen that the activation of HERVK (at least partially) promoted cell senescence by activating innate immunization route.

### Example 3. Extracellular HERVK induced cell senescence

The aim of the Example is to prove whether HERVK RVLP generated by senescent cells can be released extracellularly and transmit senescent signal to non-senescent cells.

### 3.1. Detecting HERVK RVLP in conditioned medium (CM)

ddPCR was performed on CM harvested from wild type and prematurely senescent hMPCs, HERVK RNA therein was detected, and the amplicon was HERVK6 in Table 2.

The results showed that HERVK RNA level in CM from prematurely senescent hMPCs was 5~12 folds higher than wild type hMPCs (Fig. 21A).

In addition, ELISA conducted using anti-HERVK-Env antibody also showed that HERVK-Env protein level was increased in media from senescent hMPCs (Fig. 21B).

According to TEM image, RVLPs in hMPCs were more than wile type, with a diameter range of 80-120 nm, some HERVK viral particles were budded in the cell surface or adjacent to cell surface, while some particles were contained in coating vesicles and released to extracellular environment (Fig. 21C). Immuno-TEM showed the consistent results with TEM; the increase in the presence of HERVK in CM of senescent human fibroblasts was also observed (result not shown).

### 3.2. HERVK in CM induced cell senescence

CMs collected from HGPS, WS or RS hMPCs (collectively known as "old" CM) were used to treat young hMPCs (WT), and CM collected from young WT hMPCs were used as control.

TEM image showed that HERVK particles attached to young hMPCs (Fig. 22A). 48h after old CM treatment, qRT-PCR detection found that HERVK RNA in young hMPCs was increased (Fig. 22B), which meant HERVK in old CM might spread to target cell.

In addition, SA-β-gal and Ki67 staining and clonal amplification ability test showed that young hMPCs incubated with old CM induced accelerated cell senescence (Figs. 22C-E).

Furthermore, HERVK in old CM was removed by anti-HERVK-Env antibody. After young hMPCs were treated by HERVK-removed old CM, TEM analysis showed that less HERVK RVLP attached to cell surface or entered into the same (Fig. 23A). In addition, compared with HERVK-unremoved old CM, removing HERVK from old CM caused decreased HERVK RNA in young WT hMPCs and alleviated senescence phenotype (Figs. 23B-E).

In addition, old CM from senescent hMPCs activated cGAS-STING pathway and induced SASP gene expression in young hMPCs; the knockdown of STING or the knockdown of TKB1 may remove senescence phenotype induced by old CM (data not shown). This showed that similar to endogenous HERVK expression, old CM (at least partly) drove cell senescence by activating innate immunization route.

Finally, to further confirm that HERVK virus may infect target cell and cause cell senescence. Vector for expressing and packaging HERVK virus was constructed, and HERVK virus was labelled by green fluorescence (as shown by Fig. 24A). RNA, protein and viral particle of HERVK virus as well as GFP signal were detected in 293T cells (as shown by Figs. 24B-E).

hMPC cells were infected with puried HERVK virus with green fluorescence label, electron microscope showed that viral particle entered cell, green fluorescence can be detected in hMPC cells, indicating HERVK virus may enter hMPC cells (as shown by Figs. 25A-C).

Furthermore, cells infected by HERVK virus and having green fluorescence were sorted by flow cytometer (as shown by Figs. 25D-E). Compared with cell uninfected by HERVK virus, GFP RNA and gene copy number of cell infected by HERVK virus were increased (as shown by Figs. 25F-G),indicating HERVK not only expressed, also integrated into the genome. Western blot, ChIP-qPCR, RT-qPCR, SA-β-gal and Ki67 staining as well as clonal amplification ability test showed that infective HERVK was recognized by cGAS, innate immune response was activated to drive cell senescence (as shown by Figs. 25H-M).

In addition, anti-HERVK antibody was used to neutralize HERVK virus. Neutralized HERVK and untreated HERVK (e.g., α HERVK in Fig. 26A was murine anti-HERFVK-Env antibody in Table 2, IgG was murine immune globulin used as control) contacted with hMPCs, respectively, analysis of classical aging characteristics of Example 2 was conducted. The results showed that, compared with untreated HERVK virus, the treatment of HERVK virus by neutralized antibody alleviated activation of innate immune response and cell senescence caused by HERVK virus (Figs. 26A-F).

In conclusion, HERVK generated in senescent cell may be released in a paracrine manner and induce cell senescence of young cell.

### Example 4. ERV and individual aging

In the Example, the relationship between ERV and animal individual aging was studied.

### 4.1. MMTV and aging of mouse

Since homologous virus in mice corresponding to HERVK was MMTV, we detected MMTV in mice.

Liver biopsy samples from mice of 2 months old (5), 8 months old (5) and 24 months old (5) were obtained, RNA was extracted and MMTV levels in mice liver were detected using primers in Table 2, the amplicons were MMTV1 and MMTV2. The results were shown in Fig. 27A. Expression quantities of MMTV1 in mice with 8 months old and 24 months old were significantly higher than that of mice with 2 months old; while expression quantity of MMTV2 in mice with 24 months old was significantly higher than that of mice with 2 months old and 8 months old.

In addition, Western blot and immunohistochemical staining analysis showed that compared with young mice, MMTV expression levels in isolated lung, liver and skin tissues of old mice were increased with age (Figs. 27B-C). Innate immune response was also overactivated in naturally aged mice (Fig. 27B).

Above results showed that endogenous retrovirus MMTV was activated in senescent mice.

### 4.2. ERVW and aging of cynomolgus monkey

Since ERVK family was not present in the genome of cynomolgus monkey (Fig. 2), ERVW family which is the youngest in the evolution of cynomolgus monkey was selected to for detection.

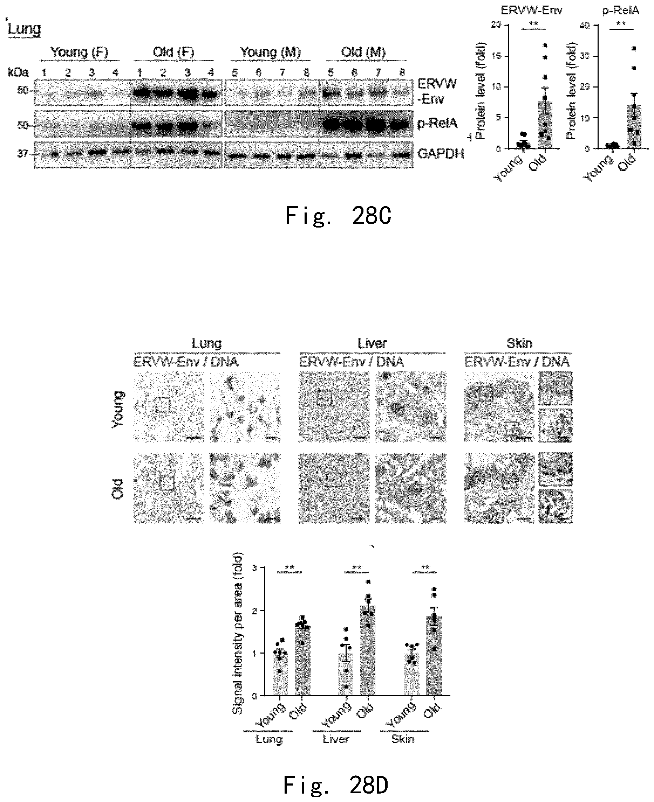

Similar to HERVK family activated in human senescent cell, Western blot and immunohistochemical staining analysis found that relative to WT cynomolgus monkey with the same age, ERVW-Env signals were increased in lung, liver and skin tissues of HGPS cynomolgus monkey (Fig. 28). The results also showed that the innate immune response in tissues of HGPS cynomolgus monkey was enhanced, e.g., increased RelA phosphorylation (Fig. 28A) and up-regulated SASP gene (data not shown).

In addition, when comparing isolated lung, liver and skin tissues of yong and physiological senescent cynomolgus monkey, ERVW expression levels were increased with age (Fig. 28A-C). SASP was overactivated in naturally aged monkey, similar to senescent monkey.

### 4.3. HERVK and human aging

HERVK levels in primary hMPCs from young and old donors were analyzed by qPCR. The results showed that relative to young individuals, the expression of HERVK genes (env, pol and gag) in old individuals was significantly up-regulated (Fig. 29A). In skin tissues obtained from young and old donors, immunohistochemical staining analysis showed that HERVK-Env expression was significantly increased with age (Fig. 29B). More importantly, ELISA of human serum showed that HERVK-Env level in sera from old individuals was significantly increased relative to young individuals (Fig. 29C).

Furthermore, primary hMPCs from young individuals were cultured using meidum containing young or old individual serum. Detections corresponding to Example 3.2 were conducted, the results showed that, serum in old individual (OS) promoted the senescence of primary hMPCs and caused senescence-associated inflammatory response, while the immune elimination of HERVK in OS removed senescence-promoting effect of OS on young primary hMPCs, and alleviated senescence-associated inflammatory phenotype (Fig. 30).

### Example 5. Inhibiting ERV delayed aging of tissue and organ as well as individual

The Example aimed to explore whether inhibiting ERV will achieve the goal of delaying aging of tissue and organ as well as individual.

### 5.1. Knockdown of MMTV delayed joint aging of old mice

Lentivirus which suppressed MMTV by Cas9KRAB inhibition system was injected to articular cavity of old mice (21 months old), and repeatedly injected 4 weeks after the first injection (as shown by Fig. 31A) (n=12).

First, Western blot in MEF cells showed that after inhibition by CRISPR-dCas system, MMTV protein level was decreased, meanwhile, the level of a protein associated with innate immunization pathway, p-RelA was decreased (as shown by Fig. 31B); limb tension of mice was tested 8 weeks after injection, the results showed that as compared to mice with MMTV uninhibited, after inhibiting MMTV, tension of mice significantly improved (as shown by Fig. 31C); micro nuclear magnetism showed that after inhibiting MMTV, bone density of joint was increased (as shown by Fig. 31D); the whole knee-joint (including distal femur, upper tibia, patella, articular cartilage, articular ligament and joint fluid) was taken, RNA was extracted, and RT-qPCR showed that after inhibiting MMTV, the level of inflammatory factor IL1B was decreased, and the expression of senescence associated protein p21 was decreased.

Above results showed that the knockdown of MMTV delayed joint aging of old mice.

### 5.2. Retrovirus inhibitor Abacavir delayed joint aging of old mice

### Retrovirus inhibitor Abacavir was injected to articular cavity of old mice (23 months old), concentration was 10 mg/ml, once a week, 10µl every time (as shown by Fig. 32A). (n=10)

8 weeks after injecting retrovirus inhibitor Abacavir, limb tension of mice was tested, the results showed that compared with mice injected by vehicle, tension of mice injected by retrovirus inhibitor Abacavir significantly was enhanced (as shown by Fig. 32B); although individual differences are too big, P value was not significantl, it can be seen that forced treadmill testing shows retrovirus inhibitor Abacavir may improve running distance and time of mice, and reduce the trend of shocked time (as shown by Fig. 32C); miniature nuclear magnetic showed that 15 weeks after injecting retrovirus inhibitor Abacavir, bone density of joint was increased (as shown by Fig. 32D); after sampling of joint, RNA was extracted, RT-qPCR showed that after injecting retrovirus inhibitor Abacavir, the levels of inflammatory factors IL1B and IL6 were decreased, and the expression of senescence-associated protein p21 was decreased.

Above result showed that administering retrovirus inhibitor Abacavir delayed joint aging of old mice.

### 5.3. Administering Abacavir delayed individual aging of old mice and improved survival rate

Retrovirus inhibitor Abacavir was dissolved in drinking water for mice (containing 0.3% DMSO) at a concentration of 2 mg/ml (n=23), and mice of 18 months old was administered daily (as shown by Fig. 33A).

12 weeks after administering retrovirus inhibitor Abacavir, limb tension of mice was tested, and the results showed that after injecting retrovirus inhibitor Abacavir, tension of mice was significantly enhanced (as shown by Fig. 33B); 24 weeks after administering retrovirus inhibitor Abacavir, the weight, skin, hair, hollow back, cataract and mobility test of mice were tested, the results showed that reverse transcriptase inhibitor Abacavir may enhance mobility ability of mice, and improve the conditions of hair and hollow back (as shown by Fig. 33C). 30 weeks after administering retrovirus inhibitor Abacavir, mice were subjected to Y Maze test, the results showed that the total times that the mice administered with the reverse transcriptase inhibitor Abacavir entered the arms and the rotations that the mice entered in turn the three arms of the Y maze were increased, indicating that spatial short-term memory ability of the mice was enhanced (as shown by Fig. 33D).

24 weeks after administering Abacavir, 2 mice died in control group administered with vehicle, 0 died in Abacavir group, 30 weeks after administering Abacavir, 4 mice died in control group administered with vehicle, 1 died in Abacavir group, indicating that the reverse transcriptase inhibitor Abacavir may improve survival rate of old mice (as shown by Fig. 33E). The inventor believed that as continuing to administer Abacavir, significant difference will be further shown in survival rate.

Above results showed that administering the retrovirus inhibitor Abacavir delayed the aging of old individuals and improved survival rate.

### 5.4. Administering retrovirus integrase inhibitor Ratelgravir delayed individual aging of old mice

The retrovirus integrase inhibitor Ratelgravir was dissolved in 30%PEG300 + 2% Tween80 +2% DMSO at a concentration of 50 mg/ml, mice at 18 months old was injected intraperitoneally,(n=22), 200µl/injection, twice a week (as shown by Fig. 34A).

16 weeks after administering the integrase inhibitor Ratelgravir, limb tension of mice was tested, and the results showed that compared with mice injected by vehicle, tension of mice injected with the integrase inhibitor Ratelgravir significantly was increased (as shown by

Fig. 34B); meanwhile, the weight, skin, hair, hollow back, cataract and mobility test of mice were tested, the results suggested that the integrase inhibitor Ratelgravir may improve the conditions of hair and hollow back (as shown by Fig. 34B).

Above result showed that the integrase inhibitor Ratelgravir delayed individual aging of old mice.

### Example 6. Synthesis and characterization of antibodies recognizing ERV

The Example aimed to synthesize specific neutralizing antibody for endogenous virus MMTV in mice, and detect antibody titer and neutralization efficiency for in vivo therapy.

MMTV antibodies were provided by Sino Biological Inc. In brief, according to structural analysis, MTV-SU subunit (SEQ ID NO: 151) coupled with N-signal peptide and N-his-tag were expressed in HEK293 system. Recombinantly expressed MMTV-SU subunit was used as immunogen to immunize mice, and antibodies binding to the immunogen were screened by ELISA.

Supernatant of currently obtained 16 major clones were characterized. Western blot showed that all the 16 polyclonal antibodies can detect MMTV-Env protein, and MMTV-Env protein level in kidney of old mice was increased (as shown by Fig. 35A); immunohistochemical staining showed that antibodies of Nos. 1, 2, 5, 6, 11, 12, 14 and 15 may detect correct cell localization of MMTV (located in cytoplasm), wherein antibodies of Nos. 2 and 14 exhibited the best staining effect.

Monoclonal antibody was isolated, and MMTV virus was treated by the isolated antibody. Mice cells were infected by treated MMTV virus, and compared with the control, the titer of treated MMTV virus was decreased, and the antibody for treating MMTV virus was characterized as neutralizing antibody.

Furthermore, above-mentioned characterized neutralizing antibody was administered to old mice, the weight, skin, hair, hollow back, cataract and mobility test of mice were tested. In addition, mice were also subjected to Y Maze test, survival time of mice was recorded. At a later stage of the experiment, samples were collected from various tissues and organs (including brain, lung, liver, skin, blood, joint), aging phenotypes of various organs were confirmed by sequencing techniques, including transcriptome sequencing, immunohistochemical staining, Western immunoblotting and RT-qPCR.

### Sequence information

SEQ ID NO: 1, exemplary HERVK-Env amino acid sequence 
MVTPVTWMDNPIEVYVNDSVWVPGPTDDRCPAKPEEEGMMINISIGYHYPPICLGRAPGCLM
PAVQNWLVEVPTVSPNSRFTYHMVSGMSLRPRVNCLQDFSYQRSLKFRPKGKTCPKEIPKGS
KNTEVLVWEECVANSVVILQNNEFGTIIDWAPRGQFYHNCSGQTQSCPSAQVSPAVDSDLTE
SLDKHKHKKLQSFYLWEWEEKGISTPRPKIISPVSGPEHPELWRLTVASHHIRIWSGNQTLE
TRYRKPFYTIDLNSILTVPLQSCVKPPYMLVVGNIVIKPASQTITCENCRLFTCIDSTFNWQ
HRILLVRAREGMWIPVSTDRPWEASPSIHILTEILKGVLNRSKRFIFTLIAVIMGLIAVTAT
AAVAGVALHSSVQSVNFVNYWQKNSTRLWNSQSSIDQKLASQINDLRQTVIWMGDRLMTLEH
HFQLQCDWNTSDFCITPQIYNESEHHWDMVRRHLQGREDNLTLDISKLKEQIFEASKAHLNL
VPGTEAIAGVADGLANLNPVTWIKTIRSTMIINLILIVVCLFCLLLVCRCTQQLRRDSDIEN
GP
SEQ ID NO: 2, exemplary HERVK-Pol amino acid sequence 
MIPKDWPLIIIDLKDCFFTIPLAEQDCEKFAFTIPAINNKEPATRFQWKVLPQGMLNSPTLC
QTFVGRALQPVRDKFSDCYIIHYFDDILCAAETKDKLIDCYTFLQAEVANAGLAIASDKIQT
STPFHYLGMQIENRKIKPQKIEIRKDTLKTLNDFQKLLGDINWIRPTLGIPTYAMSNLFSIL
RGDSDLNSKRMLTPEATKEIKLVEEKIQSAQINRIDPLAPLQLLIFATAHSPTGIIIQNTDL
VEWSFLPHSTVKTFTLYLDQIATLIGPTRLRIIKLCGNDPDKIVVPLTKEQVRQAFINSGAW
QIGLANFVGIIDNHYPKTKIFQFLKLTTWILPKITRREPLENALTVFTDGSSNGKVAYTGPK
ERVIKTPYQSAQRAELVAVITVLQDFDQPINIISDSAYVVQATRDVETALIKYSMDDQLNQL
FNLLQQTVRKRNFPFYITHIRAHTNLPGPLTKANEQADLLVSSAFIKAQELHALTHVNAAGL
KNKFDVTWKQAKDIVQHCTQCQVLDLPTQEAGVNPEVCVLMHYGKWMSHMYLHLGRLSYVHV
TVDTYSHFMCATCQTGESTSHVKKHLLSCFAVMGVPEKIKTDNGPGYCSKAFQKFLSQWKIS
HTTGIPYNSQGQAIVERTNRTLKTQLVKQKEGGDSKECTTPQMQLNLALYTLNFLNIYRNQT
TTSAEHLTGKKNSPHEGKLI
SEQ ID NO: 3, exemplary HERVK-Pro amino acid sequence 
KWATIVGKRAKGPASGPTTNWGIPNSAICSSGFSGTTTPTVPSVSGNKPVTTIQQLSPATSG
SAAVDLCTIQAVSLLPGEPPQKIPTGVYGPLPEGTVGLILGRSSLNLKGVQIHTSVVDSDYK
GEIQLVISSSIPWSASPGDRIAQLLLLPYIKGGNSEIKRIGGLGSTDPTGKAAYWASQVSEN
RPVCKAIIQGKQFEGLVDTGADVSIIALNQWPKNWPKQKAVTGLVGIGTASEVYQSTEILHC
LGPDNQESTVQPMITSIPLNLWGRDLLQQWGAEITMPAPLYSPTSQKIMTKMGYIPGKGLGK
NEDGIKIPVEAKINQKREGIGYPF
SEQ ID NO: 4, exemplary HERVK-Gag amino acid sequence MGQTKTKSKYASYLSFIKILLKRGGVRVSTKNLIKLFQTTEQFCPWFPEQGNLDLEDWKRIG 
KELKQAGRKGNIIPLTVWNDWPIIKAALEPFQTEDSVSVSDAPGSCIIDCNEKTRKKSQKET
ETLHCEYVAEPLMAQSTQNVDYNQLQEVIYPETLKLEGKGPELVGPLESKPRGPSPLSAGQV
TVTLQPQAQVRENKTQLPVAYQYWPPAELQYRPPPESQYGYLGMPPAPQDREPYPQPPTRRQ
CYGTT
SEQ ID NO: 5, exemplary HERVK-Env coding nucleotide sequence 
atggtaacaccagtcacatggatggataatcctatagaagtatatgttaatgatagtgtatg
ggtacctggccccacagatgatcgctgccctgccaaacctgaggaagaggatgatgataa
atatttccattgggtatcattatcctcctatttgcctagggagagcaccaggatgtttaatg
cctgcagtccaaaattggttggtagaagtacctactgtcagtcctaacagtagattcactta
tcacatggtaagcgggatgtcactcaggccacgggtaaattgtttacaagacttttcttatc
aaagatcattaaaatttagacctaaagggaaaacttgccccaaggaaattcctaaaggatca
aagaatacagaagttttagtttgggaagaatgtgtggccaatagtgtggtgatattacaaaa
caatgaattcggaactattatagattgggcacctcgaggtcaattctaccacaattgctcag
gacaaactcagtcgtgtccaagtgcacaagtgagtccagctgtcgatagcgacttaacagaa
agtctagacaaacataagcataaaaaattacagtctttctacctttgggaatgggaagaaaa
aggaatctctaccccaagaccaaaaataataagtcctgtttctggtcctgaacatccagaat
tgtggaggcttactgtggcctcacaccacattagaatttggtctggaaatcaaactttagaa
acaagatatcgtaagccattttatactatcgacctaaattccattctaacggttcctttaca
aagttgcgtaaagcccccttatatgctagttgtaggaaatatagttattaaaccagcctccc
aaactataacctgtgaaaattgtagattgtttacttgcattgattcaacttttaattggcag
caccgtattctgctggtgagagcaagagaaggcatgtggatccctgtgtccacggaccgacc
gtgggaggcctcgccatccatccatattttgactgaaatattaaaaggcgttttaaatagat
ccaaaagattcatttttactttaattgcagtgattatgggattaattgcagtcacagctacg
gctgctgtggcaggagttgcattgcactcttctgttcagtcagtaaactttgttaattattg
gcaaaagaattctacaagattgtggaattcacaatctagtattgatcaaaaattggcaagtc
aaattaatgatcttagacaaactgtcatttggatgggagacaggctcatgaccttagaacat
catttccagttacagtgtgactggaatacgtcagatttttgtattacaccccaaatttataa
tgagtctgagcatcactgggacatggttagacgccatctacagggaagagaagataatctca
ctttagacatttccaaattaaaagaacaaattttcgaagcatcaaaagcccatttaaatttg
gtgccaggaactgaggcaattgcaggagttgctgatggcctcgcaaatcttaaccctgtcac
ttggattaagaccatcagaagtactatgattataaatctcatattaatcgttgtgtgcctgt
tttgtctgttgttagtctgcaggtgtacccaacagctccgaagagacagtgacatcgagaac
gggccatga
SEQ ID NO: 6, exemplary HERVK- Pol coding nucleotide sequence 
atgatcccaaaagattggcctttaattataattgatctaaaggactgcttttttaccatccc
tctggcagagcaggattgtgaaaaatttgcctttactataccagccataaataataaagaac
cagccaccaggtttcagtggaaagtgttacctcagggaatgcttaatagtccaactctttgt
cagacttttgtaggtcgagctcttcaaccagttagagacaagttttcagactgttatattat
tcattattttgatgatattttatgtgctgcagaaacgaaagataaattaattgactgttata
catttctgcaagcagaggttgccaatgcaggactggcaatagcatctgataagatccaaacc
tctactccttttcattatttagggatgcagatagaaaatagaaaaattaagccacaaaaaat
agaaataagaaaagacacattaaaaacactaaatgattttcaaaaattgctgggagatatta
attggattcggccaactctaggcattcctacttatgccatgtcaaatttgttctctatctta
agaggagactcagacttaaatagtaaaagaatgttaaccccagaggcaacaaaagaaattaa
attagtggaagaaaaaattcagtcagcgcaaataaatagaatagatcccttagccccactcc
 
aacttttgatttttgccactgcccattctccaacaggcatcattattcaaaatactgatctt
gtggagtggtcattccttcctcacagtacagttaagacttttacattgtacttggatcaaat
agctactttaattggtccgacaagattacgaataataaaattatgtggaaatgacccagaca
aaatagttgtccctttaaccaaggaacaagttagacaagcctttatcaattctggtgcatgg
cagattggtcttgctaattttgtgggaattattgataatcattacccaaaaacaaaaatctt
ccagttcttaaaattgactacttggattctacctaaaattaccagacgtgaacctttagaaa
atgctctaacagtatttactgatggttccagcaatggaaaagtggcttacacagggccaaaa
gaacgagtaatcaaaactccatatcaatcggctcaaagagcagagttggttgcagtcattac
agtgttacaagattttgatcaacctatcaatattatatcggattctgcatatgtagtacagg
ctacaagggatgttgagacagctctaattaaatatagcatggacgatcagttaaaccagcta
ttcaatttattacaacaaactgtaagaaaaagaaacttcccattttatattactcatattcg
agcacacactaatttaccagggcctttgactaaagcaaatgaacaagctgacttactggtat
catctgcattcataaaagcacaagaacttcatgctttgactcatgtaaatgcagcaggatta
aaaaacaaatttgatgtcacatggaaacaggcaaaagatattgtacaacattgcacccagtg
tcaagtcttagacctgcccactcaagaggcaggagttaacccagaggtctgtgtcctaatgc
attatggcaaatggatgtcacacatgtaccttcatttgggaagattatcatatgttcatgta
acagttgatacttattcacatttcatgtgtgcaacttgccaaacaggagaaagtacttccca
tgttaaaaaacatttattgtcttgttttgctgtaatgggagttccagaaaaaatcaaaactg
acaatggaccaggatattgtagtaaagctttccaaaaattcttaagtcagtggaaaatttca
catacaacaggaattccttataattcccaaggacaggccatagttgaaagaactaatagaac
actcaaaactcaattagttaaacaaaaagaagggggagacagtaaggagtgtaccactcctc
agatgcaacttaatctagcactctatactttaaattttttaaacatttatagaaatcagact
actacttctgcagaacatcttactggtaaaaagaacagcccacatgaaggaaaactaattta
g
SEQ ID NO: 7, exemplary HERVK- Pro coding nucleotide sequence 
aatgggcaaccattgtcgggaaacgagcaaaggggccagcctcaggccccacaacaaactgg
ggcattcccaattcagccatttgttcctcagggttttcagggacaacaacccccactgtccc
aagtgtttcagggaataagccagttaccacaatacaacaattgtcccccgccacaagcggca
gtgcagcagtagatttatgtactatacaagcagtctctctgcttccaggggagcccccacaa
aaaatccccacaggggtatatggccccctgcctgaggggactgtaggactaatcttgggaag
atcaagtctaaatctaaaaggagttcaaattcatactagtgtggttgattcagactataaag
gcgaaattcaattggttattagctcttcaattccttggagtgccagtccaggagacaggatt
gctcaattattactcctgccatatattaagggtggaaatagtgaaataaaaagaataggagg
gcttggaagcactgatccaacaggaaaggctgcatattgggcaagtcaggtctcagagaaca
gacctgtgtgtaaggccattattcaaggaaaacagtttgaagggttggtagacactggagca
gatgtctctatcattgctttaaatcagtggccaaaaaattggcctaaacaaaaggctgttac
aggacttgtcggcataggcacagcctcagaagtgtatcaaagtacggagattttacattgct
tagggccagataatcaagaaagtactgttcagccaatgattacttcaattcctcttaatctg
tggggtcgagatttattacaacaatggggtgcggaaatcaccatgcccgctccattatatag
ccccacgagtcaaaaaatcatgaccaagatgggatatataccaggaaagggactagggaaaa
atgaagatggcattaaaattccagttgaggctaaaataaatcaaaaaagagaaggaataggg
tatcctttttagg
SEQ ID NO: 8, exemplary HERVK- Gag coding nucleotide sequence 
atggggcaaactaaaactaaaagtaaatatgcctcttatcttagcttcattaaaattctttt
aaaaagagggggagttagagtatccaccaaaaatctaatcaagctatttcaaacaacagaac
 
aattttgcccatggtttccagaacaaggaaatttagatctagaagattggaaaagaattggt
aaggaactaaaacaagcaggtaggaagggtaatatcattccacttacagtatggaatgattg
gcccattattaaagcagctttagaaccatttcaaacagaagatagcgtttcagtttctgatg
cccctggaagctgtataatagattgtaatgaaaagacaaggaaaaaatcccagaaggaaacg
gaaactttacattgcgaatatgtagcagagccgttaatggctcagtcaacgcaaaatgttga
ctataatcaattacaggaggtgatatatcctgaaacattaaaattagaaggaaaaggtccag
aattagtggggccattagagtctaaaccacgagggccaagtcctctttcagcaggtcaggtg
accgtaacattacaacctcaagcgcaggttagagaaaataagacccaactgccagtagctta
tcaatactggccaccggccgaacttcagtatcggccacccccagaaagtcagtatggatatc
taggaatgccaccagcaccacaggacagggagccataccctcagccgcccactaggagacaa
tgctatggcaccacctag
SEQ ID NO: 149, exemplary HERVK- Rev coding nucleotide sequence 
atgaacccatcagagatgcaaagaaaagcacctccgcggagacggagacaccgcaatcgagc
accgttgactcacaagatgaacaaaatggtgacgtcagaagaacagatgaagttgccatcca
ccaagaaggcagagccgccgacttgggcacaactaaagaagctgacgcagttagctacaaaa
tatctagagaacacaaaggtgacacaaaccccagagagtatgctgcttgcagccttgatgat
tgtatcaatggtgtctgcaggtgtacccaacagctccgaagagacagcgaccatcgagaacg
ggccatga
SEQ ID NO: 150, exemplary HERVK full length coding nucleotide sequence 
tgtggggaaaagcaagagagatcagattgttactgtgtctgtgtagaaagaagtagacatag
gagactccattttgttatgtactaagaaaaattcttctgccttgagattctgttaatctatg
accttacccccaaccccgtgctctctgaaacatgtgctgtgtcaactcagagttaaatggat
taagggcggtgcaggatgtgctttgttaaacagatgcttgaaggcagcatgctccttaagag
tcatcaccactccctaatctcaagtacccagggacacaaaaactgcggaaggccgcagggac
ctctgcctaggaaagccaggtattgtccaaggtttctccccatgtgatagtctgaaatatgg
cctcgtgggaagggaaagacctgaccgtcccccagcccgacacccgtaaagggtctgtgctg
aggaggattagtaaaagaggaaggaatgcctcttgcagttgagacaagaggaaggcatctgt
ctcctgcctgtccctgggcaatggaatgtctcggtataaaacccgattgtatgctccatcta
ctgagatagggaaaaaccgccttagggctggaggtgggacctgcgggcagcaatactgcttt
gtaaagcactgagatgtttatgtgtatgcatatctaaaagcacagcacttaatcctttacat
tgtctatgatgcaaagacctttgttcacgtgtttgtctgctgaccctctccccacaattgtc
ttgtgaccctgacacatccccctctttgagaaacacccacagatgatcaataaatactaagg
gaactcagaggctggcgggatcctccatatgctgaacgctggttccccgggtccccttattt
ctttctctatactttgtctctgtgtctttttcttttccaaatctctcgtcccaccttacgag
aaacacccacaggtgtgtaggggcaacccacccctacatctggtgcccaacgtggaggcttt
tctctagggtgaaggtacgctcgagcgtggtcattgaggacaagtcgacgagagatcccgag
tacgtctacagtcagccttacggtaagcttgtgcgctcggaagaagctagggtgataatggg
gcaaactaaaagtaaaattaaaagtaaatatgcctcttatctcagctttattaaaattcttt
taaaaagagggggagttaaagtatctacaaaaaatctaatcaagctatttcaaataatagaa
caattttgcccatggtttccagaacaaggaactttagatctaaaagattggaaaagaattgg
taaggaactaaaacaagcaggtaggaagggtaatatcattccacttacagtatggaatgatt
gggccattattaaagcagctttagaaccatttcaaacagaagaagatagcgtttcagtttct
gatgcccctggaagctgtataatagattgtaatgaaaacacaaggaaaaaatcccagaaaga
aacggaaggtttacattgcgaatatgtagcagagccggtaatggctcagtcaacgcaaaatg
ttgactataatcaattacaggaggtgatatatcctgaaacgttaaaattagaaggaaaaggt
 
ccagaattagtggggccatcagagtctaaaccacgaggcacaagtcctcttccagcaggtca
ggtgcccgtaacattacaacctcaaaagcaggttaaagaaaataagacccaaccgccagtag
cctatcaatactggccgccggctgaacttcagtatcggccacccccagaaagtcagtatgga
tatccaggaatgcccccagcaccacagggcagggcgccataccctcagccgcccactaggag
acttaatcctacggcaccacctagtagacagggtagtgaattacatgaaattattgataaat
caagaaaggaaggagatactgaggcatggcaattcccagtaacgttagaaccgatgccacct
ggagaaggagcccaagagggagagcctcccacagttgaggccagatacaagtctttttcgat
aaaaatgctaaaagatatgaaagagggagtaaaacagtatggacccaactccccttatatga
ggacattattagattccattgctcatggacatagactcattccttatgattgggagattctg
gcaaaatcgtctctctcaccctctcaatttttacaatttaagacttggtggattgatggggt
acaagaacaggtccgaagaaatagggctgccaatcctccagttaacatagatgcagatcaac
tattaggaataggtcaaaattggagtactattagtcaacaagcattaatgcaaaatgaggcc
attgagcaagttagagctatctgccttagagcctgggaaaaaatccaagacccaggaagtac
ctgcccctcatttaatacagtaagacaaggttcaaaagagccctatcctgattttgtggcaa
ggctccaagatgttgctcaaaagtcaattgccgatgaaaaagcccgtaaggtcatagtggag
ttgatggcatatgaaaacgccaatcctgagtgtcaatcagccattaagccattaaaaggaaa
ggttcctgcaggatcagatgtaatctcagaatatgtaaaagcctgtgatggaatcggaggag
ctatgcataaagctatgcttatggctcaagcaataacaggagttgttttaggaggacaagtt
agaacatttggaggaaaatgttataattgtggtcaaattggtcacttaaaaaagaattgccc
agtcttaaacaaacagaatataactattcaagcaactacaacaggtagagagccacctgact
tatgtccaagatgtaaaaaaggaaaacattgggctagtcaatgtcgttctaaatttgataaa
aatgggcaaccattgtcgggaaacgagcaaaggggccagcctcaggccccacaacaaactgg
ggcattcccaattcagccatttgttcctcagggttttcagggacaacaacccccactgtccc
aagtgtttcagggaataagccagttaccacaatacaacaattgtcccccgccacaagcggca
gtgcagcagtagatttatgtactatacaagcagtctctctgcttccaggggagcccccacaa
aaaatccccacaggggtatatggccccctgcctgaggggactgtaggactaatcttgggaag
atcaagtctaaatctaaaaggagttcaaattcatactagtgtggttgattcagactataaag
gcgaaattcaattggttattagctcttcaattccttggagtgccagtccaggagacaggatt
gctcaattattactcctgccatatattaagggtggaaatagtgaaataaaaagaataggagg
gcttggaagcactgatccaacaggaaaggctgcatattgggcaagtcaggtctcagagaaca
gacctgtgtgtaaggccattattcaaggaaaacagtttgaagggttggtagacactggagca
gatgtctctatcattgctttaaatcagtggccaaaaaattggcctaaacaaaaggctgttac
aggacttgtcggcataggcacagcctcagaagtgtatcaaagtacggagattttacattgct
tagggccagataatcaagaaagtactgttcagccaatgattacttcaattcctcttaatctg
tggggtcgagatttattacaacaatggggtgcggaaatcaccatgcccgctccattatatag
ccccacgagtcaaaaaatcatgaccaagatgggatatataccaggaaagggactagggaaaa
atgaagatggcattaaaattccagttgaggctaaaataaatcaaaaaagagaaggaataggg
tatcctttttaggggcggccactgtagagcctcctaaacccataccattaacttggaaaaca
gaaaaaccggtgtgggtaaatcagtggccgctaccaaaacaaaaactggaggctttacattt
attagcaaatgaacagttagaaaagggtcatattgagccttcgttctcaccttggaattctc
ctgtgtttgtaattcagaagaaatcaggcaaatggcgtatgttaactgacttaagggctgta
aacgccgtaattcaacccatggggcctctccaacccgggttgccctctccggccatgatccc
aaaagattggcctttaattataattgatctaaaggattgcttttttaccatccctctggcag
agcaggattgtgaaaaatttgcctttactataccagccataaataataaagaaccagccacc
aggtttcagtggaaagtgttacctcagggaatgcttaatagtccaactatttgtcagacttt
 
tgtaggtcgagctcttcaaccagttagagaaaagttttcagactgttatattattcattata
ttgatgatattttatgtgctgcagaaacgaaagataaattaattgactgttatacatttctg
caagcagaggttgccaatgctggactggcaatagcatctgataagatccaaacctctactcc
ttttcattatttagggatgcagatagaaaatagaaaaattaagccacaaaaaatagaaataa
gaaaagacacattaaaaacactaaatgattttcaaaaattactaggagatattaattggatt
cggccaactctaggcattcctacttatgccatgtcaaatttgttctctatcttaagaggaga
ctcagacttaaatagtaaaagaatgttaaccccagaggcaacaaaagaaattaaattagtgg
aagaaaaaattcagtcagcgcaaataaatagaatagatcccttagccccactccaacttttg
atttttgccactgcacattctccaacaggcatcattattcaaaatactgatcttgtggagtg
gtcattccttcctcacagtacagttaagacttttacattgtacttggatcaaatagctacat
taatcggtcagacaagattacgaataataaaattatgtggaaatgacccagacaaaatagtt
gtccctttaaccaaggaacaagttagacaagcctttatcaattctggtgcatggcagattgg
tcttgctaattttgtgggaattattgataatcattacccaaaaacaaagatcttccagttct
taaaattgactacttggattctacctaaaattaccagacgtgaacctttagaaaatgctcta
acagtatttactgatggttccagcaatggaaaagcagcttacacagggccgaaagaacgagt
aatcaaaactccatatcaatcggctcaaagagcagagttggttgcagtcattacagtgttac
aagattttgaccaacctatcaatattatatcagattctgcatatgtagtacaggctacaagg
gatgttgagacagctctaattaaatatagcatggatgatcagttaaaccagctattcaattt
attacaacaaactgtaagaaaaagaaatttcccattttatattactcatattcgagcacaca
ctaatttaccagggcctttgactaaagcaaatgaacaagctgacttactggtatcatctgca
ctcataaaagcacaagaacttcatgctttgactcatgtaaatgcagcaggattaaaaaacaa
atttgatgtcacatggaaacaggcaaaagatattgtacaacattgcacccagtgtcaagtct
tacacctgcccactcaagaggcaggagttaatcccagaggtctgtgtcctaatgcattatgg
caaatggatgtcacgcatgtaccttcatttggaagattatcatatgttcatgtaacagttga
tacttattcacatttcatatgggcaacttgccaaacaggagaaagtacttcccatgttaaaa
aacatttattgtcttgttttgctgtaatgggagttccagaaaaaatcaaaactgacaatgga
ccaggatattgtagtaaagctttccaaaaattcttaagtcagtggaaaatttcacatacaac
aggaattccttataattcccaaggacaggccatagttgaaagaactaatagaacactcaaaa
ctcaattagttaaacaaaaagaagggggagacagtaaggagtgtaccactcctcagatgcaa
cttaatctagcactctatactttaaattttttaaacatttatagaaatcagactactacttc
tgcagaacaacatcttactggtaaaaagaacagcccacatgaaggaaaactaatttggtgga
aagataataaaaataagacatgggaaatagggaaggtgataacgtgggggagaggttttgct
tgtgtttcaccaggagaaaatcagcttcctgtttggatacccactagacatttgaagttcta
caatgaacccatcagagatgcaaagaaaagcacctccgcggagacggagacaccgcaatcga
gcaccgttgactcacaagatgaacaaaatggtgacgtcagaagaacagatgaagttgccatc
caccaagaaggcagagccgccgacttgggcacaactaaagaagctgacgcagttagctacaa
aatatctagagaacacaaaggtgacacaaaccccagagagtatgctgcttgcagccttgatg
attgtatcaatggtggtaagtctccctatgcctgcaggagcagctgcagctaactatacct a
ctgggcctatgtgcctttcccgcccttaattcgggcagtcacatggatggataatcctatag
aagtatatgttaatgatagtgtatgggtacctggccccatagatgatcgctgccctgccaaa
cctgaggaagaagggatgatgataaatatttccattgggtatcgttatcctcctatttgcct
agggagagcaccaggatgtttaatgcctgcagtccaaaattggttggtagaagtacctactg
tcagtcccatcagtagattcacttatcacatggtaagcgggatgtcactcaggccacgggta
aattatttacaagacttttcttatcaaagatcattaaaatttagacctaaagggaaaccttg
ccccaaggaaattcccaaagaatcaaaaaatacagaagttttagtttgggaagaatgtgtgg
 
ccaatagtgcggtgatattacaaaacaatgaatttggaactattatagattgggcacctcga
ggtcaattctaccacaattgctcaggacaaactcagtcgtgtccaagtgcacaagtgagtcc
agctgttgatagcgacttaacagaaagtttagacaaacataagcataaaaaattgcagtctt
tctacccttgggaatggggagaaaaaggaatctctaccccaagaccaaaaataataagtcct
gtttctggtcctgaacatccagaattatggaggcttactgtggcctcacaccacattagaat
ttggtctggaaatcaaactttagaaacaagagatcgtaagccattttatactgtcgacctaa
attccagtctaacagttcctttacaaagttgcgtaaagcccccttatatgctagttgtagga
aatatagttattaaaccagactcccagactataacctgtgaaaattgtagattgcttacttg
cattgattcaacttttaattggcaacaccgtattctgctggtgagagcaagagagggcgtgt
ggatccctgtgtccatggaccgaccgtgggaggcctcaccatccatccatattttgactgaa
gtattaaaaggtgttttaaatagatccaaaagattcatttttactttaattgcagtgattat
gggattaattgcagtcacagctacggctgctgtagcaggagttgcattgcactcttctgttc
agtcagtaaactttgttaatgattggcaaaaaaattctacaagattgtggaattcacaatct
agtattgatcaaaaattggcaaatcaaattaatgatcttagacaaactgtcatttggatggg
agacagactcatgagcttagaacatcgtttccagttacaatgtgactggaatacgtcagatt
tttgtattacaccccaaatttataatgagtctgagcatcactgggacatggttagacgccat
ctacagggaagagaagataatctcactttagacatttccaaattaaaagaacaaattttcga
agcatcaaaagcccatttaaatttggtgccaggaactgaggcaattgcaggagttgctgatg
gcctcgcaaatcttaaccctgtcacttgggttaagaccattggaagtactacgattataaat
ctcatattaatccttgtgtgcctgttttgtctgttgttagtctgcaggtgtacccaacagct
ccgaagagacagcgaccatcgagaacgggccatgatgacgatggcggttttgtcgaaaagaa
aagggggaaatgtggggaaaagcaagagagatcagattgttactgtgtctgtgtagaaagaa
gtagacataggagactccattttgttatgtactaagaaaaattcttctgccttgagattctg
ttaatctatgaccttacccccaaccccgtgctctctgaaacatgtgctgtgtcaactcagag
ttgaatggattaagggcggtgcaggatgtgctttgttaaacagatgcttgaaggcagcatgc
tccttaagagtcatcaccactccctaatctcaagtacccagggacacaaaaactgcggaagg
ccgcagggacctctgcctaggaaagccaggtattgtccaaggtttctccccatgtgatagtc
tgaaatatggcctcgtgggaagggaaagacctgaccgtcccccagcccgacacccgtaaagg
gtctgtgctgaggaggattagtaaaagaggaaggaatgcctcttgcagttgagacaagagga
aggcatctgtctcctgcctgtccctgggcaatggaatgtctcggtataaaacccgattgtat
gctccatctactgagatagggaaaaaccgccttagggctggaggtgggacctgcgggcagca
atactgctttgtaaagcattgagatgtttatgtgtatgcatatctaaaagcacagcacttaa
tcctttacattgtctatgatgcaaagacctttgttcacgtgtttgtctgctgaccctctccc
cacaattgtcttgtgaccctgacacatccccctctttgagaaacacccacagatgatcaata
aatactaagggaactcagaggctggcgggatcctccatatgctgaacgctggttccccgggt
ccccttatttctttctctatactttgtctctgtgtctttttcttttccaaatctctcgtccc
accttacgagaaacacccacaggtgtgtaggggcaacccacccctaca
SEQ ID NO: 151, MMTV antigen sequence 
ESYWAYLPKPPILHPVGWGSTDPIRVLTNQTMYLGGSPDFHGFRNMSGNVHFEGKSDTLPIC
FSFSFSTPTGCFQVDKQVFLSDTPTVDNNKPGGKGDKRRMWELWLHTLGNSGANTKLVPIKK
KLPPKYPHCQIAFKKDAFWEGDESAPPRWLPCAFPDKGVSFSPKGALGLLWDFSLPSPSVDQ
SDQIKSKKDLFGNYTPPVNKEVHRWYEAGWVEPTWFWENSPKDPNDRDFTALVPHTELFRLV
AASRHLILKRPGFQEHEMIPTSACVTYPYAILLGLPQLIDIEKRGSTFHISCSSCRLTNCLD
SSAYDYAAIIVKRPPYVLLPVDIGDEPWFDDSAIQTFRYATDLIRA
SEQ ID NO: 152, GFP reporter sequence 
gaacaaacgacccaacacccgtgcgttttattctgtctttttattgccgatcccctcagaag
aactcgtcaagaaggcgatagaaggcgatgcgctgcgaatcgggagcggcgataccgtaaag
cacgaggaagcggtcagcccattcgccgccaagctcttcagcaatatcacgggtagccaacg
ctatgtcctgatagcggtcggccgctttacttgtacagctcgtccatgccgagagtgatccc
ggcggcggtcacgaactccagcaggaccatgtgatcgcgcttctcgttggggtctttgctca
gggcggactgggtgctcaggtagtggttgtcgggcagcagcacggggccgtcgccgatgggg
gtgttctgctggtagtggtcggccaggtgagtccaggagatgtttcagcactgttgccttta
gtctcgaggcaacttagacaactgagtattgatctgagcacagcagggtgtgagctgtttga
agatactggggttgggggtgaagaaactgcagaggactaactgggctgagacccagtggcaa
tgttttagggcctaaggaatgcctctgaaaatctagatggacaactttgactttgagaaaag
agaggtggaaatgaggaaaatgacttttctttattagatttcggtagaaagaactttcatct
ttcccctatttttgttattcgttttaaaacatctatctggaggcaggacaagtatggtcatt
aaaaagatgcaggcagaaggcatatattggctcagtcaaagtggggaactttggtggccaaa
catacattgctaaggctattcctatatcagctggacacatataaaatgctgctaatgcttca
ttacaaacttatatcctttaattccagatgggggcaaagtatgtccaggggtgaggaacaat
tgaaacatttgggctggagtagattttgaaagtcagctctgtgtgtgtgtgtgtgtgtgtgt
gtgtgtgtgtgtgcgcgcacgtgtgtttgtgtgtgtgtgagagcgtgtgtttcttttaacgt
tttcagcctacagcatacagggttcatggtggcaagaagataacaagatttaaattatggcc
agtgactagtgctgcaagaagaacaactacctgcatttaatgggaaagcaaaatctcaggct
ttgagggaagttaacataggcttgattctgggtggaagctgggtgtgtagttatctggaggc
caggctggagctctcagctcactatgggttcatctttattgtctcctttcatctcaacagct
gcacgctgccgtcctcgatgttgtggcggatcttgaagttcaccttgatgccgttcttctgc
ttgtcggccatgatatagacgttgtggctgttgtagttgtactccagcttgtgccccaggat
gttgccgtcctccttgaagtcgatgcccttcagctcgatgcggttcaccagggtgtcgccct
cgaacttcacctcggcgcgggtcttgtagttgccgtcgtccttgaagaagatggtgcgctcc
tggacgtagccttcgggcatggcggacttgaagaagtcgtgctgcttcatgtggtcggggta
gcggctgaagcactgcacgccgtaggtcagggtggtcacgagggtgggccagggcacgggca
gcttgccggtggtgcagatgaacttcagggtcagcttgccgtaggtggcatcgccctcgccc
tcgccggacacgctgaacttgtggccgtttacgtcgccgtccagctcgaccaggatgggcac
caccccggtgaacagctcctcgcccttgctcaccatggtggcgaattcgaagcttgagctcg
agatctgagtccggtagcgctagcggatctgacggttcactaaaccagctctgcttatatag
acctcccaccgtacacgcctaccgcccatttgcgtcaatggggcggagttgttacgacattt
tggaaagtcccgttgattttggtgccaaaacaaactcccattgacgtcaatggggtggagac
ttggaaatccccgtgagtcaaaccgctatccacgcccattgatgtactgccaaaaccgcatc
accatggtaatagcgatgactaatacgtagatgtactgccaagtaggaaagtcccataaggt
catgtactgggcataatgccaggcgggccatttaccgtcattgacgtcaatagggggcgtac
ttggcatatgatacacttgatgtactgccaagtgggcagtttaccgtaaatactccacccat
tgacgtcaatggaaagtccctattggcgttactatgggaacatacgtcattattgacgtcaa
tgggcgggggtcgttgggcggtcagccaggcgggccatttaccgtaagttatgtaacg
SEQ ID NO: 153, CMV-GFP sequence

gcaaatgggcggtaggcgtgtacggtgggaggtctatataagcagagctggtttagtgaacc
gtcagatccgctagcgctaccggtcgccaccatggtgagcaagggcgaggagctgttcaccg
gggtggtgcccatcctggtcgagctggacggcgacgtaaacggccacaagttcagcgtgtcc
ggcgagggcgagggcgatgccacctacggcaagctgaccctgaagttcatctgcaccaccgg
cagctgcccgtgccctggcccaccctcgtgaccaccctgacctacggcgtgcagtgcttcag

ccgctaccccgaccacatgaagcagcacgacttcttcaagtccgccatgcccgaaggctacg
tccaggagcgcaccatcttcttcaaggacgacggcaactacaagacccgcgccgaggtgaag
ttcgaggcgacaccctggtgaaccgcatcgagctgaagggcatcgacttcaaggaggacggc
aacatcctggggcacaagctggagtacaactacaacagccacaacgtctatatcatggccga
caagcagaagaacggcatcaaggtgaacttcaagatccgccacaacatcgaggacggcagcg
tgcagctcgccgaccactaccagcagaacacccccatcggcgacggccccgtgctgctgccc
gacaaccactacctgagcacccagtccgccctgagcaaagaccccaacgagaagcgcgatca
catggtcctgctggagttcgtgaccgccgccgggatcactctcggcatggacgagctgtaca
ag

## Claims

1. A method for identifying the degree of aging of a subject or a cell, tissue or organ thereof, or evaluating the physical age of a subject, or diagnosing progeroid diseases, or assessing the degree of senescence of an isolated animal cell or cell population, the method comprising
a) detecting the activation level of an endogenous retrovirus (ERV) in a sample from the subject or in the cell; and
b) comparing the result of the detection with the reference value of the activation level of the ERV

2. The method of claim 1, wherein the reference value is obtained by detecting reference subject.

3. The method of claim 1 or 2, wherein the reference value is obtained by detecting a population of reference subjects.

4. The method of any one of claims 1-3, wherein the activation level of the ERV includes
the methylation level of the ERV locus,
the level of an ERV transcript,
the level of an ERV protein,
the presence, localization of the ERV particles in the cell and/or the release of the ERV particles from the cell, and/or
the transposition probability of ERV locus.

5. The method of claim 4, wherein the ERV transcript is selected from transcripts of Env, Pol, Pro and Gag genes.

6. The method of claim 4, wherein the ERV protein is selected from Env, Pol, Pro and Gag proteins.

7. The method of any one of claims 1-6, wherein the sample is selected from lung, liver, skin, myocardium and brain samples.

8. A method for regulating the senescence of a cell, the method comprising contacting the cell with an agent regulating the activation of an ERV locus.

9. The method of claim 8, wherein the regulating agent is an agent inhibiting the activation of ERV locus or an agent inhibiting the ERV.

10. The method of claim 8, wherein the regulating agent is an agent promoting the activation of an ERV locus in the cell.

11. A method for treating premature aging in a subject in need or a cell, tissue of organ thereof, or preventing or delaying the aging of a subject or a cell, tissue of organ thereof, the method comprising administering to the subject an agent inhibiting the activation of an ERV locus or an agent inhibiting the ERV.

12. A method for culturing isolated animal cell or cell population, or preventing or delaying cell senescence, the method comprising contacting the cell with an agent inhibiting the activation of an ERV locus in the cell or an agent inhibiting the ERV.

13. A method for promoting cell senescence, the method comprising contacting the cell with an agent promoting the activation of an ERV locus in the cell or the ERV.

14. The method of claim 10 or 13, wherein the agent promoting the activation of the ERV locus is a transcriptional activation system targeting the ERV locus.

15. The method of claim 14, wherein the transcriptional activation system is a CRISPR-dCas transcriptional activation system.

16. The method of any one of claims 1-15, wherein the ERV is selected from the group comprising families HERVK (including HERVK11), MER61, MER61C, Charlie4, MER101B, MamGyp, HERV16, HUERS-P2 and ERVW and MMTV and homologs thereof.

17. The method of any one of claim 1-16, wherein the subject is a rodent, and optionally, the ERV is from MMTV family.

18. The method of any one of claim 1-16, wherein the subject is a non-human primate, and optionally, the ERV is from ERVW family.

19. The method of any one of claims 1-16, wherein the subject is a human, and optionally, the ERV is from HERVK family, e.g., HERVK in provirus HML2_1q22 position.

20. A method for promoting cell senescence, the method comprising contacting the cell with a DNA methyltransferase inhibitor, e.g., decitabine, Lomeguatrib, SGI-1027 or 5-azacytidine.

21. The method of claim 20, wherein the cell is a non-human mammal cell, a non-human primate cell or a human cell.

22. A method for treating premature aging in a subject in need or a tissue of organ thereof, or preventing or delaying the aging of a subject or a tissue of organ thereof, or preventing or delaying aging of local tissues, including joint and skin, or treating progeroid diseases and aging-related diseases, the method comprising administering a retrovirus inhibitor to the subject.

23. The method of claim 22, wherein the retrovirus inhibitor comprises a reverse transcriptase inhibitor, an integrase inhibitor and/or a protease inhibitor.

24. The method of claim 23, wherein the reverse transcriptase inhibitor includes nucleoside reverse transcriptase inhibitor such as enofovir, abacavir, stavudine (D4T), lamivudine (3TC) and/or zidovudine, and/or non-nucleoside reverse transcriptase inhibitor such as efavirenz, etravirine and/or nevirapine.

25. The method of claim 23, wherein the integrase inhibitor includes Raltegravir.

26. The method of claim 23, wherein the protease inhibitor includes Lopinavir and/or Darunavir.

27. The method of any one of claims 22-26, wherein the retrovirus inhibitor includes lamivudine (3TC), abacavir and/or Raltegravir.

28. A method for preventing or delaying aging of local tissues, including joint and skin, or treating progeroid diseases and aging-related diseases, the method comprising administering an agent inhibiting the activation of ERV locus or an agent inhibiting the ERV to subject in need.

29. The method of claims 9, 11, 12 or 28, wherein the agent inhibiting the activation of ERV locus promotes the methylation level of ERV locus, or is a gene editing system directed to the ERV locus, or an antisense nucleic acid or interfering nucleic acid against Env, Pol, Pro and/or Gag genes of the ERV, or a binding molecule capable of binding to a protein of the ERV such as Env protein, e.g., an antibody or the antigen-binding fragment or antibody derivative thereof, preferably an ERV neutralizing antibody or the antigen-binding fragment or antibody derivative thereof.

30. The method of any one of claims 22-29, wherein the progeria is HGPS or WS, wherein the aging-related diseases is selected from arthritis, premature ovarian failure, hepatic fibrosis, pulmonary fibrosis, frailty and angiocardiopathy.
